(19) Europäisches Patentamt
European Patent Office
Office européen des brevets

(11) EP 1 309 649 B1

(12) **EUROPÄISCHE PATENTSCHRIFT**

(45) Veröffentlichungstag und Bekanntmachung des Hinweises auf die Patenterteilung:
**07.07.2004 Patentblatt 2004/28**

(21) Anmeldenummer: **01971792.5**

(22) Anmeldetag: **27.07.2001**

(51) Int Cl.[7]: **C08G 77/46**, A61K 7/48, D06M 15/647, C08G 77/54

(86) Internationale Anmeldenummer:
**PCT/EP2001/008699**

(87) Internationale Veröffentlichungsnummer:
**WO 2002/010257 (07.02.2002 Gazette 2002/06)**

(54) **POLYAMMONIUM-POLYSILOXAN-VERBINDUNGEN, VERFAHREN ZU IHRER HERSTELLUNG UND IHRE VERWENDUNG**

POLYAMMONIUM-POLYSILOXANE COMPOUNDS, METHODS FOR THE PRODUCTION AND USE THEREOF

COMPOSES DE POLYAMMONIUM-POLYSILOXANE ET PROCEDES DE PREPARATION ET D'UTILISATION DESDITS COMPOSES

(84) Benannte Vertragsstaaten:
**AT BE CH CY DE DK ES FI FR GB GR IE IT LI LU MC NL PT SE TR**

(30) Priorität: **27.07.2000 DE 10036541**
**27.07.2000 DE 10036530**
**27.07.2000 DE 10036543**
**27.07.2000 DE 10036542**

(43) Veröffentlichungstag der Anmeldung:
**14.05.2003 Patentblatt 2003/20**

(73) Patentinhaber: **GE Bayer Silicones GmbH & Co. KG**
**40699 Erkrath (DE)**

(72) Erfinder:
- **LANGE, Horst**
  **44879 Bochum (DE)**
- **WAGNER, Roland**
  **51061 Köln (DE)**
- **WITOSSEK, Anita**
  **40764 Langenfeld (DE)**
- **STACHULLA, Karl-Heinz**
  **51379 Leverkusen (DE)**
- **TEUBER, Siegfried**
  **47799 Krefeld (DE)**
- **KROPFGANS, Martin**
  **51519 Odenthal (DE)**
- **SOCKEL, Karl-Heinz**
  **51373 Leverkusen (DE)**
- **MÖLLER, Annette**
  **51381 Leverkusen (DE)**

(56) Entgegenhaltungen:
**EP-A- 0 282 720**
**WO-A-00/50491**
**WO-A-01/41720**
**WO-A-99/50338**
**DE-A- 19 817 776**
**FR-A- 2 535 730**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 309 649 B1

Printed by Jouve, 75001 PARIS (FR)

## Beschreibung

Die Erfindung betrifft Polysiloxan-Verbindungen, Verfahren zu deren Herstellung und deren Verwendung.

Tertiäre Aminogruppen enthaltende Polysiloxane sind als textile Weichmacher in der EP-A-0 441 530 offenbart. Die zusätzliche Einführung von Ethylenoxid-/Propylenoxideinheiten als hydrophilierender Komponente fuhrt zu einer Verbesserung des Effekts, wie in der US 5 591 880 und der US 5 650 529 beschrieben. Dort wurde vorgeschlagen worden, in Seitenketten Alkylenoxideinheiten und tertiäre Aminogruppen zu positionieren, die über Esterstrukturen mit der Siloxanhauptkette verbunden sind. Nachteilig an diesem Konzept ist die komplizierte Veresterung in Gegenwart tertiärer Aminogruppen. Alternativ hierzu ist aus der US 5 981 681 bekannt, α,ω-epoyxmodifizierte Siloxane mit sekundäre Aminofunktionen aufweisenden Polyalkylenoxiden zur Reaktion zu bringen (s.a. US 5807956).

Es ist ebenfalls die Reaktion von α,ω-epoxymodifizierten Siloxanen mit Piperazin beschrieben worden, die in Abhängigkeit von der eingesetzten Piperazinmenge zu oligomeren bis polymeren Strukturen mit tertiären Aminofunktionen in der Hauptkette führt wie in der US 4 847 154 beschrieben.

α,ω-Diquartäre Polysiloxane sind aus der US 4 891 166 bekannt Die Synthese erfolgt durch Reaktion von α,ω-Diepoxiden mit tertiären Aminen in Gegenwart von Säuren. Die US 4 833 225 offenbart lineare polyquartäre Polysiloxane, die durch die Reaktion von α,ω-Diepoxiden mit di-tertiären Aminen in Gegenwart von Säuren synthetisiert werden. Alternativ können α,ω-halogenalkylmodifizierte Siloxane mit ditertiären Aminen in polymere Polyquats überführt werden gemäß der US 4 587 321.

Die Substanzen gemäß US 4 891 166, US 4 833 225 und US 4587321 besitzen eine ausgeprägte Tendenz, auf Festkörperoberflächen aufzuziehen.

Die US 5 196 499 beschreibt Polysiloxanverbindungen mit terminalen quartären Ammoniumgruppen. Polysiloxanverbindungen mit innenständigen, zwei- oder dreiwertigen, verbrückenden quartären Ammoniumgruppen werden dort jedoch nicht erwähnt

Alkylenoxidmodifizierte quartäre Polysiloxane sind aus α,ω-OH terminierten Polysiloxanen und Trialkoxysilanen durch Kondensation synthetisiert worden. Die quartäre Ammoniumstruktur wird über das Silan eingebracht, wobei das quartäre Stickstoffatom durch Alkylenoxideinheiten substituiert worden ist, wie bereits in der US 5 625 024 beschrieben.

Streng kammartige alkylenoxidmodifizierte quartäre Polysiloxane sind aus der US 5 098 979 bereits bekannt. Die Hydroxylgruppen von kammartig substituierten Polyethersiloxanen werden mit Epichlorhydrin in die entsprechenden Chlorhydrinderivate überführt. Hieran schließt sich die Quartärnierung mit tertiären Aminen an. Nachteilig an dieser Synthese ist der notwendige Umgang mit Epichlorhydrin und die relativ geringe Reaktivität der Chlorhydrin-Gruppierung während der Quartämierung.

Aus diesem Grund heraus sind die Hydroxylgruppen kammartig substituierter Polyethersiloxane alternativ mit Chloressigsäure verestert worden. Durch die Carbonylaktivierung kann die abschließende Quartärnierung erleichtert vollzogen werden, wie in der US 5 153 294 und der US 5 166 297 offenbart.

Lineare Polyammonium-Strukturen, die Siloxan-, Alkylenoxid- und verbrückende Ammoniumeinheiten enthalten, sind über die beschriebenen Prozesse nicht zugänglich.

In der DE-OS 3236466 liefert die Umsetzung von OH-terminierten Siloxanen mit quartäre Ammoniumstrukturen enthaltenden - Alkoxysilanen reaktive Zwischenprodukte, die mit geeigneten Vernetzungsagenzien, wie Trialkoxysilanen, auf der Faseroberfläche zu waschbeständigen Schichten vernetzen sollen. Ein wesentlicher Nachteil dieses Ansatzes ist, daß die über Stunden notwendige Stabilität eines wässrigen Ausrüstungsbades nicht garantiert werden kann und unvorhergesehene Vernetzungsreaktionen im Bad bereits vor der Textilausrüstung auftreten können.

Auch ist eine weitere Verbesserung der Eigenschaften der Polysiloxanverbindungen wünschenswert.

Es ist somit eine Aufgabe der Erfindung, spezielle Polyammonium-Polysiloxanverbindungen, deren Herstellung und Verwendung als waschbeständige hydrophile Weichmacher zu beschreiben, wobei die Polyammonium-Polysiloxanverbindungen den Textilien nach entsprechender Applikation einen silicontypischen weichen Griff und eine ausgeprägte Hydrophilie verleihen und dieses Eigenschaftsbild auch nach Einwirkung von Detergenzienformulierungen während wiederholter Waschprozesse bei ggf. erhöhter Temperatur nicht verloren geht. Es ist eine weitere Aufgabe der Erfindung, die Verwendung dieser Polyammonium-Polysiloxanverbindungen als separate Weichmacher nach bzw. als Weichmacher in auf nichtionogenen oder anionischen/nichtionogenen Tensiden beruhenden Formulierungen zur Wäsche von Fasern und Textilien, sowie als Mittel zur Verhinderung bzw. Rückgängigmachung von Textilverknitterungen zu beschreiben.

Es ist weiterhin eine Aufgabe der Erfindung Polyammonium-Polysiloxanverbindungen zur Verwendung als waschbeständige, hydrophile Weichmacher für Textilien bereitzustellen, die dem Angriff konzentrierter Tensidlösungen mit hohem Fett- und Schmutzlösevermögen widerstehen können. Ferner sollten die Polyammonium-Polysiloxanverbindungen den in modernen Waschmitteln enthaltenen stark alkalischen Komplexbildnern, oxidativ wirkenden Bleichmitteln und komplexen Enzymsystemen widerstehen, auch wenn die Fasern der Einwirkung durch solche Waschmittel oftmals über Stunden bei erhöhten Temperaturen ausgesetzt sind.

Eine weitere Aufgabe der Erfindung besteht darin, Behandlungsmittel für Haare bereitzustellen, die in Gegenwart von Tensiden einer geringen Auswaschbarkeit unterliegen. Dementsprechend sollen durch die vorliegende Erfindung auch kosmetische Formulierungen bereitgestellt werden, die die Polyammonium-Polysiloxanverbindungen enthalten.

Es ist deshalb die Aufgabe der vorliegenden Erfindung gewesen, Polyammonium-Strukturen, die Siloxan-, Alkylenoxid- und verbrückende Ammoniumeinheiten enthalten, zur Verfügung zu stellen, die nicht die Nachteile des Standes der Technik aufweisen.

Die Aufgabe wird erfindungsgemäß durch Polysiloxanverbindungen gelöst, in denen Siloxan- und Alkylenoxidsubstrukturen über Ammoniumstrukturen miteinander verbunden sind.

Die Erfindung betrifft Polysiloxan-Verbindung enthaltend:

a) mindestens eine Polyalkylenoxid-Struktureinheit der allgemeinen Formeln:

$$-A-E-,\ -E-A-,\ -A-E-A'-\ \text{und/oder}\ -A'-E-A-$$

worin

A = $-CH_2C(O)O-$, $-CH_2CH_2C(O)O-$, $-CH_2CH_2CH_2C(O)O-$, $-OC(O)CH_2-$, $-OC(O)CH_2-$, $-OC(O)CH_2CH_2-$ und/oder $-OC(O)CH_2CH_2CH_2-$

A'= $-CH_2C(O)-$, $-CH_2CH_2C(O)-$, $-CH_2CH_2CH_2C(O)-$, $-C(O)CH_2-$, $-C(O)CH_2-$, $-C(O)CH_2CH_2-$ und/oder $-C(O)CH_2CH_2CH_2-$

E = eine Polyalkylenoxidgruppe der allgemeinen Formeln:

$$-[CH_2CH_2O]_q-[CH_2CH(CH_3)O]_r-$$

und/oder

$$-[OCH(CH_3)CH_2]_r-[OCH_2CH_2]_q-$$

mit

q = 1 bis 200,
r = 0 bis 200,

wobei das endständige Sauerstoffatom der Gruppe A an die endständige $-CH_2$-Gruppe der Gruppe E, und das endständige Carbonyl-Kohlenstoffatom der Gruppe A' an das endständige Sauerstoffatom der Gruppe E jeweils unter Ausbildung von Estergruppen binden,
und/oder mindestens eine endständige Polyalkylenoxid-Struktureinheit der Formel

$$-A-E-R^2$$

worin A und E die oben genannte Bedeutung aufweisen, und

$R^2$ = H, geradkettiger, cyclischer oder verzweigter $C_1$-$C_{20}$-Kohlenwasserstoffrest der durch -O-, oder -C(O)- unterbrochen und mit -OH substituiert und acetylenisch, olefinisch oder aromatisch sein kann,

b) mindestens ein zweiwertiger oder dreiwertiger organischer Rest, der mindestens eine Ammoniumgruppe enthält,

c) mindestens eine Polysiloxan-Struktureinheit der allgemeinen Formel:

-K-S-K-,

mit

S =

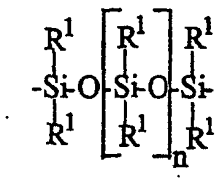

worin

$R^1$ = $C_1$-$C_{22}$-Alkyl, $C_1$-$C_{22}$-Fluoralkyl oder Aryl,
n = 0 bis 1000, und wenn mehrere Gruppen S in der Polysiloxanverbindung vorliegen, diese gleich oder verschieden sein können,

K = ein zweiwertiger oder dreiwertiger geradkettiger, cyclischer oder verzweigter $C_2$-$C_{40}$-Kohlenwasserstoffrest, der durch -O-, -NH-,

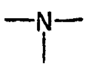
,

$-NR^1-$, -C(O)-, -C(S)-,

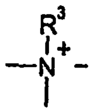
und
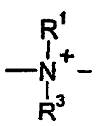

unterbrochen und mit -OH substituiert sein kann, worin

$R^1$ wie oben definiert ist, oder gegebenenfalls eine Bindung zu einem zweiwertigen Rest $R^3$ darstellt,
$R^3$ einen einwertigen oder zweiwertigen geradkettigen, cyclischen oder verzweigten $C_1$-$C_{20}$-Kohlenwasserstoffrest, der durch -O-, -NH-, -C(O)-, -C(S)- unterbrochen und mit -OH substituiert sein kann, oder $-A-E-R^2$ darstellt, worin A, E und $R^2$ wie oben definiert ist,

wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt,

d) einen organischen oder anorganischen Säurerest zur Neutralisation der aus der(n) Ammoniumgruppe(n) resultierenden Ladungen.

Die erfindungsgemäßen Polysiloxanverbindungen sind dadurch gekennzeichnet, dass sie die vorstehend definierten Komponenten a) bis d) aufweisen.

Die Polysiloxanverbindungen werden dabei durch Bindung der genannten Struktureinheiten bzw. Reste a) bis c) aneinander gebildet Die Komponente d) dient der Neutralisation der aus der Komponente b) resultierenden positiven Ladungen.

Die erfindungsgemäßen Polysiloxanverbindungen können oligomere oder polymere Verbindungen sein. Oligomere Verbindungen schließen dabei auch den unten beschriebenen Fall ein, worin die Polysiloxanverbindung lediglich eine Wiederholungseinheit aufweist.

Polymere erfindungsgemäße Polysiloxanverbindungen entstehen dabei naturgemäß durch alternierende Verknüpfung von zweiwertigen Resten.

Im Falle der polymeren erfindungsgemäßen Polysiloxanverbindungen resultieren die endständigen Atomgruppierungen aus den endständigen Atomgruppierungen der eingesetzten Ausgangsmaterialien. Dies ist dem Fachmann an sich bekannt.

In einer bevorzugten Ausführungsform sind die polymeren erfindungsgemäßen Polysiloxanverbindungen lineare Polyammonium-Polysiloxanverbindungen, die sich aus den Struktur-Komponenten a) bis c) zusammensetzen. So können die linearen polymeren erfindungsgemäßen Polysiloxanverbindungen, insbesondere deren aus den Wiederholungseinheiten gebildete lineare polymere Hauptkette, durch alternierende Aneinanderreihung von Polyalkylenoxid-Struktureinheiten a), organischen Resten, die mindestens eine, vorzugsweise quartäre Ammoniumgruppe enthalten b) und Polysiloxan-Struktureinheiten c) aufgebaut werden. D.h. die darüber hinaus gegebenenfalls in den Strukturkomponenten vorhandenen freien Valenzen (wie sie bei dreiwertigen Resten als Komponente b) oder bei dreiwertigen Resten K auftreten können) dienen bevorzugt nicht dem Aufbau polymerer Seitenketten bzw. polymerer Verzweigungen.

In einer weiteren Ausfuhrungsform kann die Hauptkette der linearen polymeren erfindungsgemäßen Polysiloxanverbindungen von den organischen Resten, die mindestens eine Ammoniumgruppe enthalten b) und den Polysiloxan-Struktureinheiten c) aufgebaut werden, und die Polyalkylenoxid-Struktureinheiten a) binden als Seitenketten an den dreiwertigen organischenAmmoniumgruppenrest.

So können beispielsweise folgende Aufbauten resultieren:

-(Polyalkylenoxidstruktureinheit-Polysiloxanstruktureinheit-

Polyalkylenoxidstruktureinheit-bevorzugt quartärer Ammoniumgruppenrest)$_x$-

-(Polysiloxanstruktureinheit-bevorzugt quartärer Ammoniumgruppenrest)$_x$-

Polyalkylenoxidstrutureinheit)$_x$-

—(Polysiloxanstruktureinheit—bevorzugt quartärer Ammoniumgruppenrest)$_x$—
|
Polyalkylenoxidstruktureinheit

Je nach molarem Verhältnis der monomeren Ausgangsverbindungen können erfindungsgemäße Polysiloxanverbindungen resultieren, die ledigliche eine Wiederholungseinheit aufweisen. Dies ist dem Fachmann an sich bekannt Dieser Fall führt beispielsweise zu erfindungsgemäßen Polysiloxanverbindungen des Aufbaus:

(endständige Polyalkylenoxidstruktureinheit-quartärer Ammoniumgruppenrest-Polysiloxanstruktureinheit-quartärer Ammoniumgruppenrest-endständige Polyalkylenoxidstruktureinheit).

Die erfindungsgemäße Polysiloxanverbindungen bestehen bevorzugt im wesentlichen aus den Komponenten a) bis d), wobei die polymeren erfindungsgemäßen Polysiloxanverbindungen naturgemäß die aus der Umsetzung der monomeren Ausgangsmaterialien resultierenden terminalen Gruppen aufweisen. Es können aber auch monofunktionelle Kettenabbruchsmittel eingesetzt werden.

Bei den Polyalkylenoxid-Struktureinheiten a) kann es sich um zweiwertige Reste der allgemeinen Formeln:

-A-E-, -E-A-, -A-E-A'- und/oder -A'-E-A-

handeln. Die Reste A bzw. A' bedeuten dabei:

A = $-CH_2C(O)O-$, $-CH_2CH_2C(O)O-$, $-CH_2CH_2CH_2C(O)O-$, $-OC(O)CH_2-$, $-OC(O)CH_2-$, $-OC(O)CH_2CH_2-$ und/oder $-OC(O)CH_2CH_2CH_2-$

A' = $-CH_2C(O)-$, $-CH_2CH_2C(O)-$, $-CH_2CH_2CH_2C(O)-$, $-C(O)CH_2-$, $-C(O)CH_2$, $-C(O)CH_2CH_2-$ und/oder $-C(O)CH_2CH_2CH_2-$

Die Polyalkylenoxidgruppe E der allgemeinen Formeln:

$$-[CH_2CH_2O]_q-[CH_2CH(CH_3)O]_r- \text{ und/oder } -[OCH(CH_3)CH_2]_r-[OCH_2CH_2]_q-$$

mit q = 1 oder 2 bis 200 und r = 0 bis 200, schließen dabei alle möglichen Ethylenoxid/Propylenoxid-Gruppierungen ein. So kann es sich um statistische Ethylenoxid/Propylenoxid-Copolymergruppen oder Ethylenoxid/Propylenoxid-Block-Copolymergruppen mit beliebiger Anordnung von einem oder mehreren Ethylenoxidoder Propylenoxid-Blöcken handeln.

Die Anbindung der Reste A bzw. A' an die Gruppe E erfolgt dabei so, dass das endständige Sauerstoffatom der Gruppe A an die endständige $-CH_2$-Gruppe der Gruppe E, und das endständige Carbonyl-Kohlenstoffatom der Gruppe A' an das endständige Sauerstoffatom der Gruppe E jeweils unter Ausbildung von Estergruppen binden.

Bei den Polyalkylenoxid-Struktureinheiten a) kann es sich weiterhin um einwertige, d.h. endständige Polyalkylenoxid-Struktureinheit der Formel

$$-A-E-R^2$$

handeln, worin A und E die oben genannte Bedeutung aufweisen, und

$R^2$ H, geradkettiger, cyclischer oder verzweigter $C_1$-$C_{20}$-Kohlenwasserstoffrest ist, der durch -O-, oder -C(O)- unterbrochen und mit -OH substituiert und acetylenisch, olefinisch oder aromatisch sein kann.

Die Komponente b) aus der sich die erfindungsgemäßen Polysiloxanverbindungen zusammensetzen, ist mindestens ein zweiwertiger oder dreiwertiger organischer Rest, der mindestens eine Ammoniumgruppe enthält. Die Bindung des Restes an die übrigen Komponenten der erfindungsgemäßen Polysiloxanverbindungen erfolgt bevorzugt über das Stickstoffatom einer oder mehrerer Ammoniumgruppen in dem organischen Rest. Der Begriff "zweiwertig" bzw. "dreiwerkig" bedeutet, dass der organische Ammonium-Rest zur Ausbildung von Bindungen insbesondere zu den übrigen Komponenten der erfindungsgemäßen Polysiloxanverbindungen zwei oder drei freie Valenzen aufweist. Der Ammoniumrest wird zweckmäßig durch eine $NH_4^+$-Gruppe dargestellt, in der mindestens zwei Wasserstoffatome durch organische Gruppen substituiert sind. Vorzugsweise handelt es sich um eine sekundäre oder quartäre, besonders bevorzugt um eine quartäre Ammoniumgruppe. Eine quartäre Ammoniumgruppe ist nach allgemeiner Definition (s. z.B. Römpp-Chemie-Lexikon) eine Gruppe bei der alle vier Wasserstoffatome einer $NH_4^+$-Gruppe durch organische Reste ersetzt sind.

Die Komponente c) der erfindungsgemäßen Polysiloxanverbindungen ist mindestens eine Polysiloxan-Struktureinheit der allgemeinen Formel:

-K-S-K-,

S ist darin eine Polysiloxangruppe der allgemeinen Formel

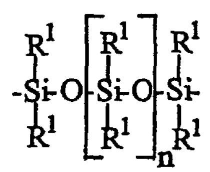

worin $R^1$ = $C_1$-$C_{22}$-Alkyl, $C_1$-$C_{22}$-Fluoralkyl oder Aryl, vorzugsweise Phenyl, n = 0 bis 1000, und wenn mehrere Gruppen S in der Polysiloxanverbindung vorliegen, diese gleich oder verschieden sein können.

$R^1$ ist bevorzugt $C_1$-$C_{18}$-Alkyl, $C_1$-$C_{18}$-Fluoralkyl und Aryl. Weiterhin ist $R^1$ bevorzugt $C_1$-$C_{18}$-Alkyl, $C_1$-$C_6$-Fluoralkyl und Aryl. Weiterhin ist $R^1$ bevorzugt $C_1$-$C_6$-Alkyl, $C_1$-$C_6$-Fluoralkyl, bevorzugter $C_1$-$C_4$-Fluoralkyl, und Phenyl. Noch bevorzugter ist $R^1$ Methyl, Ethyl, Trifluorpropyl und Phenyl.

Der Begriff "$C_1$-$C_{22}$-Alkyl" bedeutet im Rahmen der vorliegenden Erfindung, daß die aliphatische Kohlenstoffwasserstoffgruppen 1 bis 22 Kohlenstoffatomen besitzen, die geradkettig oder verzweigt sein können. Beispielhaft seien Methyl, Ethyl, Propyl, n-Butyl, Pentyl, Hexyl, Heptyl, Nonyl, Decyl, Undecyl, iso-Propyl, Neopentyl, und 1,2,3

Trimethylhexyl aufgeführt.

Der Begriff "$C_1$-$C_{22}$-Fluoralkyl" bedeutet im Ramen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2-Triflourbutyl aufgeführt.

Der Begriff "Aryl" bedeutet im Rahmen der vorliegenden Erfindung unsubstituierte oder ein oder mehrfach mit OH, F, Cl, $CF_3$ $C_1$-$C_6$-Alkyl, $C_1$-$C_6$-Alkoxy, $C_3$-$C_7$-Cycloalkyl $C_2$-$C_6$-Alkenyl oder Phenyl substituiertes Phenyl. Der Ausdruck kann gegebenenfalls auch Naphthyl bedeuten.

K stellt einen zweiwertigen oder dreiwertigen geradkettigen, cyclischen oder verzweigten $C_2$-$C_{40}$-Kohlenwasserstoffrest dar, der durch -O-, -NH-,

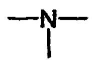,

-$NR^1$, -C(O)-, -C(S)-,

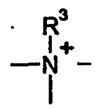 und 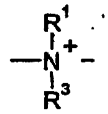

unterbrochen und mit -OH substituiert sein kann.

"Unterbrochen" bedeutet dabei, das im Falle der zweiwertigen Reste eine -$CH_2$-Gruppierung im Falle der dreiwertigen Reste eine

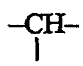

Gruppierung des Kohlenwasserstoffrestes durch die genannten Gruppen ersetzt sind. Dies gilt auch für den übrigen Teil der Beschreibung, wenn diese Bezeichnung verwendet wird.

Die Gruppe K bindet über ein Kohlenstoffatom an das Siliziumatom der Gruppe S.

Die Gruppe K kann, wie oben zu sehen, ebenfalls bevorzugt quartäre Ammoniumgruppen aufweisen, so dass Ammoniumgruppen zusätzlich zu den Ammoniumgruppen in der genannten Komponente b) in den erfindungsgemäßen Polysiloxanverbindungen resultieren.

Die erfindungsgemäßen Polysiloxanverbindungen können, wie zum Beispiel in dem Rest K, Aminogruppen aufweisen. Die Umsetzung der erfindungsgemäßen Polysiloxanverbindungen mit Säuren führt zu deren Protonierung. Solche protonierte Aminogruppen aufweisende Polysiloxanverbindungen sind im Umfang der vorliegenden Erfindung enthalten.

Die Bindung der Komponente c), der Polysiloxan-Struktureinheit -K-S-K-, zu den übrigen Aufbaukomponenten über den Rest K erfolgt bevorzugt nicht über ein Stickstoffatom des Restes K.

$R^1$ ist wie oben definiert oder stellt gegebenenfalls eine Bindung zu einem zweiwertigen Rest $R^3$ dar, so dass ein Cyclus resultiert.

$R^3$ stellt einen einwertigen oder zweiwertigen geradkettigen, cyclischen oder verzweigten $C_1$-$C_{20}$-Kohlenwasserstoffrest, der durch -O-, -NH-, -C(O)-, -C(S)- unterbrochen und mit -OH substituiert sein kann, oder -A-E-$R^2$ dar, worin A, E und $R^2$ wie oben definiert ist,

Die Reste K können gleich oder verschieden voneinander sein, und im Falle, dass K einen dreiwertigen Rest darstellt, erfolgt die Absättigung der dritten Valenz über eine Bindung an den vorstehend genannten organischen Rest, der mindestens eine Ammoniumgruppe enthält.

Die erfindungsgemäßen Polysiloxanverbindungen enthalten weiterhin die Komponente d), mindestens einen organischen oder anorganischen anionischen Säurerest zur Neutralisation der aus der(n) Ammoniumgruppe(n) resultierenden Ladungen. Organische oder anorganische Säurereste sind Reste, die formal aus der Abspaltung von eines oder mehrerer Protonen aus organischen oder anorganischen Säuren resultieren und schließen beispielsweise ein Halogenide, wie Fluorid, Chlorid, Bromid, Sulfate, Nitrate, Phosphate, Carboxylate, wie Formiat, Acetat, Propionat etc., Sulfonate, Sulfate, Polyethercarboxylate und Polyethersulfate etc. Bevorzugt ist Chlorid. Die organischen oder anorganischen anionischen Säurereste als Komponente d) der erfindungsgemäßen Polysiloxanverbindungen können gleich oder verschieden voneinander sein. So resultieren aus der Umsetzung der Amine mit Alkylhalogeniden bevorzugt Halogenidionen, während zum Beispiel Carboxylate aus den Carbonsäuren, die bei der Umsetzung von Bisepoxiden mit Aminen zugesetzt werden können, resultieren.

In einer bevorzugten Ausführungsform der erfindungsgemäßen Polysiloxanverbindungen stellt K einen zweiwertigen oder dreiwertigen geradkettigen, cyclischen oder verzweigten $C_2$-$C_{40}$-Kohlenwasserstoffrest dar, der durch -O-, -NH-,

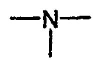 ,

-$NR^1$-, -C(O)-, -C(S)- unterbrochen und mit -OH substituiert sein kann, worin $R^1$ wie oben definiert ist, und wobei die Reste K gleich oder verschieden voneinander sein können.

Der zuvor genannte organische Rest, der mindestens eine, bevorzugt quartäre Ammoniumgruppe enthält, ist bevorzugt ein Rest der allgemeinen Formel:

$$-N^1-F-N^1-$$

worin $N^1$ eine quartäre Ammoniumgruppe der allgemeinen Formel

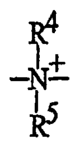

ist, worin $R^4$ einen einwertigen oder zweiwertigen geradkettigen, cyclischen oder verzweigten $C_1$-$C_{20}$-Kohlenwasserstoffrest darstellt, der durch -O-, -NH-, -C(O)-, -C(S)- unterbrochen und mit -OH substituiert sein kann, und $R^5$ ist ein einwertiger geradkettiger, cyclischer oder verzweigter $C_1$-$C_{20}$-Kohlenwasserstoffrest darstellt, der durch -O-, -NH-, -C (O)-, -C(S)- unterbrochen und mit -OH substituiert sein kann, oder eine Einfachbindung zu einem zweiwertigen Rest $R^4$ oder einem vierwertigen Rest F, und die Reste $R^4$ und $R^5$ innerhalb der Gruppe -$N^1$-F-$N^1$- sowie in der Polysiloxanverbindung gleich oder verschieden voneinander sein können,

F ist in zweiwertiger oder vierwertiger geradkettiger, cyclischer oder verzweigter $C_2$-$C_{30}$-Kohlenwasserstoffrest, der durch -O-, -NH-,

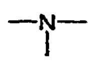 ,

-C(O)-, -C(S)-, eine Siloxankette S, wobei für S die oben genannten Bezüge gelten, unterbrochen und mit -OH substituiert sein kann,

Bezüglich weiterer Einzelheiten der Definitionen der quartären Ammoniumgruppe der Formel -$N^1$-F-$N^1$- (bevorzugte Ausführungsformen etc.) sei auf die Erläuterungen der ersten Ausführungsform der vorliegenden Erfindung, in der diese Gruppe realisiert ist, verwiesen, und die auch in diesem allgemeineren Kontext Gültigkeit besitzen.

Der zuvor genannte organische Rest, der mindestens eine, bevorzugt quartäre Ammoniumgruppe enthält, kann weiterhin bevorzugt ein Rest der allgemeinen Formel:

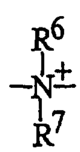

sein, worin $R^6$ ein einwertiger oder zweiwertiger geradkettiger, cyclischer oder verzweigter $C_1$-$C_{30}$-Kohlenwasserstoffrest ist, der durch -O-, -NH- -C(O)-, -C(S)-unterbrochen und mit -OH substituiert sein kann, oder $R^6$ stellt eine Einfachbindung zu einem dreiwertigen Rest K dar.

$R^7$ ist ein einwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann, oder -A-B-$R^2$, worin -A-E-$R^2$ die oben genannte Bedeutung aufweist, oder eine Einfachbindung zu einem zweiwertigen Rest $R^6$ oder zu einem dreiwertigen Rest K.
Die Reste $R^6$ und $R^7$ können gleich oder verschieden voneinander sein.

Bezüglich weiterer Einzelheiten der Definitionen der quartären Ammoniumgruppe der Formel

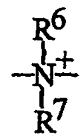

(bevorzugte Ausführungsformen) sei auf die Erläuterungen der zweiten, dritten und vierten Ausführungsform der vorliegenden Erfindung verwiesen, in der diese Gruppe realisiert ist, und die auch in diesem allgemeineren Kontext Gültigkeit besitzen.

Der zuvor genannte organische Rest, der mindestens eine Aminoniumgrappe enthält, kann weiterhin bevorzugt ein Rest der allgemeinen Formel:

$$-N^5-F^1-N^5-$$

sein, worin

$N^5$ eine Ammoniumgruppe der allgemeinen Formel

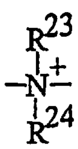

ist, worin

$R^{23}$ Wasserstoff: ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,

$R^{24}$ Wasserstoff, ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu . einem zweiwertigen Rest $R^{23}$ darstellt, und die Reste $R^{23}$ und $R^{24}$ innerhalb der Gruppe -$N^5$-$F^1$-$N^5$- sowie in der Polysiloxanverbindung gleich oder verschieden voneinander sein können,

$F^1$ = ein zweiwertiger geradkettiger, cyclischer oder verzweigter Kohlenwasserstoffrest darstellt, der durch -O-, -NH-,

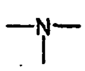 ,

-C(O)-, -C(S)-oder durch eine Gruppe -E- unterbrochen sein kann,

und worin eine Mehrzahl der Gruppen $N^5$ und $F^1$ jeweils gleich oder verschieden voneinander sein können.

Bezüglich weiterer Einzelheiten der Definitionen der Ammoniumgruppe der Formel

$$-N^5-F^1-N^5-$$

(bevorzugte Ausführungsformen) sei auf die Erläuterungen der fünften Ausführungsform der vorliegenden Erfindung verwiesen, in der diese Gruppe beispielhaft realisiert ist, und die auch in diesem allgemeineren Kontext Gültigkeit besitzen.

Im folgenden wird die Erfindung anhand von fünf bevorzugten Ausführungsformen der Erfindung näher beschrieben.

Eine besondere Ausführungsform der Erfindung (die im folgenden als erste Ausführungsform der Erfindung bezeichnet wird), worin der zuvor genannte organische Rest, der mindestens eine, bevorzugt quartäre Ammoniumgruppe enthält, als Komponente b) der erfindungsgemäßen Polysiloxanverbindungen einen Rest der allgemeinen Formel:

$$-N^1-F-N^1-$$

darstellt, wird durch die Polysiloxan-Verbindungen der folgenden allgemeinen Formel (I) dargestellt:

$$[B-N^1-F-N^1]_m- \qquad (I)$$

worin

m = 2 bis 500,

B = -A-E-K-S-K-E-A- und zusätzlich gegebenenfalls -A-E-A'- bzw. -A'-E-A- ist,

worin S, K, -A-E-, -E-A-, -A-E-A'- bzw. -A'-E-A- und $-N^1-F-N^1-$ wie oben definiert sind, und der Anteil der Gruppe -A-E-A'- bzw. -A'-E-A- in der Gruppe B so gewählt sein kann, dass die Masse von -A-E-A'- bzw. -A'-E-A- von 0 bis 90 %, bevorzugt 0% oder 0,1 bis 50% der Masse des Polysiloxananteils S im Polymer beträgt.

Die erste Ausführungsform der Erfindung betrifft bevorzugt lineare alkylenoxidmodifizierte polyquarternäre Polysiloxane der allgemeinen Formel (I'),

$$-[B-N^1-F-N^1]_m- \qquad (I')$$

worin

m 2 bis 500,

B -A-E-K-S-K-E-A-

S

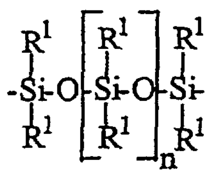

$R^1$ $C_1$-$C_{22}$-Alkyl, $C_1$-$C_{22}$-Fluoralkyl oder Aryl,

n 0 bis 1000,

K ein zweiwertiger geradkettiger, cyclischer oder verzweigter $C_2$-$C_{20}$-Kohlenwasserstoffrest der durch -O-, -NH-, $-NR^1-$, -C(O)-, -C(S)- unterbrochen und mit -OH substituiert sein kann,

E eine Polyalkylenoxideinheit der Struktur

$$-[CH_2CH_2O]_q-[CH_2CH(CH_3)O]_r-$$

mit

q 1 bis 200,

r 0 bis 200 und

A $-CH_2C(O)O-$, $-CH_2CH_2C(O)O-$ oder $-CH_2CH_2CH_2C(O)O-$,

$N^1$ eine quarternäre Ammoniumstruktur

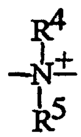

$R^4$ ein einwertiger oder zweiwertiger geradkettiger, cyclischer oder verzweigter $C_1$-$C_{20}$-Kohlenwasserstoffrest darstellt, der durch O-, -NH-, -C(O)-, -C(S)- unterbrochen und mit -OH substituiert sein kann,

$R^5 = R^4$ oder eine Einfachbindung zu $R^4$ oder F darstellt,

F ein zweiwertiger oder vierwertiger geradkettiger, cyclischer oder verzweigter $C_2$-$C_{30}$-Kohlenwasserstoffrest darstellt, der durch -O-, -NH-,

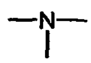 ,

-C(O)-, -C(S)-, eine Siloxankette S, wobei für S die oben genannten Bezüge gelten, unterbrochen und mit -OH substituiert sein kann.

Die Möglichkeit einer vierwertigen Substruktur für F bedeutet, daß F ein verzweigtes oder Ringsystem mit den begrenzenden $N^1$ bilden kann, so daß F dann mit jeweils zwei Bindungen an der Quartärnierung von beiden begrenzenden $N^1$ beteiligt ist. Zur Illustration sei auf die in Beispiel 1 behandelte Pipe verwiesen.

In einer weiteren Ausführungsform der vorliegenden Erfindung bedeutet die Möglichkeit einer zweiwertigen Substruktur für $R^4$, daß es sich in diesen Fällen um eine cyclische Systeme bildende Struktur handelt, worin $R^5$ in diesem Fall eine Einfachbindung zu $R^4$ ist. Beispiele sind Morpholinyl- und Piperidinylstrukturen.

Bevorzugtere Ausführungsformen dieser sogenannten ersten Ausführungsform der Erfindung sowie Verfahren zur Herstellung der genannten Polysiloxanverbindungen der Formel (I) bzw. (I') werden nachfolgend beschrieben.

$R^4$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$,

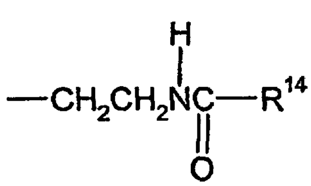

oder

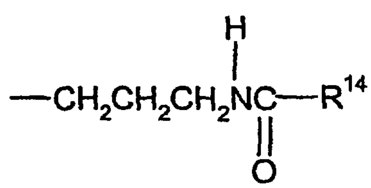 ,

worin $R^{14}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann ist.

$R^4$ und $R^5$ können wie vorstehend erwähnt auch gemeinsam eine cyclische Struktur der Formeln

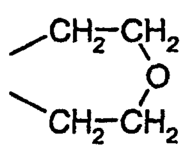

oder

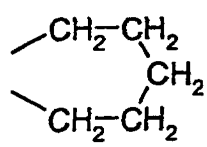

bilden.

Zu den bevorzugten Bedeutungen von $R^1$ in der sogenannten ersten Ausführungsform der Erfindung kann zu den vorstehenden Ausführungen verwiesen werden.

In der sogenannten ersten Ausführungsform der Erfindung ist $R^4$ bevorzugt ein einwertiger oder zweiwertiger geradkettiger, cyclischer oder verzweigter $C_1$-$C_{16}$-, bevorzugter $C_3$-$C_{16}$-Kohlenwasserstoffrest, der durch -O-, -NH-, -C(O)-, -C(S)-unterbrochen und mit OH substituiert sein kann, bevorzugter ein $C_3$-$C_{16}$-Kohlenwasserstoffrest der durch -O-, -NH-, -$NR^1$-, -C(O)-, -C(S)- unterbrochen und mit -OH substituiert sein kann, worin $R^1$ die obengenannte Bedeutung besitzt.

In der sogenannten ersten Ausführungsform der Erfindung ist F bevorzugt ein zweiwertiger oder vierwertiger geradkettiger, cyclischer oder verzweigter $C_2$-$C_{20}$-Kohlenwasserstoffrest, der durch -O-, -NH-,

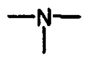 ,

-C(O)-, -C(S), eine Siloxankette S, wobei für S die oben genannten Bezüge gelten, unterbrochen und mit -OH substituiert sein kann.

In der sogenannten ersten Ausführungsform der Erfindung ist K bevorzugt $-CH_2CH_2CH_2-$, $-(CH_2)_4-$, $-(CH_2)_6-$, $-CH=CHCH_2-$ und $-CH_2CH_2CH_2OCH_2CH(OH)CH_2-$.

In der sogenannten ersten Ausführungsform der Erfindung stellt $R^{14}$ bevorzugt unsubstituierte $C_5$-$C_{17}$-Kohlenwasserstoffreste dar, die sich von den entsprechenden Fettsäuren ableiten oder aber hydroxylierte $C_3$-$C_{17}$-Reste, die auf hydroxylierte Carbonsäuren, bevorzugt Saccharidcarbonsäuren zurückgeführt werden können.

In der sogenannten ersten Ausführungsform der Erfindung stellt $R^{14}$ weiterhin bevorzugt hydroxylierte Reste aus der Gruppe bestehend aus

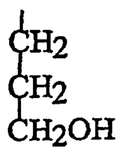 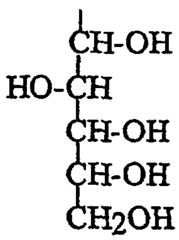 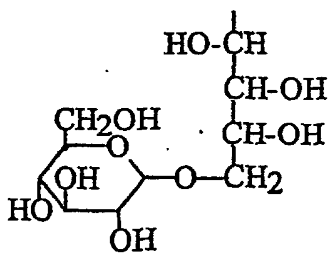

dar.

In der sogenannten ersten Ausführungsform der Erfindung ist m 2 bis 100, bevorzugt 2 bis 50.

In der sogenannten ersten Ausführungsform der Erfindung ist n 0 bis 1000, bevorzugt 0 bis 100, bevorzugter 0 bis 80 und besonders bevorzugt 10 bis 80.

In der sogenannten ersten Ausführungsform der Erfindung ist q 1 bis 200, bevorzugt 1 bis 50, bevorzugter 2 bis 20 und besonders bevorzugt 2 bis 10.

In der sogenannten ersten Ausführungsform der Erfindung ist r 0 bis 200, bevorzugt 0 bis 100, bevorzugter 0 bis 50 und noch bevorzugter 0 bis 20.

Die erfindungsgemäßen Polysiloxan-Verbindungen der sogenannten ersten Ausführungsform der Erfindung können zweckmäßig hergestellt werden, in dem $\alpha,\omega$-Wasserstoffpolysiloxane der allgemeinen Formel

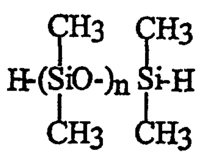

mit, bezogen auf SiH-Gruppen, 1,0 bis 1,5 mol eines Halogencarbonsäureesters, der sich von niedermolekularen, oligomeren und polymeren alkenyl- oder alkinylmodifizierten Alkylenoxiden der allgemeinen Zusammensetzung

$$HO[CH_2CH_2O]_q\text{-}[CH_2CH(CH_3)O]_rR^{15}$$

worin q und r wie vorstehend definiert sind, ableitet, durch Hydrosilylierung zur Reaktion gebracht werden und die gebildeten $\alpha,\omega$-haloacylierten Siloxan-Alkylenoxidzwischenstufen mit di-tertiären Aminen in polyquarternäre Polysiloxanderivate überführt werden, wobei das stöchiometrische Verhältnis der Haloacylgruppen zu den tertiären Aminogruppen vorzugsweise 1 : 1 beträgt.

Sofern die $\alpha,\omega$ Si-H funktionalisierte Siloxane der allgemeinen Struktur

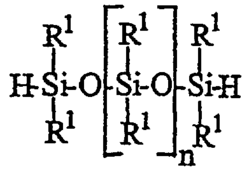

nicht kommerziell erhältlich sind, können diese Siloxane nach bekannten Verfahren, z.B. durch Äquilibrierung hergestellt werden (Silicone, Chemie und Technologie, Vulkan-Verlag, Essen 1989, S. 82-84).

Die Einführung der Alkylenoxidblöcke gelingt über die entsprechenden Halogencarbonsäureester der Alkylenoxide. Bevorzugte Ausgangsmaterialien für deren Synthese sind die oben genannten niedermolekulare, oligomere und polymere Alkylenoxide der allgemeinen Zusammensetzung

$$HO[CH_2CH_2O]_q\text{-}[CH_2CH(CH_3)O]_rR^{15}$$

in der q und r die oben angegebenen Bedeutungen aufweisen. Bei dem Rest $R^{15}$ handelt es sich zweckmäßig um Alkenyl- und Alkinylstrukturen, welche durch die an sich bekannte Addition von $\equiv$SiH in K übergehen. Beispiele für $R^{15}$ sind Allyl-, Butenyl-, Hexenyl-, Propinyl- und Allylglycidylstrukturen. Bevorzugte Vertreter hinsichtlich des Alkylenoxidblockes sind Diethylenglycol, Triethylenglycol, Tetraethylenglycol, die Oligoethylenglycole mit Molgewichten von 300 bis 1000 g/mol, bevorzugt werden Molgewichte von etwa 400, 600 und 800 g/mol (400 bis 800 g/mol) verwendet, sowie Dipropylenglycol. Die Herstellung der alkenyl- bzw. alkinylmodifizierten Alkylenoxide erfolgt durch sauer öder alkalisch katalysierte Addition von Ethylenoxid und/oder Propylenoxid an die entsprechenden Alkohole wie in der US 5 625 024 oder im Organikum, Organisch-chemisches Grundpraktikum, 17. Auflage, VEB Deutscher Verlag der Wissenschaften, Berlin 1988, S. 259 beschrieben.

Die Veresterung der alkenyl- bzw. alkinylmodifizierten Alkylenoxide erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den $C_2$-$C_4$-Halogencarbonsäuren, deren Anhydriden oder Säurechloriden. Bevorzugt werden als Säurechloride Chloressigsäure und 3-Chlor-propionsäure eingesetzt. Die Reaktion wird in Abwesenheit von Lösungsmitteln durchgeführt. Einzelheiten der Reaktionsführung können den Beispielen entnommen werden.

In einem nachfolgenden Schritt werden die $\alpha$-Alkenyl/alkinyl-, $\omega$-Haloacyl-Alkylenoxide mit den $\alpha,\omega$ Si-H funktionalisierten Siloxanen zur Reaktion gebracht. Die allgemeine Durchführung von Hydrosilylierungen mit ungesättigten Halogencarbonsäureestem ist bekannt (B.Marciniec, Comprehensive Handbook on Hydrosilylation, PergamonPress, Oxford 1992, S. 134-137,151-155).

In einer bevorzugten Ausführungsform des vorstehend beschriebenen Verfahrens werden durch Hydrosilylierung der alkenyl- bzw. alkinylmodifizierten Alkylenoxide zunächst $\alpha,\omega$-OH-terminierte reaktive Zwischenprodukte erzeugt, die in einem nachfolgenden Veresterungsschritt in die entsprechenden $\alpha,\omega$-Haloacylverbindungen überführt werden können. Diese $\alpha,\omega$-haloacylierten Siloxan-Alkylenoxidzwischenstufen können mit geeigneten Aminen in polyquaternäre Polymere überführt werden.

Es ist generell möglich, sekundäre Amine einzusetzen, die bei einem stöchiometrischen Verhältnis von 2 : 1 von Halogenacylgruppen zu dem sekundären Amin, die entsprechenden polyquartäre Verbindungen liefern. In einer weiteren bevorzugten Ausführungsform ist die Verwendung von Aminen erwünscht, die über zwei tertiäre Aminofunktionen verfügen, in der dann das stöchiometrische Verhältnis von Halogenacylgruppen zu der tertiären Aminogruppen vorzugsweise 1 : 1 beträgt.

Bei diesen Aminen kann es sich um Moleküle mit geradkettigem Spacer zwischen den tertiären Aminofunktionen, wie N,N,N',N'-Tetramethylethylendiamin und die entsprechenden höheren Alkylenderivate handeln. Andererseits ist es möglich, cyclische Amine einzusetzen. Beispielhaft seien N,N'-Dimethylpiperazin und Triethylendiamin aufgeführt.

In einer weiteren Ausfuhrungsform des vorstehend beschriebenen Verfahrens zur Herstellung der Polysiloxanverbindung gemäß der ersten Ausführungsform der Erfindung werden bevorzugt Amine mit mehr als zwei tertiären Aminofunktionen eingesetzt, wenn zwei tertiäre Aminofunktionen über einen hinreichend großen Reaktivitätsvorteil gegenüber den anderen tertiären Aminofunktionen verfügen.

Beispielhaft sei N,N,N',N",N"-Pentamethyldipropylentriamin aufgeführt. Derartige Moleküle werden dann als difunktionell betrachtet.

Zusätzlich ist es möglich, funktionelle Strukturen zwischen den tertiären Aminostrukturen zu positionieren. So liegt es im Rahmen der Erfindung, α,ω-tertiär aminomodifizierte Siloxane einzusetzen, die beispielsweise durch die Reaktion von α,ω-SiH funktionalisierten Siloxanen mit N,N-Dialkylallylaminen, bevorzugt N,N-Dimethylallyamin, hergestellt werden können (B.Marciniec, Comprehensive Handbook on Hydrosilylation, Pergamon Press, Oxford 1992, S. 122-124). Alternativ können zur Synthese der α,ω-tertiär aminomodifizierten Siloxane ensprechende α,ω-epoxyfunktionalisierte Siloxane mit sekundären Aminen, aus der Gruppe der Dialkylamine, z.B. Dimethylamin oder der cyclischen Aminen, wie Morpholin oder Piperidin, oder sekundär-tertiäre Diamine, wie N-Methylpiperazin zur Reaktion gebracht werden. Überraschenderweise wurde an dieser Variante gefunden, daß beim Hydrosilylierungsschritt von Allylaminderivaten die generell leichter handhabbaren Epoxidderivate, zum Beispiel solche aus der Gruppe der Allylglycidether, verwendet werden können. In einer weiteren bevorzugten Ausführungsform des vorstehend beschriebenen Verfahrens können Siloxane mit mehr als zwei tertiären Aminogruppen eingesetzt werden, wenn sich deren Reaktivität, wie oben erläutert, hinreichend unterscheidet. Ein Beispiel stellen die Aminosiloxane dar, welche in einer Zweistufenreaktion aus α,ω-SiH funktionalisierten Siloxanen mit Allylglycidether und anschließender Alkylierung mit N-Methylpiperazin synthetisiert werden können.

Werden die sekundären Amine oder die mindestens zwei tertiäre Aminostrukturen enthaltenden Moleküle mit den α,ω-haloacylierten Siloxan-Alkylenoxidzwischenstufen in den oben behandelten Stöchiometrien zur Reaktion gebracht, entstehen dem Wesen nach lineare Produkte, in denen Siloxan- Alkylenoxidblöcke über quartäre Ammoniumfunktionen miteinander verbunden sind. Mit der gezielten Nutzung von Aminen, die über mehr als zwei tertiäre Aminofunktionen verfügen, gelingt es, regioselektive Produkte mit tertiären und quartären Stickstoffatomen in der Polymerstruktur zu synthetisieren.

In einer weiteren bevorzugten Ausführungsform der vorstehend beschriebenen ersten Ausführungsform der Erfindung können mehrere α,ω-halogenacylierte Siloxan-Alkylenoxidzwischenstufen eingesetzt werden, die sich hinsichtlich der Kettenlängen der Siloxankomponenten und/oder der Alkylenoxidkomponenten unterscheiden können. In einer weiteren Ausführungsform ist es auch möglich, einen Teil der α,ω-halogenacylierten Siloxan-Alkylenoxidzwischenstufen durch α,ω-halogenacylierte Alkylenoxidderivate ohne Siloxananteil zu ersetzen.

In einer weiteren bevorzugten Ausführungsform In einer weiteren bevorzugten Ausführungsform der vorstehend beschriebenen ersten Ausführungsform der Erfindung können die α,ω-haloacylierten Siloxan-Alkylenoxidzwischenstufen mit unterschiedlichen Aminen verbunden werden. Voraussetzung für diese vorteilhaften Ausgestaltungen ist jedoch die Beibehaltung der gewünschten Gesamtstöchiometrie der Reaktion.

Als Anionen kommen insbesondere die während der Quartämierung gebildeten Halogenidionen, bevorzugt Chloridionen, in Betracht. Jedoch können durch lonenaustausch auch andere Anionen eingeführt werden. Beispielhaft seien Anionen, wie Carboxylate, Sulfonate, Sulfate, Polyethercarboxylate und Polyethersulfate aufgeführt.

Die Quartärnierungsreaktionen werden bevorzugt in polaren organischen Lösungsmitteln ausgeführt. Geeignet sind z.B. Alkohole aus der Gruppe bestehend aus Methanol, Ethanol, i-Propanol und n-Butanol; Glycole aus der Gruppe bestehend aus Ethylenglycol, Diethylenglycol, Triethylenglycol, die Methyl-, Ethyl- und Butylether der genannten Glycole, 1,2-Propylenglycol und 1,3-Propylenglycol; Ketone, wie Aceton und Methylethylketon, Ester, wie Ethylacetat, Butylacetat und 2-Ethyl-hexylacetat, Ether, wie Tetrahydrofuran und Nitroverbindungen, wie Nitromethan. Die Wahl des Lösungsmittels richtet sich wesentlich nach der Löslichkeit der Reaktionspartner und der angestrebten Reaktionstemperatur.

Die Reaktionen werden im Bereich von 20 °C bis 130°C, vorzugsweise 40 °C bis 100 °C ausgeführt. Die Reaktionszeiten richten sich nach der Temperatur und der Art der miteinander reagierenden Gruppen. Generell können die Reaktionen in einem Bereich von einer bis 10 Stunden vollzogen werden.

Eine besondere Ausführungsform der Erfindung (die im folgenden als sogenannte zweite Ausführungsform der Erfindung bezeichnet wird) wird durch die Polysiloxan-Verbindungen der allgemeinen Formel (II) dargestellt,

$$R^2\text{-E-A-}N^2\text{-K-S-K-}N^2\text{-A E-}R^2 \qquad \text{(II)}$$

worin

S, K, -A-E-, -E-A- und $R^2$ die oben genannten Bedeutungen aufweisen, und

$N^2$ ein organischer Rest, der mindestens eine quartäre Ammoniumgruppe enthält, der allgemeinen Formel

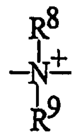

ist, worin

$R^8$ = ein einwertiger oder zweiwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann,

$R^9$ ein einwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann, oder eine Einfachbindung zu einem zweiwertigen Rest $R^8$ oder zu einem dreiwertigen Rest K darstellt, und die Reste $R^8$ und $R^9$ innerhalb der Polysiloxanverbindung der allgemeinen Formel (II) gleich oder verschieden voneinander sein können.

Bevorzugt handelt es sich bei den Polysiloxanverbindungen der zweiten Ausführungsform der Erfindung um α,ω-Alkylenoxid- und polyquartemär modifizierte Polysiloxane der allgemeinen Formel (II'),

$$R^{16}\text{-E-A-N}^2\text{-K-S-K-N}^2\text{-A-E-R}^{16} \qquad \text{(II')}$$

worin

S =

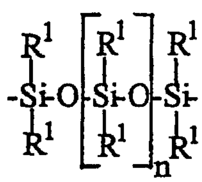

mit

$R^1$ = $C_1$-$C_{22}$-Alkyl, $C_1$-$C_{22}$-Fluoralkyl oder Aryl,
n = 0 bis 1000,
K = ein zweiwertiger oder dreiwertiger geradkettiger, cyclischer oder verzweigter $C_2$-$C_{20}$-Kohlenwasserstoffrest, der durch -O-, -NH-, -$NR^1$-,

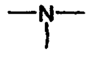 ,

- C(O)-, -C(S)- unterbrochen und mit -OH substituiert sein kann,

$N^2$ eine quartäre Ammoniumstruktur

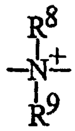

$R^8$ ein einwertiger oder zweiwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann,
$R^9 = R^8$ oder eine Einfachbindung zu K oder $R^8$ darstellt,
A $-CH_2C(O)O-$, $-CH_2CH_2C(O)O-$ oder $-CH_2CH_2CH_2C(O)O-$
E eine Polyalkylenoxideinheit der Struktur

$$-[CH_2CH_2O]_q-[CH_2CH(CH_3)O]_r-$$

mit

q 1 bis 200
r 0 bis 200 und
$R^{16}$ H, geradkettiger, cyclischer oder verzweigter $C_1$-$C_{20}$-Kohlenwasserstoffrest, der durch -O-, oder -C(O)- unterbrochen und -OH substituiert und acetylenisch, olefinisch oder aromatisch sein kann.

Bevorzugtere Ausführungsformen dieser sogenannten zweiten Ausführungsform der Erfindung sowie Verfahren zur Herstellung der genannten Polysiloxanverbindungen der Formel (II) bzw. (II') werden nachfolgend beschrieben.

Die Möglichkeit einer dreiwertigen Substruktur für K bedeutet hier, daß K verzweigt sein kann und dann mit zwei Bindungen an der Quartämierung von $N^2$ beteiligt ist. Die Möglichkeit einer zweiwertigen Substruktur für $R^8$ bedeutet, daß es sich in diesen Fällen um eine cyclische Systeme bildende Struktur handelt, wobei $R^9$ dann eine Einfachbindung zu $R^2$ ist.

$R^8$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$,

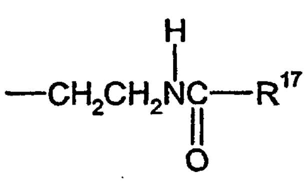

oder

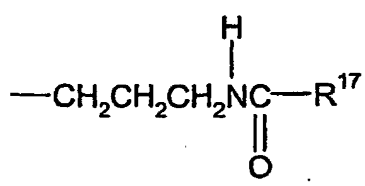

,

worin $R^{17}$ einen geradkettigen, cyclischen oder verzweigten $C_1$-$C_{18}$-Kohlenwasserstoffrest, der durch -O-, -NH-, -C (O)-, -C(S)- unterbrochen und mit -OH substituiert sein kann ist.

$R^8$ und $R^9$ können wie vorstehend erwähnt auch gemeinsam eine cyclische Struktur der Formeln

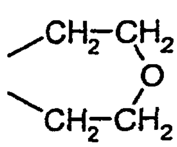

oder

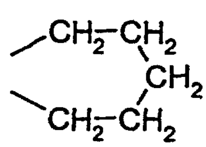

bilden.

Zu den bevorzugten Bedeutungen von $R^1$ in der sogenannten zweiten Ausführungsform der Erfindung kann zu den vorstehenden Ausführungen verwiesen werden.

In der sogenannten zweiten Ausführungsform der Erfindung ist K bevorzugt ein zweiwertiger oder dreiwertiger geradkettiger, cyclischer oder verzweigter $C_3$-$C_{16}$-Kohlenwasserstoffrest, der durch -O-, -NH-, $-NR^1-$,

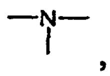

-C(O)-, -C(S)-unterbrochen und mit -OH substituiert sein kann, worin $R^1$ wie vorstehend definiert ist Bevorzugt für K sind zum Beispiel Reste der folgenden Strukturen:

$$-CH_2CH_2CH_2-$$

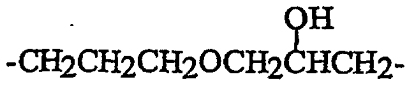

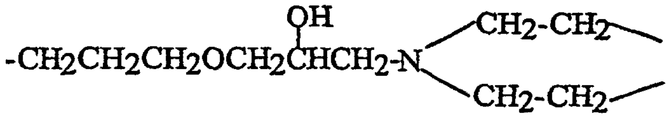

$R^8$ ist bevorzugt ein einwertiger oder zweiwertiger geradkettiger, cyclischer oder verzweigter $C_1$-$C_{16}$-Kohlenwasserstoffrest, der durch -O-, -NH-, -C(O)-, -C(S)-unterbrochen und mit -OH substituiert sein kann.

$R^{16}$ ist bevorzugt ein geradkettiger, cyclischer oder verzweigter $C_1$-$C_{18}$-Kohlenwasserstoffrest, der durch -O- oder -C(O)- unterbrochen und mit -OH substituiert und acetylenisch oder olefinisch sein kann.

Weiterhin ist $R^{16}$ bevorzugt $C_5$-$C_{17}$-Alkyl, $-CH_2CH{=}CH_2$, $-CH_2CH(OH)CH_2OCH_2CH{=}CH_2$, $-CH_2C{=}CH$, C(O) $CH_3$, $-C(O)CH_2CH_3$.

$R^{17}$ stellt bevorzugt unsubstituierte $C_5$-$C_{17}$-Kohlenwasserstoffreste, die sich von den entsprechenden Fettsäuren ableiten oder aber hydroxylierte $C_3$-$C_{17}$-Reste, die auf hydroxylierte Carbonsäuren, bevorzugt auf Saccharidcarbonsäuren zurückgeführt werden können, dar.

$R^{17}$ wird besonders bevorzugt aus der Gruppe aus

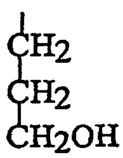 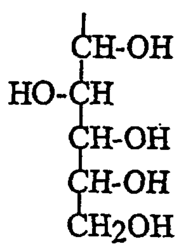 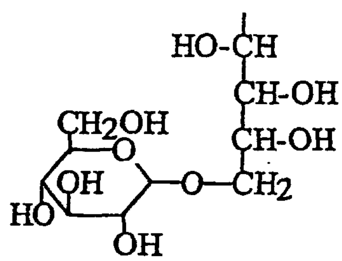

ausgewählt.

In der sogenannten zweiten Ausführungsform der Erfindung ist n bevorzugt 0 bis 200, bevorzugter 0 bis 80, besonders bevorzugt 10 bis 80.

In der sogenannten zweiten Ausführungsform der Erfindung ist q bevorzugt 1 bis 50, bevorzugter 2 bis 20 und besonders bevorzugt 2 bis 10.

In der sogenannten zweiten Ausführungsform der Erfindung ist r bevorzugt 0 bis 100 und bevorzugter 0 bis 50.

In der sogenannten zweiten Ausführungsform der Erfindung ist r bevorzugt 0 bis 20 und bevorzugter 0 bis 10.

Die erfindungsgemäßen Polysiloxan-Verbindungen der sogenannten zweiten Ausführungsform der Erfindung können zweckmäßig hergestellt werden, in dem die in $\alpha,\omega$-Stellung tertiäre Aminogruppen ausweisenden Siloxanderivate durch Hydrosilylierung von tertiäre Aminogruppen tragenden ungesättigten Strukturen, mit $\alpha,\omega$ Si-H funktionalisierten Siloxanen der allgemeinen Struktur

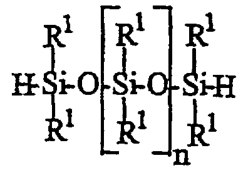

worin $R^1$ und n die oben angegebenen Bedeutungen haben, hergestellt werden. Ausgangspunkt für das Verfahren sind α,ω Si-H funktionalisierte Siloxane der allgemeinen Struktur

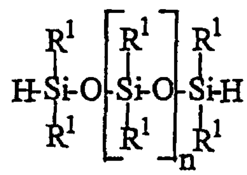

worin $R^1$ und n die oben angegebenen Bedeutungen haben. Sofern nicht kommerziell erhältlich, können diese Siloxane nach bekannten Verfahren, z.B. durch Äquilibriexung hergestellt werden (Silicone, Chemie und Technologie, Vulkan-Verlag, Essen 1989, S. 82-84). Die anschließende Einführung tertiärer Aminofunktionen kann auf zwei Wegen erfogen. Einerseits ist es möglich, tertiäre Aminofunktionen tragende ungesättigte Strukturen, beispielsweise N,N Dimethylallylamin, durch Hydrosilylierung direkt an das Siloxan zu binden. Dieser Prozeß ist allgemein bekannt (B. Marciniec, Comprehensive Handbook on Hydrosilylation, Pergamon Press, Oxford 1992, S. 122-124).
Weiterhin können die in α,ω-Stellung tertiäre Aminogruppen aufweisenden Siloxanderivate und die Halogencarbonsäureesterderivate der Alkylenoxide in einem molaren Verhältnis von Siloxan zu Halogencarbonsäureester von 1 : 2 zur Reaktion gebracht werden.

Bevorzugt werden die in α,ω-Stellung tertiäre Aminogruppen aufweisenden Siloxanderivate in einem Zweistufenprozeß durch Hydrosilylierung von halogenierten Alkenen, ungesättigten Halogencarbonsäureestem und epoxyfunktionellen Alkenen, mit α,ω Si-H funktionalisierten Siloxanen der allgemeinen Struktur

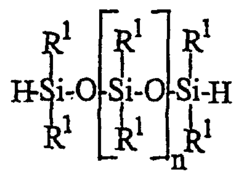

worin $R^1$ und n die oben genannten Bedeutungen haben, und nachfolgender Alkylierung von sekundäre Aminofunktionen tragenden Verbindungen, aus der Gruppe bestehend aus N,N-Dialkylaminen, cyclischen sekundären Aminen, sekundäre Aminofunktionen tragenden Aminoamiden, und sekundär-tertiären Diaminen, umgesetzt.

Bevorzugt werden als halogenierte Alkene bevorzugt Allylchlorid und Allylbromid verwendet.

Als ungesättigte Halogencarbonsäureester werden bevorzugt solche aus der Gruppe, bestehend aus Chloressigsäureallylester, Chloressigsäurepropargylester, 3-Chlorpropionsäureallylester und 3-Chlorpropionsäurepropargylester verwendet.

Als epoxyfunktionelle Alkene werden bevorzugt Vinylcyclohexenoxid und, Allylglycidether verwendet.
Als N,N-Dialkylamine werden bevorzugt Dimethylamin, Diethylamin, Dibutylamin, Diethanolamin und N-Methylglucamin verwendet.

Als cyclische sekundäre Amine werden bevorzugt Morpholin und Piperidin verwendet.

Als sekundäre Aminofunktionen tragende Aminoamide werden Umsetzungsprodukte von Diethylentriamin oder Dipropylentriamin mit Lactonen, bevorzugt γ-Butyrolacton, Gluconsäure-δ-lacton und Glucopyranosylarabonsäurelacton verwendet.

D.h., es ist es bevorzugt, durch die Hydrosilylierung zunächst reaktive Zwischenprodukte zu erzeugen, welche in einem nachfolgenden Schritt in tertiäre Aminostrukturen umgewandelt werden können. Geeignete Ausgangsstoffe

zur Erzeugung reaktiver Zwischenstufen sind beispielsweise halogenierte Alkene, bevorzugt Allylchlorid und Allylbromid, ungesättigte Halogencarbonsäureester, bevorzugt Chloressigsäureallylester, Chloressigsäurepropargylester, 3-Chlorpropionsäureallylester und 3-Chlorpropionsäurepropargylester und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B.Marciniec, Comprehensive Handbook on Hydrosilylation, Pergamon Press, Oxford 1992, S. 116-121, 127-130, 134-137, 151-155).

In einem nachfolgenden Schritt können die reaktiven Zwischenstufen dann mit sekundären Aminofunktionen tragenden Verbindungen zur Reaktion gebracht werden. Geeignete Vertreter sind N,N-Dialkylamine, beispielsweise Dimethylamin, Diethylamin, Dibutylamin, Diethanolamin und N-Methylglucamin, cyclische sekundäre Amine, beispielsweise Morpholin und Piperidin, sekundäre Aminofunktionen tragende Aminoamide, beispielsweise die Umsetzungsprodukte von Diethylentriamin oder Dipropylentriamin mit Lactonen, wie $\gamma$-Butyrolacton, Gluconsäure-$\delta$-lacton und Glucopyranosylarabonsäurelacton (DE-OS 4318536, Beispiele 11a, 12a, 13a) und sekundär-tertiäre Diamine, wie beispielsweise N-Methylpiperazin.

Bei Verwendung sekundär-tertiärer Diamine sollten bevorzugt Epoxidderivate als Partner zur Reaktion gebracht werden, da auf diese Weise Alkylierungen der tertiären Aminofunktion ohne zusätzlichen Aufwand ausgeschlossen werden können.

Bevorzugt werden Halogencarbonsäureester basierend auf niedermolekularen, oligomeren und polymeren Alkylenoxiden der allgemeinen Zusammensetzung

$$HO[CH_2CH_2O]_q\text{-}[CH_2CH(CH_3)O]_rR^{16}$$

worin q, r und $R^{16}$ die oben genannten Bedeutungen aufweisen, und bevorzugt monosubstituierten Derivate aus der Gruppe, bestehend aus Diethylenglycol, Triethylenglycol, Tetraethylenglycol, oder Oligoethylenglycole mit Molgewichten von 300 bis 1000 g/mol, und Dipropylenglycol verwendet werden.

Als Halogencarbonsäureester werden bevorzugt solche aus der Gruppe der Oligoethylenglycole mit Molgewichten von etwa 400, 600 und 800 g/mol (etwa 400 bis und 800 g/mol) verwendet.

D.h., die Einführung der Alkylenoxidblöcke gelingt bevorzugt über die entsprechenden Halogencarbonsäureester der Alkylenoxide. Bevorzugte Ausgangsmaterialien sind niedermolekulare, oligomere und polymere Alkylenoxide der allgemeinen Zusammensetzung

$$HO[CH_2CH_2O]_q\text{-}[CH_2CH(CH_3)O]_rR^{16}$$

wobei q, r und $R^{16}$ die oben angegebenen Bedeutungen aufweisen. Bevorzugte Vertreter sind die entsprechend monosubstituierten Derivate von Diethylenglycol, Triethylenglycol, Tetraethylenglycol, der Oligoethylenglycole mit Molgewichten von 300 bis 1000 g/mol, bevorzugt sind solche aus der Gruppe mit Molgewichten von 400, 600 und 800 g/mol, sowie Dipropylenglycol. Die Herstellung dieser Ether und Ester erfolgt in bekannter Weise durch sauer oder alkalisch katalysierte Addition von Ethylenoxid und/oder Propylenoxid an die entsprechenden Alkohole (Organikum, Organisch-chemisches Grundpraktikum, 17. Auflage, VEB Deutscher Verlag der Wissenschaften, Berlin 1988, S. 259; US 5625024) oder Carbonsäuren (E.Sung, W. Umbach, H. Baumann, Fette Seifen Anstrichmittel 73, 88 [1971]).

Die nachfolgende Synthese der Halogencarbonsäureester erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den $C_2$-$C_4$-Halogencarbonsäuren, deren Anhydriden oder Säurechloriden. Die selektive Synthese hydroxyfunktioneller Halogencarbonsäureester, in denen $R^4$ Wasserstoff entspricht, gelingt durch Addition von Ethylenoxid und/oder Propylenoxid an die entsprechenden Halogencarbonsäuren unter sauren Bedingungen.

Die in $\alpha,\omega$-Stellung tertiäre Aminogruppen aufweisenden Siloxanderivate und die Halogencarbonsäureesterderivate der Alkylenoxide werden bevorzugt in einem molaren Verhältnis von Siloxan zu dem Halogencarbonsäureester von 1 : 2 zur Reaktion gebracht. Bei dieser Arbeitsweise werden dem Wesen nach Produkte synthetisiert, in denen die Siloxan- und Alkylenoxidblöcke über quarternäre Ammoniumfunktionen miteinander verbunden sind.

Mit der gezielten Einführung z.B. adäquat substituierter Piperazinringe gelingt es, regioselektiv Produkte mit einem tertiären und einem quartären Stickstoffatom in der Polymerstruktur zu synthetisieren. Ein Überschuß an Halogencarbonsäureester führt in diesem Fall zu einer beginnenden Quartärnierung der verbliebenen tertiären Aminofunktionen.

In einer weiteren bevorzugten Ausführungsform der vorliegenden Erfindung können, mehrere Siloxankomponenten und/oder Alkylenoxidderivate unterschiedlicher jeweiliger Kettenlänge unter Beibehaltung der gewünschten Gesamtstöchiometrie zur Reaktion gebracht werden. Es folgt hieraus z.B. die Möglichkeit, eine gewünschte Siloxankettenlänge durch Einsatz einer einzigen Siloxankomponente oder aber durch gezielte Mischung mehrerer Siloxankomponenten einzustellen. Analog dazu ist es möglich, eine vorteilhafte durchschnittliche Alkylenoxidblocklänge in Form einer monomodalen, bimodalen oder polymodalen Verteilung darzustellen.

Als Anionen kommen die während der Quartärnierung gebildeten Halogenidionen, bevorzugt Chloridionen, in Betracht. Jedoch können durch Ionenaustausch auch andere Anionen eingeführt werden. Zu nennen sind beispielsweise Anionen, wie Carboxylate, Sulfonate, Sulfate, Polyethercarboxylate und Polyethersulfate.

Die Quartärnierungsreaktionen werden bevorzugt in polaren organischen Lösungsmitteln ausgeführt. Geeignet sind z.B. Alkohole, speziell Methanol, Ethanol, i-Propanol und n-Butanol, Glycole, wie Ethylenglycol, Diethylenglycol, Triethylenglycol, die Methyl-, Ethyl- und Butylether der genannten Glycole, 1,2-Propylenglycol und 1,3-Propylenglycol, Ketone, wie Aceton und Methylethylketon, Ester, wie Ethylacetat, Butylacetat und 2-Ethyl-hexylacetat, Ether, -wie Tetrahydrofuran und Nitroverbindungen, wie Nitromethan. Die Wahl des Lösungsmittels richtet sich im wesentlichen nach der Löslichkeit der Reaktionspartner und der angestrebten Reaktionstemperatur.

Die Reaktionen werden im Bereich von 20 °C bis 130 °C, vorzugsweise 40°C bis 100 °C ausgeführt.

Eine besondere Ausführungsform der Erfindung (die im folgenden als sogenannte dritte Ausführungsform der Erfindung bezeichnet wird) wird durch die Polysiloxan-Verbindungen der allgemeinen Formel (III) dargestellt:

$$-[K\text{-}S\text{-}K\text{-}N^3]_m- \qquad \text{(III)}$$

in der

S, K und m wie oben definiert sind,

$N^3$ ein organischer Rest, der mindestens eine quartäre Ammoniumgruppe enthält, der allgemeinen Formel

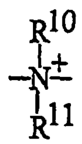

ist, worin $R^{10}$ ein einwertiger geradkettiger, cyclischer oder verzweigter $C_1$-$C_{30}$-Kohlenwasserstoffrest, der durch -O-, -NH-, -C(O)-, -C(S)- unterbrochen und mit -OH substituiert sein kann oder eine Einfachbindung zu K darstellt,

$R^{11}$= -A-E-$R^2$, worin-A-E-$R^2$ die oben genannte Bedeutung aufweist.

Bevorzugt handelt es sich bei den Polysiloxanverbindungen der dritten Ausführungsform der Erfindung um Alkylenoxidmodifizierte polyquarternäre Polysiloxane der allgemeinen Formel (III'),

$$-[K\text{-}S\text{-}K\text{-}N^3]_m- \qquad \text{(III')}$$

in der m 2 bis 500 ist,

S =

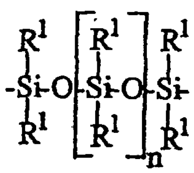

$R^1$ = $C_1$-$C_{22}$-Alkyl, $C_1$-$C_{22}$-Fluoralkyl oder Aryl,

n = 0 bis 1000,

$N^3$ = eine quartäre Ammoniumstruktur

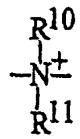

worin $R^{10}$ ein einwertiger oder zweiwertiger geradkettiger, cyclischer oder verzweigter $C_1$-$C_{30}$-Kohlenwasserstoffrest, der durch -O-, -NH-, -C(O)-, -C(S)- unterbrochen und mit -OH substituiert sein kann oder eine Einfachbindung zu K darstellt,
$R^3$ -A-E- ist, mit

A = $-CH_2C(O)O-$, $-CH_2CH_2C(O)O-$ oder $-CH_2CH_2CH_2C(O)O-$ und
E = eine Polyalkylenoxideinheit der Struktur

$$-[CH_2CH_2O]_q-[CH_2CH(CH_3)O]_r-R^{18}$$

q = 1 bis 200,
r = 0 bis 200,
$R^{18}$ = H, geradkettiger, cyclischer oder verzweigter $C_1$-$C_{20}$-Kohlenwasserstoffrest der durch -O-, oder -C(O)- unterbrochen und mit -OH substituiert und acetylenisch, olefinisch oder aromatisch sein kann, sowie
K = ein zweiwertiger oder dreiwertiger geradkettiger, cyclischer oder verzweigter $C_2$-$C_{40}$-Kohlenwasserstoffrest, der durch -O-, -NH-, $NR^1$-,

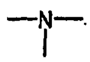,

-C(O)-, -C(S)- unterbrochen und mit -OH substituiert sein kann oder eine quartäre Ammoniumstruktur $N^5$ enthält, mit

$N^5$=

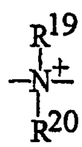

$R^{19}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann oder eine Einfachbindung zu $R^{10}$ darstellt, und
$R^{20}$ -A-E- ist, das wie oben definiert ist.

Bevorzugtere Ausführungsformen der sogenannten dritten Ausführungsform der Erfindung sowie Verfahren zur Herstellung der genannten Polysiloxanverbindungen der Formel (III) bzw. (III') werden nachfolgend beschrieben.
$R^{10}$ und $R^{19}$ sind unabhängig voneinander bevorzugt $-CH_3$, $-CH_2CH_3$, $-(CH_2)_2CH_3$, $-(CH_2)_3CH_3$, $-(CH_2)_5CH_3$, $-CH_2CH_2OH$,

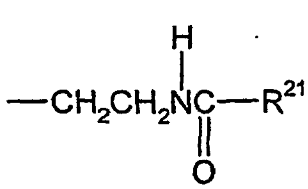

oder

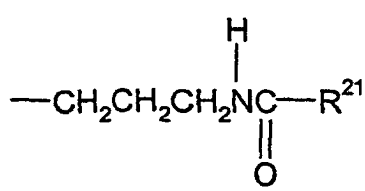

,

worin $R^{21}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann ist.

In einer Ausführungsform der sogenannten dritten Ausführungsform der Erfindung handelt es sich bei einer zweiwertige Substruktur für $R^{10}$ um eine ein cyclisches System bildende Struktur, wobei $R^{10}$ dann eine Einfachbindung zu K besitzt, bevorzugt zu einer tertiären Aminostruktur oder aber zur quartären Struktur $N^5$ über $R^{19}$.

Zu den bevorzugten Bedeutungen von $R^1$ in der sogenannten dritten Ausführungsform der Erfindung kann zu den obigen Ausführungen verwiesen werden.

Bevorzugt ist $R^{10}$ ein einwertiger oder zweiwertiger geradkettiger, cyclischer oder verzweigter $C_1$-$C_{25}$-Kohlenwasserstoffrest ist, der durch -O-, -NH-, -C(O)-, -C(S)-unterbrochen und mit -OH substituiert sein kann

Bevorzugt ist $R^{19}$ ein einwertiger oder zweiwertiger geradkettiger, cyclischer oder verzweigter $C_1$-$C_{25}$-Kohlenwasserstoffrest ist, der durch -O-, -NH-, -C(O)-, -C(S)-unterbrochen und mit -OH substituiert sein kann.

In der sogenannten dritten Ausführungsform der Erfindung ist K weiterhin bevorzugt ein zweiwertiger oder dreiwertiger geradkettiger,.cyclischer oder verzweigter $C_3$-$C_{30}$-Kohlenwasserstoffrest, der durch -O-, -NH-, -$NR^1$-,

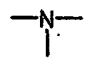 ,

-C(O)-, -C(S)-unterbrochen und mit -OH substituiert sein kann, noch bevorzugter ist K

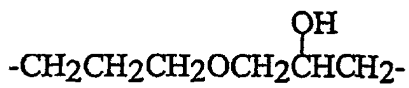

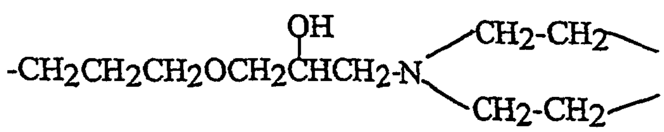

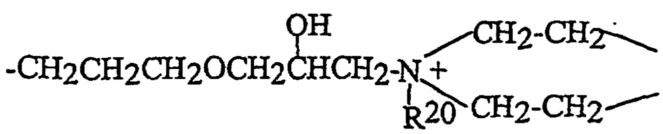

worin $R^{20}$ wie oben definiert ist.

In der sogenannten dritten Ausführungsform der Erfindung ist $R^2$ bzw. $R^{18}$ bevorzugt ein geradkettiger, cyclischer oder verzweigter $C_1$-$C_{18}$-Kohlenwasserstoffrest, der durch -O- oder -C(O)- unterbrochen und -OH substituiert und acetylenisch oder olefinisch sein kann. Bevorzugter ist $R^2$ bzw. $R^{18}$ $C_1$-$C_6$-Alkyl, $-CH_2CH=CH_2$, $-CH_2CH(OH)CH_2OCH_2CH=CH_2$, $-CH_2C\equiv CH$, $-C(O)CH_3$ oder $-C(O)CH_2CH_3$.

Bevorzugt ist $R^{21}$ ein unsubstituierter $C_5$-$C_{17}$-Kohlenwasserstoffrest, der sich von den entsprechenden Fettsäuren ableitet oder aber hydroxylierte $C_3$-$C_{17}$-Reste aufweist, und aus der Gruppe von hydroxylierten Carbonsäuren, bevorzugt Saccharidcarbonsäuren stammt.

So ist $R^{21}$ beispielsweise:

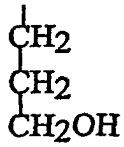 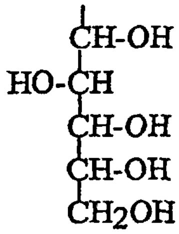 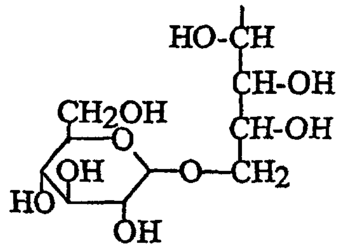

In der sogenannten dritten Ausfeihnmgsform der Erfindung ist m bevorzugt 2 bis 100, und besonders bevorzugt 2 bis 50, n ist 0 bis 100, bevorzugt 0 bis 80, und besonders bevorzugt 10 bis 80, q ist 1 bis 50, bevorzugt 2 bis 50 besonders bevorzugt 2 bis 20, und noch bevorzugter ist q 2 bis 10, r ist 0 bis 100, bevorzugt 0 bis 50, besonders bevorzugt 0 bis 20, und noch bevorzugter ist r 0 bis 10.

Die erfindungsgemäßen Polysiloxan-Verbindungen der sogenannten dritten Ausführungsform der Erfindung können zweckmäßig hergestellt werden, in dem tertiäre Aminofunktionen tragende Polymere der allgemeinen Struktur

$$-[K\text{-}S\text{-}K\text{-}T]_m-$$

mit T =

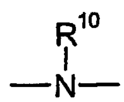

worin K, S, $R^{10}$ und m die oben angegebenen Bedeutungen haben, mit Halogencarbonsäureestern zu quarternären Ammoniumverbindungen umgesetzt werden, die sich von Alkylenoxidderivaten der allgemeinen Struktur

$$HO[CH_2CH_2O]_q\text{-}[CH_2CH(CH_3)O]_rR^2$$

worin q, r und $R^2$ die oben angegebenen Bedeutungen aufweisen, ableiten, und die molare Menge an Halogencarbonsäureester 5% der molaren Menge an tertiären Aminofunktionen ist.

Bevorzugt ist dabei die molare Menge an Halogencarbonsäureester 25%, besonders bevorzugt 50%, ganz besonders bevorzugt 70%, zur molaren Menge an tertiären Aminofunktionen.

Bevorzugt werden äquimolare Mengen an Halogencarbonsäureester verwendet.

Die Herstellung der tertiäre Aminofunktionen tragenden Polymere der allgemeinen Struktur

$$-[K\text{-}S\text{-}K\text{-}T]_m-$$

mit T =

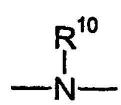

worin K, S, $R^{10}$ und m die oben angegebenen Bedeutungen haben, erfolgt bevorzugt durch Alkylierung von durch Hydrosilylierung sekundäre Aminofunktionen tragender ungesättigter Strukturen aus der Gruppe bestehend aus N-Methylallylamin oder $CH_2{=}CHCH_2OCH_2CH(OH)CH_2NHCH_3$, mit $\alpha,\omega$ Si-H funktionalisierten Siloxanen der allgemeinen Struktur

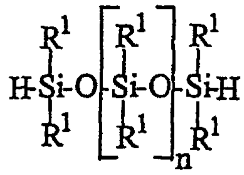

worin $R^1$ und n die oben angegebenen Bedeutungen haben, gewonnener α,ω sekundär aminomodifizierter Siloxane mit reaktiven, alkylierenden Siloxanzwischenprodukten, die durch Hydrosilylierung von halogenierten Alkenen, ungesättigten Halogencarbonsäureestern, und epoxyfunktionellen Alkenen, mit den α,ω Si-H funktionalisierten Siloxanen der allgemeinen Struktur

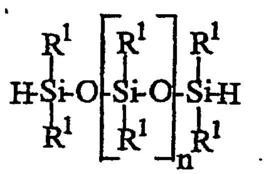

umgesetzt werden, wobei die Stöchiometrie von der sekundären Aminogruppen zu den alkylierenden Gruppen 1 : 1 beträgt.

Weiterhin bevorzugt erfolgt die Herstellung der die tertiäre Aminofunktionen tragenden Polymeren der allgemeinen Formel (III) bzw. (III'), worin K, S, $R^{10}$ und m die oben angegebenen Bedeutungen haben, durch Alkylierung von primären Aminen, bespielsweise Methylamin, mit den reaktiven, alkylierenden Siloxanzwischenprodukten, die durch Hydrosilylierung von halogenierten Alkenen, ungesättigten Halogencarbonsäureesternund epoxyfunktionellen Alkenen mit den α,ω Si-H funktionalisierten Siloxanen der allgemeinen Struktur

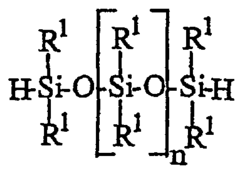

hergestellt werden, wobei die Stöchiometrie von dem primären Amin zu der alkylierenden Gruppen 1 : 2 beträgt.

Bevorzugt werden als halogenierte Alkene bevorzugt Allylchlorid und Allylbromid verwendet.

Bevorzugt werden als ungesättigte Halogencarbonsäureester solche aus der Gruppe bestehend aus Chloressigsäureallylester, Chloressigsäurepropargylester, 3-Chlorpropionsäureallylester und 3-Chlorpropionsäurepropargylester verwendet.

Bevorzugt werden als epoxyfunktionelle Alkene bevorzugt Vinylcyclohexenoxid und Allylglycidether verwendet.

Weiterhin bevorzugt werden die tertiären Aminofunktionen tragenden Polymeren der allgemeinen Formel (III) bzw. (III') worin K, S, $R^{10}$ und m die oben angegebenen Bedeutungen haben, durch Alkylienmg von difunktionellen sekundären Aminen oder entsprechende sekundär aminofunktionellen Aminoamiden mit den α,ω Si-H funktionalisierten Siloxanen der allgemeinen Struktur

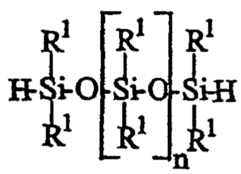

hergestellt, wobei die Stöchiometrie von der sekundären Aminogruppen zu der alkylierenden Gruppen 1 : 1 beträgt.

Bei den vorstehend beschriebenen Ausführungsformen zur Herstellung der Polysiloxanverbindungen der sogenannten dritten Ausführungsform der Erfindung beruhen die Halogencarbonsäureester auf niedermolekularen, oligomeren und polymeren Alkylenoxiden der allgemeinen Zusammensetzung

$$HO[CH_2CH_2O]_q\text{-}[CH_2CH(CH_3)O]_rR^2$$

worin q, r und $R^2$ die oben angegebenen Bedeutungen aufweisen, und es werden die entsprechend monosubstituierten Derivate von Diethylenglycol, Triethylenglycol, Tetraethylenglycol, der Oligoethylenglycole mit Molgewichten von 300 bis 1000 g/mol, bevorzugt mit Molgewichten von 400, 600 und 800 g/mol (400 bis 800 g/mol), sowie Dipropylenglycol verwendet.

Eine besondere Ausfübrungsform der Erfindung (die im folgenden als sogenannte vierte Ausführungsform der Erfindung bezeichnet wird) wird durch die Polysiloxan-Verbindungen der allgemeinen Formel (IV) dargestellt:

$$\text{-}[N^4\text{-K-S-K-}N^4\text{-A-E-A'}]_m\text{- bzw. -}[N^4\text{-K-S-K-}N^4\text{-A'-E-A}]_m\text{-} \quad \text{(IV)}$$

worin

m, K, S, -A-E-A'- und -A'-E-A- wie oben definiert sind, und

$N^4$ ein organischer Rest, der mindestens eine quartäre Ammoniumgruppe enthält, der allgemeinen Formel

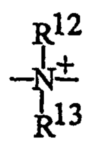

ist, worin $R^{12}$ ein einwertiger oder zweiwertiger geradkettiger, cyclischer oder verzweigter $C_1$-$C_{20}$-Kohlenwasserstoffrest ist, der durch -O-, -NH-, -C(O)-, -C(S)-unterbrochen und mit -OH substituiert sein kann,

$R^{13}$ die Bedeutungen von $R^{12}$ aufweisen kann, oder eine Einfachbindung zu K oder $R^{12}$ darstellt, und die Reste $R^{12}$ und $R^{13}$ gleich oder verschieden voneinander sein können.

Bevorzugt handelt es sich bei den Polysiloxanverbindungen der vierten Ausführungsform der Erfindung um Alkylenoxidmodifizierte polyquarternäre Polysiloxane der allgemeinen Formel (IV'),

$$\text{-}[N^4\text{-K-S-K-}N^4\text{-A-E A}]_m\text{-} \quad \text{(IV')}$$

worin m = 2 bis 500,

S =

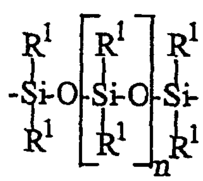

worin

$R^1$ = $C_1$-$C_{22}$-Alkyl, $C_2$-$C_{22}$-Fluoralkyl oder Aryl,

n = 0 bis 1000,

K einen zweiwertigen oder dreiwertigen geradkettigen, cyclischen oder verzweigten $C_2$-$C_{20}$-Kohlenwasserstoffrest darstellt, der durch -O-, -NH-, -$NR^1$-,

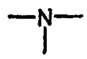

,

-C(O)-, -C(S)- unterbrochen und mit -OH substituiert sein kann,

N eine quartäre Ammoniumstruktur

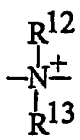

ist, worin $R^{12}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)-unterbrochen und mit -OH substituiert sein kann,

$R^{13}$ = $R^{12}$ oder eine Einfachbindung zu K oder $R^{12}$ darstellt,
A = $-CH_2C(O)O-$, $-CH_2CH_2C(O)O-$ oder $-CH_2CH_2CH_2C(O)O-$
E = eine Polyalkylenoxideinheit der Struktur

$$-[CH_2CH_2O]_q-[CH_2CH(CH_3)O]_r-$$

mit

q = 1 bis 200 und
r = 0 bis 200.

Bevorzugtere Ausführungsformen dieser sogenannten vierten Ausführungsform der Erfindung sowie Verfahren zur Herstellung der genannten Polysiloxanverbindungen der Formel (IV) bzw. (IV') werden nachfolgend beschrieben.

Die Möglichkeit einer dreiwertigen Substruktur für K bedeutet, daß K verzweigt sein kann und dann mit zwei Bindungen an der Quartärnierung von $N^4$ beteiligt sein kann.

Die Möglichkeit einer zweiwertigen Substruktur für $R^{12}$ bedeutet, daß es sich in diesen Fällen um eine cyclische Systeme bildende Struktur handelt, wobei $R^{13}$ dann eine Einfachbindung zu $R^{12}$ ist.

$R^{12}$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$,

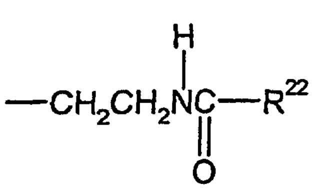

oder

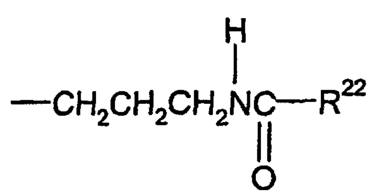

,

worin $R^{22}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.

$R^{12}$ und $R^{13}$ können wie vorstehend erwähnt auch gemeinsam eine cyclische Struktur der Formeln

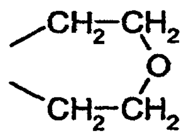 oder 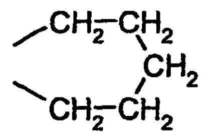

bilden.

Zu den bevorzugten Bedeutungen von $R^1$ in der sogenannten vierten Ausführungsform der Erfindung kann zu den vorstehenden Ausführungen verwiesen werden. Bevorzugt ist $R^{12}$ ein einwertiger oder zweiwertiger geradkettiger, cyclischer oder verzweigter $C_1$-$C_{16}$-Kohlenwasserstoffrest, der durch -O-, -NH-, -C(O)-, -C(S)-unterbrochen und mit -OH substituiert sein kann.

In der sogenannten vierten Ausführungsform ist K bevorzugt ein zweiwertiger oder dreiwertiger geradkettiger, cyclischer oder verzweigter $C_3$-$C_{16}$-Kohlenwasserstoffrest, der durch -O-, -NH-, -$NR^1$-,

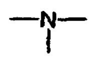 ,

-C(O)-, -C(S)- unterbrochen und mit -OH substituiert sein kann, besonders bevorzugt ist K

$-CH_2CH_2CH_2-$

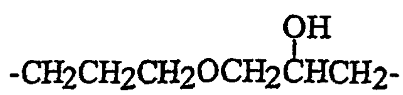

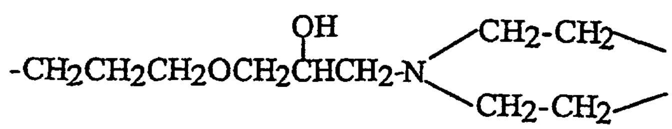

Bevorzugt ist $R^{22}$ ein unsubstituierter $C_5$-$C_{17}$-Kohlenwasserstoffrest, der sich von den entsprechenden Fettsäuren ableitet oder aber hydroxylierte $C_3$-$C_{17}$-Reste aufweist, die auf hydroxylierte Carbonsäuren, bevorzugt Saccharidcarbonsäuren zurückgeführt werden können.

Bevorzugter ist $R^{22}$:

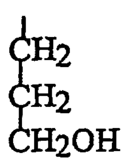 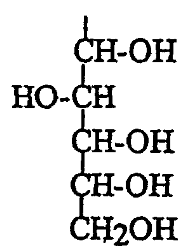 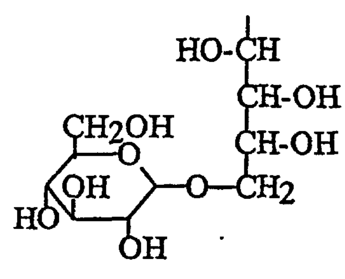

m ist bevorzugt 2 bis 100, und besonders bevorzugt 2 bis 50. n ist 0 bis 100, bevorzugt 0 bis 80, und besonders bevorzugt 10 bis 80. q ist 1 bis 50, bevorzugt 2 bis 50, und besonders bevorzugt 2 bis 20, noch bevorzugter ist q 2 bis 10. r ist 0 bis 100, bevorzugt 0 bis 50, und besonders bevorzugt 0 bis 20, noch bevorzugter ist r 0 bis 10.

Der Begriff "$C_1$-$C_{22}$-Alkyl oder $C_1$-$C_{30}$-Kohlenwasserstoffrest", wie er vorstehend verwendet wird, bedeutet im Rahmen der vorliegenden Erfindung aliphatische Kohlenstoffwasserstoffverbindungen mit 1 bis 22 Kohlenstoffatomen bzw. 1 bis 30 Kohlenstoffatomen die geradkettig oder verzweigt sein können. Beispielhaft seien Methyl, Ethyl,

Propyl, n-Butyl, Pentyl, Hexyl, Heptyl, Nonyl, Decyl, Undecyl, iso-Propyl, Neopentyl, und 1,2,3 Trimethylhexyl aufgeführt.

Der Begriff "$C_1$-$C_{22}$-Fluoralkyl" bedeutet, wie er vorstehend verwendet wird, im Ramen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monoffuormethyl, Monofluorethyl, 1,1,1-Trifluorethyl, Perflourethyl, 1,1,1-Trifluorpropyl, 1,2,2-Triflourbutyl aufgeführt.

Der Begriff "Aryl ", wie er vorstehend verwendet wird, bedeutet im Rahmen der vorliegenden Erfindung unsubstituierte oder ein oder mehrfach mit OH, F, CL, $CF_3$ $C_1$-$C_6$-Alkyl, $C_1$-$C_6$-Alkoxy, $C_3$-$C_7$-Cycloalkyl $C_2$-$C_6$-Alkenyl oder Phenyl substituiertes Phenyl. Der Ausdruck kann gegebenenfalls auch Naphthyl bedeuten.

Die erfindungsgemäßen Polysiloxan-Verbindungen der sogenannten vierten Ausführungsform der Erfindung können zweckmäßig hergestellt werden, in dem in α,ω-Stellung tertiäre Aminogruppen aufweisenden Siloxanderivate und die in α,ω-Halogencarbonsäureesterfunktionen tragenden Alkylenoxide in einem äquimolaren Verhältnis Siloxan zu Halogencarbonsäureester zur Reaktion gebracht werden.

Bevorzugt können die in α,ω-Stellung tertiäre Aminogruppen aufweisenden Siloxanderivate durch Hydrosilylierung von tertiäre Aminogruppen tragenden ungesättigten Strukturen, mit α,ω Si-H funktionalisierten Siloxanen der allgemeinen Struktur

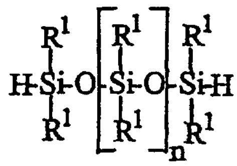

worin $R^1$ und n die oben angegebenen Bedeutungen haben, hergestellt werden.

Weiterhin können die in α,ω-Stellung tertiäre Aminogruppen aufweisenden Siloxanderivate in einem Zweistufenprozeß durch Hydrosilylierung von halogenierten Alkenen, ungesättigten Halogencarbonsäureestern und epoxylünktionellen Alkenen, mit α,ω Si-H funktionalisierten Siloxanen der allgemeinen Struktur

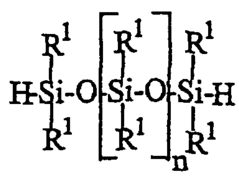

worin $R^1$ und n die oben angegebenen Bedeutungen haben, und nachfolgender Alkylierung von sekundäre Aminofunktionen tragenden Verbindungen, aus der Gruppe bestehend aus N,N-Dialkylaminen, cyclischen sekundären Aminen, sekundäre Aminofunktionen tragenden Aminoamiden, und sekundär-tertiären Diaminen, umgesetzt werden.

Als halogenierte Alkene werden dabei bevorzugt Allylchlorid und Allylbromid verwendet.

Als ungesättigte Halogencarbonsäureester werden solche aus der Gruppe bestehend aus Chloressigsäureallylester, Chloressigsäurepropargylester, 3-Chlorpropionsäureallylester und 3-Chlorpropionsäurepropargylester verwendet.

Als epoxyfunktionelle Alkene werden bevorzugt Vinylcyclohexenoxid und Allylglycidether verwendet.

Als N,N Dialkylaminen werden bevorzugt Dimethylamin, Diethylamin, Dibutylamin, Diethanolamin und N-Methylglucamin verwendet.

Als cyclische sekundäre Amine werden bevorzugt Morpholin und Piperidin verwendet werden.

Als sekundäre Aminofunktionen tragenden Aminoamide werden bevorzugt Umsetzungsprodukte von Diethylentriamin oder Dipropylentriamin mit Lactonen, bevorzugt y-Butyrolacton, Gluconsäure-δ-lacton und Glucopyranosylarabonsäurelacton verwendet.

Bevorzugt werden als α,ω--Halogencarbonsäureester solche, die auf niedermolekularen, oligomeren und polymeren Alkylenoxiden der allgemeinen Zusammensetzung

$$HO[CH_2CH_2O]_q\text{-}[CH_2CH(CH_3)O]_rR^2$$

basieren, worin q, r und $R^2$ die Bedeutungen gemäß Anspruch 1 aufweisen, und bevorzugt monosubstituierten Derivate aus der Gruppe bestehend aus Diethylenglycol, Triethylenglycol, Tetraethylenglycol, oder Oligoethylenglycole mit Molgewichten von 300 bis 1000 g/mol, und Dipropylenglycol verwendet, bevorzugter werden solche aus der Gruppe der Oligoethylenglycole mit Molgewichten von etwa 400, 600 und 800 g/mol (400 - 800 g/mol) verwendet.

So werden für die Synthesen der erfindungsgemäßen Verbindungen gemäß der vierten Ausführungsform der Erfindung α,ω Si-H funktionalisierte Siloxane der allgemeinen Struktur

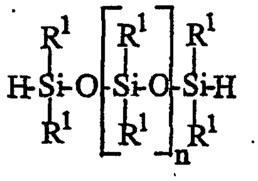

verwendet, worin $R^1$ und n die oben angegebenen Bedeutungen haben. Sofern nicht kommerziell erhältlich, können diese Siloxane nach bekannten Verfahren, z.B. durch Äquilibrierung hergestellt werden (Silicone, Chemie und Technologie, Vulkan-Verlag, Essen 1989, S. 82-84).

Die anschließende Einführung tertiärer Aminofunktionen kann beispielsweise auf zwei Wegen erfolgen. Einerseits ist es möglich, tertiäre Aminofunktionen tragende ungesättigte Strukturen, beispielsweise N,N-Dimethylallylamin, durch Hydrosilylierung direkt an das Siloxan zu binden. Dieser Prozeß ist allgemein bekannt und in B.Marciniec, Comprehensive Handbook on Hydrosilylation, Pergamon Press, Oxford 1992, S. 122-124 beschrieben.

Andererseits ist es bevorzugt, durch Hydrosilylierung zunächst reaktive Zwischenprodukte zu erzeugen, welche in einem nachfolgenden Schritt in tertiäre Aminostrukturen umgewandelt werden können. Geeignete Ausgangsstoffe zur Erzeugung reaktiver Zwischenstufen sind beispielsweise halogenierte Alkene, bevorzugt solche aus der Gruppe von Allylchlorid und Allylbromid, ungesättigte Halogencarbonsäureester, bevorzugt solche aus der Gruppe bestehend aus Chloressigsäureallylester, Chloressigsäurepropargylester, 3-Chlorpropionsäureallylester und 3-Chlorpropionsäurepropargylester; und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B.Marciniec, Comprehensive Handbook on Hydrosilylation, Pergamon Press, Oxford 1992, S. 116-121, 127-130, 134-137,151-155).

In einem nachfolgenden Schritt können die reaktiven Zwischenstufen dann mit sekundäre Aminofunktionen tragenden Verbindungen zur Reaktion gebracht werden. Geeignete Vertreter sind N,N-Dialkylamine bevorzugt solche aus der Gruppe bestehend aus Dimethylamin, Diethylamin, Dibutylamin, Diethanolamin und N-Methylglucamin, oder cyclische sekundäre Amine, beispielsweise Morpholin und Piperidin, sekundäre Aminofunktionen tragende Aminoamide, beispielsweise Umsetzungsprodukte von Diethylentriamin oder Dipropylentriamin mit Lactonen, wie γ-Butyrolacton, Gluconsäure-δ-lacton und Glucopyranosylarabonsäurelacton (wie sie in der DE-OS 4318536, in den Beispielen 11a, 12a, 13a beschrieben sind) und sekundär-tertiäre Diamine, wie beispielsweise N-Methylpiperazin.

Bei Verwendung sekundär-tertiärer Diamine sollten bevorzugt Epoxidderivate als Partner zur Reaktion gebracht werden, da auf diese Weise Alkylierungen der tertiären Aminofunktion ohne zusätzlichen Aufwand ausgeschlossen werden können.

Die Einführung der Alkylenoxidblöcke gelingt über die entsprechenden α,ω-Halogencarbonsäureester der Alkylenoxide. Bevorzugte Ausgangsmaterialien sind niedermolekulare, oligomere und polymere Alkylenoxide der allgemeinen Zusammensetzung

$$HO[CH_2CH_2O]_q\text{-}[CH_2CH(CH_3)O]_rH$$

worin q und r die oben angegebenen Bedeutungen aufweisen. Bevorzugte Vertreter sind Diethylenglycol, Triethylenglycol, Tetraethylenglycol, die Oligoethylenglycole mit Molgewichten von 300 bis 1000 g/mol, bevorzugt mit einem Molgewicht von etwa 400, 600 und 800 glmol (400 bis 800 glmol), sowie Dipropylenglycol.

Die Veresterung erfolgt in an sich bekannter Weise (Organikum, Organisch-chemisches Grundpraktikum, 17. Auflage, VEB Deutscher Verlag der Wissenschaften, Berlin 1988, S. 402-408) durch Reaktion mit den $C_2$ bis $C_4$ Halogencarbonsäuren, deren Anhydriden oder Säurechloriden.

Die in α,ω-Stellung tertiäre Aminogruppen aufweisenden Siloxanderivate und die α,ω-Halogencarbonsäureesterderivate der Alkylenoxide werden bevorzugt äquimolar zur Reaktion gebracht. Bei dieser Arbeitsweise werden dem Wesen nach Produkte synthetisiert, in denen die Siloxan- und Alkylenoxidblöcke über quarternäre Ammoniumfunktionen miteinander verbunden sind.

Mit der gezielten Einführung z.B. adäquat substituierter Piperazinringe gelingt es, regioselektiv Produkte mit einem tertiären und einem quarternären Stickstoffatom in der Polymerstruktur zu synthetisieren.

Ein Überschuß an α,ω-Halogencarbonsäureester führt durch beginnende Quartärnierung der verbliebenen tertiären Aminofunktion zu einer Vernetzung mit entsprechender Viskositätszunahme. Ein geringer Überschuß an α, ω-Aminosiloxan führt zu einer Abnahme des Polymerisationsgrades und damit einhergehend der Viskosität

Es liegt im Rahmen der Erfindung, mehrere Siloxankomponenten und/oder Alkylenoxidderivate unterschiedlicher jeweiliger Kettenlänge unter Beibehaltung der gewünschten Gesamtstöchiometrie zur Reaktion zu bringen. Es folgt hieraus z.B.die Möglichkeit, eine gewünschte Siloxankettenlänge durch Einsatz einer einzigen Siloxankomponente oder aber durch gezielte Mischung mehrerer Siloxankomponenten einzustellen. Analog dazu ist es möglich, eine vorteilhafte durchschnittliche Alkylenoxidblocklänge in Form einer monomodalen, bimodalen oder polymodalen Verteilung darzustellen.

Als Anionen kommen bevorzugt die während der Quartämiexung gebildeten Halogenidionen, speziell Chloridionen, in Betracht. Jedoch können durch Ionenaustausch auch andere Anionen eingeführt werden. Beispielhaft können Anionen aus der Gruppe bestehend aus Carboxylaten, Sulfonaten, Sulfaten, Polyethercarboxylaten und Polyethersulfaten aufgezählt werden.

Die Quartärnierungsreaktionen werden bevorzugt in polaren organischen Lösungsmitteln ausgeführt. Geeignet sind solche aus der Gruppe bestehend aus Alkoholen, Glycolen, Ketonen, Ester, Ether und Nitroverbindungen. Die Wahl des Lösungsmittels richtet sich wesentlich nach der Löslichkeit der Reaktionspartner und der angestrebten Reaktionstemperatur.

Für die Gruppe der Alkohole seien beispielhaft Methanol, Ethanol, i-Propanol und n-Butanol aufgeführt. Für die Gruppe der Glycole seien beispielhaft Ethylenglycol, Diethylenglycol, Triethylenglycol, die Methyl-, Ethyl- und Butylether der genannten Glycole, 1,2-Propylenglycol und 1,3-Propylenglycol, aufgeführt. Zur Gruppe der Ketone zählen z.B. Aceton oder Methylethylketon. Für die Gruppe der Ester seien beispielhaft Ethylacetat, Butylacetat und 2-Ethylhexylacetat augeführt. Zur Gruppe der Ether zählen solche wie Tetrahydrofuran und ähnliche. Zur Gruppe der Nitromethan sind solche aus der Gruppe von Nitromethan oder Nitroethan zu nennen.

Die Reaktionen werden im Bereich von 20 °C bis 130 °C, vorzugsweise 40 °C bis 100 °C ausgeführt

Eine besondere Ausführungsform der Erfindung (die im folgenden als sogenannte fünfte Ausführungsform der Erfindung bezeichnet wird) wird durch die Polysiloxan-Verbindungen der allgemeinen Formel (V) dargestellt:

$$[-N^5-F^1-N^5-Y-]_m \qquad (V)$$

worin

Y eine Gruppe der Formel

-K-S-K-

und

-A-E-A'- bzw. -A'-E-A-

ist, worin m, K, S, -A-E-A'- und -A'-E-A- wie oben definiert sind, die Gruppen K, S, -A-E-A'- und -A'-E-A- innerhalb der Polysiloxanverbindungen der allgemeinen Formel (V) gleich oder verschieden voneinander sein können, und das molare Verhältnis der Gruppe -K-S-K- und der Gruppe -A-E-A'- bzw. -A'-E-A- in der Polysiloxanverbindung der allgemeinen Formel (V) von 100 : 1 bis 1 : 100 ist,

$N^5$ eine Ammoniumgruppe der allgemeinen Formel

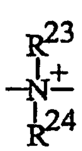

ist, worin

$R^{23}$ Wasserstoff, ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,

$R^{24}$ Wasserstoff, ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^{23}$ darstellt, und die Reste $R^{23}$ und $R^{24}$ innerhalb der Gruppe $-N^5-F^1-N^5-$ sowie in der Polysiloxanverbindung gleich oder verschieden voneinander sein können,

$F^1$ = ein zweiwertiger geradkettiger, cyclischer oder verzweigter Kohlenwasserstoffrest darstellt, der durch -O-, -NH-,

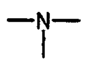 ,

-C(O)-, -C(S)- oder durch eine Gruppe -E- unterbrochen sein kann, worin E wie oben definiert ist,

und worin eine Mehrzahl von $N^5$ und $F^1$ jeweils gleich oder verschieden voneinander sein können.

Das molare Verhältnis der Gruppe -K-S-K- und der Gruppe -A-E-A'- bzw. -A'-E-A- in der Polysiloxanverbindung der allgemeinen Formel (V) liegt zwischen 100 : 1 und 1 : 100. Dieses molare Verhältnis kann wie unten gezeigt durch Wahl des molaren Verhältnisses der Ausgangsverbindungen, insbesondere des Verhältnisses der erfindungsgemäß bevorzugt verwendeten α,ω-Halogencarbonsäurepolyalkylenoxidester-Verbindungen und der Polysiloxan-Bisepoxid-Verbindungen gesteuert werden. Die Eigenschaften der Produkte hängen wesentlich vom verwendeten Verhältnis der Ausgangsmaterialien, sowie der Länge der darin enthaltenen Polyalkylenoxid- bzw. Polysiloxanblöcke ab.

In einer bevorzugten Ausführungsform der sogenannten fünften Ausführungsform der Erfindung ist K ein zweiwertiger Kohlenwasserstofftrest mit mindestens 4 Kohlenstoffatomen ist, der eine Hydroxylgruppe aufweist und der durch ein Sauerstoffatom unterbrochen sein kann.

In einer bevorzugten Ausführnmgsform der sogenannten fünften Ausführungsform der Erfindung ist $F^1$ ein zweiwertiger geradkettiger, cyclischer oder verzweigter $C_2$-$C_{30}$ Kohlenwasserstoffrest darstellt, der durch -O-, -NH-,

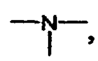 ,

-C(O)-, -C(S)- oder durch eine Gruppe -E- unterbrochen sein kann, worin E wie oben definiert ist, und worin die Kohlenstoffatome, die aus dem Rest E resultieren, nicht zu den 2 bis 30 Kohlenstoffatomen des $C_2$-$C_{30}$ Kohlenwasserstoffrest gezählt werden.

In einer weiteren bevorzugten Ausführungsform der sogenannten fünften Ausführungsform der Erfindung ist

$$-N^5-F^1-N^5-$$

eine Gruppe der Formel:

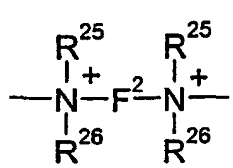

worin

$R^{25}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, besonders bevorzugt Methyl ist,

$R^{26}$ ein einwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, besonders bevorzugt Methyl ist, oder eine Einfachbindung zu einem zweiwertigen Rest $R^{25}$ darstellt, und die Reste $R^{25}$ und $R^{26}$ innerhalb der Gruppe -$N^5$-$F^2$-$N^5$- sowie in der Polysiloxanverbindung gleich oder verschieden voneinander sein können, und

$F^2$ = ein zweiwertiger geradkettiger, cyclischer oder verzweigter Kohlenwasserstoffrest ist, der durch -O-, -NH-,

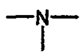 ,

-C(O)-, -C(S)-unterbrochen sein kann.

In einer noch bevorzugteren Ausführungsform ist $F^2$ eine verzweigte, bevorzugt geradkettige $C_1$-$C_6$-Alkandiyl-Gruppe, worunter eine 1,6-Hexandiyl- (bzw. Hexamethylen-) Gruppe bevorzugt ist.

In einer weiteren bevorzugten Ausführungsform der sogenannten fünften Ausführungsform der Erfindung ist

$$-N^5-F^1-N^5-$$

eine Gruppe der Formel:

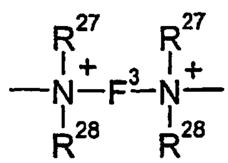

worin

$R^{27}$ und $R^{28}$ jeweils Wasserstoff, $C_1$-$C_6$-Alkyl oder Hydroxy($C_1$-$C_6$)alkyl, bevorzugt Wasserstoff, Methyl oder $-CH_2CH_2OH$ sind, und

$F^3$ ein zweiwertiger geradkettiger, cyclischer oder verzweigter Kohlenwasserstoffrest ist, der durch eine Gruppe -E- unterbrochen ist, worin E wie oben definiert ist.

$F^3$ ist besonders bevorzugt eine Gruppe der Formel

$$-D-E-D-$$

worin E wie oben definiert ist und D jeweils eine Einfachbindung oder eine geradkettige oder verzweigte $C_1$-$C_6$-Alkandiylgruppe ist, mit der Maßgabe, das D keine Einfachbindung ist, wenn es an ein endständiges Sauerstoffatom der Gruppe E bindet.

Bevorzugt wird die Gruppe -D-E-D- durch eine Gruppe der Formel

$$-D-(OCH_2CH_2)_v(OCH_2CH(CH_3))_w-O-D-$$

dargestellt, worin D eine geradkettige oder verzweigte $C_1$-$C_6$-Alkandiylgruppe ist und r und q wie oben definiert sind. In der Gruppe $-D-(OCH_2CH_2)_q(OCH_2CH(CH_3))_r-O-D$können die Ethylenoxid- und Propylenoxideinheiten beliebig angeordnet sein, z.B. als statistische Copolymereinheit oder als Blockcopolymereinheit.

v ist bevorzugt 1 bis 100, bevorzugter 1 bis 70, noch bevorzugter 1 bis 40.

w ist bevorzugt 0 bis 100, bevorzugter 0 bis 70, noch bevorzugter 0 bis 40.

In einer weiteren bevorzugten Ausfihrungsform der sogenannten fünften Ausführungsform der Erfindung wird die Gruppe

$$-N^5-F^1-N^5-$$

durch eine Gruppe der Formel:

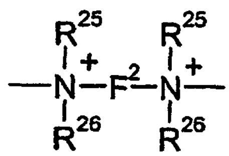

und eine Gruppe der Formel:

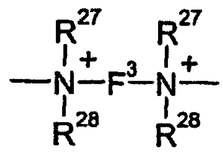

dargestellt, worin die Substituenten jeweils die vorstehenden Bedeutungen aufweisen.

Dies bedeutet, das die Polysiloxanverbindungen der allgemeinen Formel (V) aus zwei verschiedenen Typen der Gruppe -$N^5$-$F^1$-$N^5$- aufgebaut sind.

In dieser Ausführungsform beträgt das molare Verhältnis der Gruppe

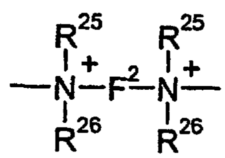

zur Gruppe

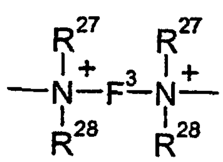

zweckmäßig 70 : 30 bis 95 : : 5, bevorzugt 80 : 20 bis 90 : 10.

Die Polysiloxanverbindungen der allgeimen Formel (V) können cylisch oder linear sein. Im Falle der linearen Verbindungen resultieren die endständigen Gruppen entweder aus den für die Herstellung verwendeten unten beschriebenen bifunktionellen Monomeren oder deren funktionalisierten Derivaten oder aus Monoaminen, die während der Polymerisation als Kettenabbruchmittel zugesetzt werden. Die aus der Verwendung der Monoamin-Kettenabbruchmittel resultierenden terminalen Gruppen, liegen bevorzugt als Ammoniumgruppen, entweder durch Quartärnierung oder Protonierung vor.

In einer weiteren bevorzugten Ausführungsform der sogenannten fünften Ausführungsform der Erfindung wird K durch Gruppen der Formel

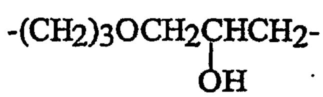 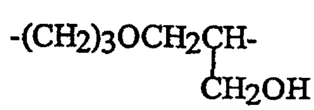

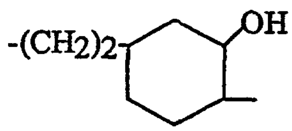

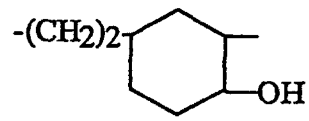

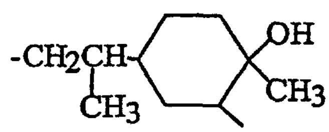

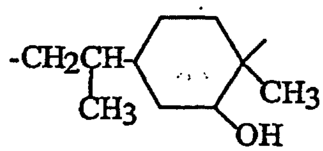

In der sogenannten fünften Ausführungsform der Erfindung liegt q bevorzugt im Bereich von 1 bis 50, insbesondere 2 bis 50, speziell 2 bis 20 und ganz speziell 2 bis 10, und r liegt im Bereich von 0 bis 100, insbesondere 0 bis 50, speziell 0 bis 20 und ganz speziell 0 bis 10,

In der sogenannten fünften Ausführungsform der Erfindung wird der organische oder anorganische Säurerest zur Neutralisation der aus der(n) Ammoniumgruppe(n) resultierenden Ladungen zweckmäßig ausgewählt aus anorganischen Resten, wie Chlorid, Bromid, Hydrogensulfat, Sulfat, bzw. organischen Resten, wie Acetat, Propionat, Octanoat, Decanoat, Dodecanoat, Tetradecanoat, Hexadecanoat, Octadecanoat und Oleat, wobei wie eingangs erwähnt Chlorid und Bromid bevorzugt aus der Umsetzung der Alkylhalogenidgruppen mit Amingruppen resultieren.

Weiterhin liegen die Polysiloxan-Verbindungen der fünften Ausführungsform der Erfindung in protonierter Form als Aminsalze oder als Amine vor.

Die Polysiloxan-Verbindungen der fünften Ausführungsform der Erfindung werden zweckmäßig hergestellt durch ein Verfahren, worin zunächst $\alpha,\omega$ Si-H funktionalisierte Siloxane der allgemeinen Struktur

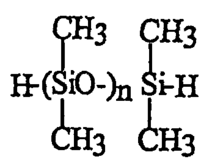

mit, bezogen auf SiH-Gruppen 1,0 bis 1,5 mol eines Alkenyl-Epoxides, welches eine endständige olefinische Bindung aufweist, wobei das Alkenyl-Epoxid mindestens 4 Kohlenstoffatome besitzt und zusätzlich eine nichtcyclische Ethergruppe enthalten kann, in Gegenwart eines Hydrosilylierungskatalysators bei Temperaturen von 50 bis 150°C umsetzt, der Überschuß an olefinischem Epoxid ggf. entfernt, und das Reaktionsprodukt mit einer Mischung aus einem Diamin, beispielsweise das Bevorzugte der Formel

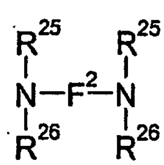

und einem $\alpha,\omega$-Halogencarbonsäureester, bevorzugt der Formel

Z-A-E-A'-Z bzw. Z-A-E-A'-Z

worin A-E-A' bzw. A'-E-A wie oben definiert sind und Z eine übliche nukleophile Abgangsgruppe, vorzugsweise Chlorid oder Bromid ist, mit der Maßgabe, dass Z an eine endständige $-CH_2$-Gruppe bindet,

in Gegenwart von Säure bei 40 bis 120°C umgesetzt wird, wobei das molare Verhältnis von ∑ (Epoxygruppen + Halogencarbonsäureestergruppen) : tertiäre Aminogruppen zweckmäßig ungefähr 1 1 und das molare Verhältnis von Epoxygruppen : Säure zweckmäßig ungefähr 1 : 1 ist.

Geeignete Säuren, die bei dieser und den nachfolgend beschriebenen Verfahren zur Herstellung der Polysiloxanverbindungen verwendet werden können, schließen beispielsweise organische oder anorganische Säuren, zweckmäßig in wässriger Lösung, ein, wie Halogenwasserstoffsäuren, wie Fluorwasserstoffsäure, Salzsäure, Bromwasserstoffsäure, Schwefelsäure, Salpetersäure, Phosphorsäure, Carbonsäuren, wie Ameisensäure, Essigsäure, Propansäure etc., Sulfonsäuren, Polyethercarbonsäure, etc. Bevorzugt werden $C_1$-$C_{30}$-Carbonsäuren, besonders bevorzugt $C_{10}$-$C_{18}$-Carbonsäuren. Über die Wahl der verwendeten Säuren können die Eigenschaften der Polysiloxanverbindungen weiter modifiziert werden.

Bezüglich der Herstellung der α,ω Si-H funktionalisierte Siloxane der allgemeinen Struktur

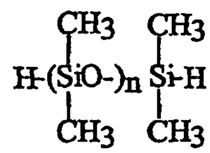

kann zu den oben beschriebenen Ausführungsformen verwiesen werden.

Ausgangsmaterialien zur Herstellung der bevorzugten α,ω-Halogencarbonsäureester, bevorzugt der Formel

Z-A-E-A'-Z bzw. Z-A-E-A'-Z

Worin Z bevorzugt Chlor, Brom ist,
sind zweckmäßig niedermolekulare, oligomere und polymere Alkylenoxide der allgemeinen Zusammensetzung

$$HO[CH_2CH_2O]_q\text{-}[CH_2CH(CH_3)O]_rH$$

wobei q und r die oben angegebenen Bedeutungen aufweisen. Bevorzugte Vertreter sind Diethylenglycol, Triethylenglycol, Tetraethylenglycol, die Oligoethylenglycole mit Molgewichten von 300 bis 1000g/mol, speziell etwa 400, etwa 600 und etwa 800, sowie Dipropylenglycol.

Die Veresterung erfolgt in an sich bekannter Weise, wobei zu den Schilderungen der obigen Ausführungsformen verwiesen werden kann.

Die Polysiloxan-Verbindungen der fünften Ausführungsform der Erfindung werden zweckmäßig weiterhin hergestellt durch ein Verfahren, worin zunächst α,ω Si-H funktionalisierte Siloxane der allgemeinen Struktur

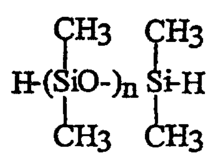

mit, bezogen auf SiH-Gruppen 1,0 bis 1,5 mol eines Alkenyl-Epoxides, welches eine endständige olefinische Bindung aufweist, wobei das Alkenyl-Epoxid mindestens 4 Kohlenstoffatome besitzt und zusätzlich eine nichtcyclische Ethergruppe enthalten kann, in Gegenwart eines Hydrosilylierungskatalysators bei Temperaturen von 50 bis 150°C umsetzt, der Überschuß an olefinischem Epoxid ggf. entfernt, und das Reaktionsprodukt mit einer Mischung aus Diaminen, beispielsweise die Bevorzugten der Formeln

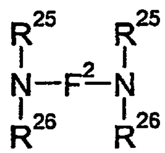

und

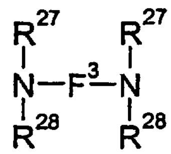

und einem α,ω-Halogencarbonsäureester, bevorzugt der Formel

Z-A-E-A'-Z bzw. Z-A-E-A'-Z

worin A-E-A' bzw. A'-E-A wie oben definiert sind und Z eine übliche nukleophile Abgangsgruppe, vorzugsweise Chlorid oder Bromid ist, mit der Maßgabe, dass Z an eine endständige -$CH_2$-Gruppe bindet,
in Gegenwart von Säure bei 40 bis 120°C umgesetzt wird, wobei das molare Verhältnis von ∑(Epoxygruppen + Halogencarbonsäureestergruppen) : ∑(primäre+ sekundäre+tertiäre) Aminogruppen zweckmäßig ungefähr 1 : 1 und das molare Verhältnis von Epoxygruppen : Säure zweckmäßig ungefähr 1 : 1 beträgt.

In den Verfahren zur Herstellung der Polysiloxan-Verbindungen der fünften Ausführungsform der Erfindung werden bevorzugt die verschiedenen Aminogruppen tragenden Spezies, die α,ω-Halogencarbonsäureester und eine zu den Aminogruppen äquimolare Menge an Säure gemeinsam dem Ansatz hinzugefügt werden.

In einer weiteren bevorzugten Ausführungsform des Verfahrens zur Herstellung der Polysiloxan-Verbindungen der fünften Ausführungsform der Erfindung werden zunächst die Epoxyderivate, die Halogencarbonsäureesterderivate und di-tertiäre Amine, bevorzugt der Formel

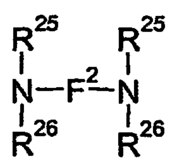

in Gegenwart einer zu den Epoxygruppen äquivalenten Menge an Säure zur Reaktion gebracht werden und nachfolgend die vorzugsweise primäre bzw. sekundäre Aminogruppen aufweisenden Alkylenoxidderivate, bevorzugt der Formel

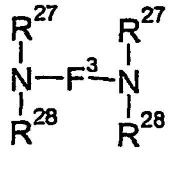

ggf. unter Zusatz von Säure bis zur Äquivalenz, der Reaktionsmischung hinzugefügt werden.
In einer weiteren bevorzugten Ausführungsform des Verfahrens zur Herstellung der Polysiloxan-Verbindungen der fünften Ausführungsform der Erfindung werden zunächst die Halogencarbonsäureesterderivate und di-tertiäre Amine, bevorzugt der Formel

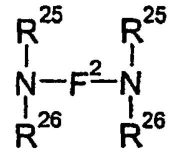

zur Reaktion gebracht werden und nachfolgend die Epoxyderivate, ggf. unter Zusatz von primären bzw. sekundären Aminogruppen aufweisenden Alkylenoxidderivaten, bevorzugt der Formel

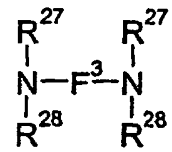

in Gegenwart einer zu den Epoxygruppen äquivalenten Menge an Säure der Reaktionsmischung zugesetzt werden.

In einer weiteren bevorzugten Ausführungsform des Verfahrens zur Herstellung der Polysiloxan-Verbindungen der fünften Ausführungsform der Erfindung können die alkylenoxidmodifizierten Diamine unter Beibehaltung der Gesamtstöchiometrie partiell durch trifunktionelle oder monofunktionelle alkylenoxidmodifizierte Amine ersetzt werden.

In einer weiteren bevorzugten Ausführungsform des Verfahrens zur Herstellung der Polysiloxan-Verbindungen der fünften Ausführungsform der Erfindung können die ditertiären Amine unter Beibehaltung der Gesamtstöchiometrie partiell durch monofunktionelle tertiäre Amine ersetzt werden.

In einer weiteren bevorzugten Ausführungsform des Verfahrens zur Herstellung der Polysiloxan-Verbindungen der fünften Ausführungsform der Erfindung können die $\alpha,\omega$-Halogencarbonsäureester unter Beibehaltung der Gesamtstöchiometrie partiell durch monofunktionelle Halogencarbonsäureester ersetzt werden können.

Die erfindungsgemäß in der fünften Ausführungsform der Erfindung verwendeten primären bzw. sekundären Aminogruppen aufweisenden Alkylenoxidderivaten, bevorzugt der Formel

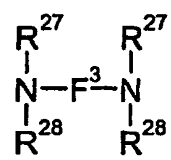

weisen beispielsweise die Formel

$$H(R^{29})N\text{-}D\text{-}(OCH_2CH_2)_v(OCH_2CH(CH_3))_w\text{-}O\text{-}D\text{-}N(R^{29})H,$$

worin D, v, w und $R^{29}$ die Bedeutungen von $R^{27}$ bzw. $R^{28}$ aufweisen kann, sind kommerziell unter der Bezeichnung Jeffamine® (Huntsman Corp.) erhältlich. Obwohl die Verwendung entsprechender Diaminoderivate bevorzugt ist, kann auch partiell auf analoge, trifunktionelle oder monofunktionelle Strukturen zurückgegriffen werden, wenn eine anteilige Vernetzung oder aber Kettenterminierung beabsichtigt ist. Der Anteil an trifunktionellen, vernetzenden bzw. monofunktionellen, kettenabbrechenden Aminoderivaten beträgt molar maximal 10%, vorzugsweise 5%, speziell 1% des Gehaltes an difunktionellem Derivat

Es liegt ebenfalls im Rahmen der vorstehend beschriebenen Ausführungsform, das di-tertiäre Amin anteilig durch monofunktionelle tertiäre Amine zu ersetzen. Deren Anteil beträgt molar ebenfalls maximal 10%, vorzugsweise 5%, vorzugsweise 1 % des Gehaltes an di-tertiärem Amin.

Es liegt ebenfalls im Rahmen der Erfindung, die $\alpha,\omega$-Halogencarbonsäureester durch monofunktionelle Halogencarbonsäureester zu ersetzen. Deren Anteil beträgt ebenfalls molar ebenfalls maximal 10%, vorzugsweise 5%, vorzugsweise 1% des Gehaltes an $\alpha,\omega$-Halogencarbonsäureester.

Hinsichtlich der Reaktionsführung liegt es im Rahmen der Erfindung, die Säure, die bei der Herstellung der Polysiloxanverbindungen verwendet wird, über das für die Quartärnierung der tertiären Aminogruppen hinausgehende Maß bis zur molaren Äquivalenz mit allen verbleibenden Aminogruppen, nach Abzug der durch die Halogencarbonsäureesterderivate alkylierten Aminogruppen, den Ansätzen hinzuzufügen. Dies bedeutet, daß die erfindungsgemäßen Polysiloxan-Verbindungen bezüglich der Struktur der Aminogruppen als freie Amine oder aber Aminsalze vorliegen können.

Wie vorstehend beschrieben, können die verschiedenen Aminogruppen tragenden Spezies in einer bevorzugten Ausführungsvariante, ggf. unter Hinzufügung äquimolarer Mengen an Säure gemeinsam mit dem Halogencarbonsäureesterderivaten dem Ansatz hinzugefügt werden. Es liegt aber auch im Rahmen der Erfindung, zunächst die Epoxydederivate, die Halogencarbonsäureesterderivate und die di-tertiären Amine in Gegenwart einer zu den Epoxygruppen äquivalenten Menge an Säure zur Reaktion zu bringen und ggf. nachfolgend auch primäre bzw. sekundäre Aminogruppen aufweisende Alkylenoxidderivate, ggf. unter Zusatz von Säure bis zur Äquivalenz mit den Aminogruppen, hinzuzufügen.

Es ist ebenfalls möglich, zunächst die Halogencarbonsäureesterderivate und die ditertiären Amine unter Bildung hydrophiler Blöcke zur Reaktion zu bringen und nachfolgend die Epoxyderivate, ggf. unter Zusatz von primäre bzw. sekundäre Aminogruppen aufweisenden Alkylenoxidderivaten, in Gegenwart einer zu den Epoxygruppen äquivalenten Menge an Säure der Reaktionsmischung zuzusetzen.

Über den Zugabezeitpunkt der einzelnen Komponenten kann die Sequenzverteilung im gebildeten Polymer beeinflußt werden.

Es ist weiterhin erfindungsgemäß, mehrere Siloxankomponenten und/oder Alkylenoxidderivate unterschiedlicher jeweiliger Kettenlänge unter Beibehaltung der gewünschten Gesamtstöchiometrie zur Reaktion zu bringen. Es folgt hieraus z.B. die Möglichkeit, eine gewünschte Siloxankettenlänge durch Einsatz einer einzigen Siloxankomponente oder aber durch gezielte Mischung mehrerer Siloxankomponenten einzustellen. Analog dazu ist es möglich, eine vorteilhafte durchschnittliche Alkylenoxidblocklänge in Form einer monomodalen, bimodalen oder polymodalen Verteilung darzustellen. Zusätzlich kann ein angestrebter Alkylenoxidanteil variabel auf die Halogencarbonsäureesterkomponente und die Aminokomponente verteilt werden.

Die Quartärnierungs- und Alkylierungsreaktionen werden bevorzugt in polaren organischen Lösungsmitteln ausgeführt. Geeignet sind z.B. Alkohole, speziell Methanol, Ethanol, i-Propanol und n-Butanol, Glycole, wie Ethylenglycol, Diethylenglycol, Triethylenglycol, die Methyl-, Ethyl- und Butylether der genannten Glycole, 1,2 Propylenglycol und 1,3-Propylenglycol, Ketone, wie Aceton und Methylethylketon, Ester, wie Ethylacetat, Butylacetat und 2 Ethyl-hexylacetat, Ether, wie Tetrahydrofuran und Nitroverbindungen, wie Nitromethan. Die Wahl des Lösungsmittels richtet sich wesentlich nach der Löslichkeit der Reaktionspartner und der angestrebten Reaktionstemperatur.

Die Reaktionen werden vorzugsweise im Bereich von 20°C bis 150°C, speziell 40°C bis 100°C ausgeführt.

Die Erfindung betrifft des weiteren die Verwendung der vorstehend beschriebenen Polysiloxanverbindungen in kosmetischen Formulierungen für die Haut- und Haarpflege, in Polituren für die Behandlung und Ausrüstung harter Oberflächen, in Formulierungen zum Trocknen von Automobilen und anderen harten Oberflächen, zum Beispiel nach maschinellen Wäschen, zur Ausrüstung von Textilien und Textilfasern, als separate Weichmacher nach dem Waschen von Textilien mit nichtionogenen oder anionischen/nichtionogen Detergenzienformulierungen, als Weichmacher in auf nichtionischen oder anionischen/nichtionischen Tensiden beruhenden Formulierungen zur Textilwäsche, sowie als Mittel zur Verhinderung bzw. Rückgängigmachung von Textilverknitterungen.

Die Erfindung betrifft des weiteren die Verwendung der vorstehend beschriebenen Polysiloxanverbindungen als waschbeständige hydrophile Weichmacher für die textile Erstausrüstung.

Ferner betrifft die Erfindung Zusammensetzungen, die mindestens eine der Polysiloxanverbindungen zusammen mit mindestens einem weiteren für die Zusammensetzung üblichen Inhaltsstoff enthält.

Im folgenden sind einige typische Beispiele für derartige Zusammensetzungen gegeben, in denen die Polysiloxanverbindungen der Erfindung vorteilhaft verwendet werden können:

Typische Hilfsstoffe in derartigen Zusammensetzungen sind z.B. diejenigen Stoffe, die in A. Domsch: Die kosmetischen Präparate Bd. I u. II 4. Aufl. Verl. für chem. Industrie, H. Ziolkowsky KG , Augsburg sowie International Cosmetic Ingredient Dictionary and Handbook 7$^{th}$ Ed. 1997 by J.A. Wenniger, G.N. McEwen Vol. 1-4 by The Cosmetic, Toiletry and Fragrance Association Washington DC bzw. unter http://www.cosmetic-world.com/inci /Incialf.htm beschrieben sind.

Anionisches Shampoo

Das Formulierungsbeispiel ist als Rahmenrezeptur gedacht Anionisches Shampoo enthalten üblicherweise die folgenden Komponenten, ohne auf diese beschränkt zu sein:

Alkylsulfate, Alkylethersulfate, Natriumlaurylsulfat, Natriumlauryl-ethersulfat, Ammoniumlaurylsulfat, Ammoniumlauryl-ethersulfat, TEA-laurylsulfat, TEA-laurylethersulfat, Alkylbenzolsulfonate, $\alpha$-Olefinsulfonate, Paraffinsulfonate, Sulfosuccinate, N-Acyltauride, Sulfat-glyceride, Sulfatierte Alkanolamide, Carboxylatsalze, N-acyl-Aminosäuresalze, Silicone, etc.

| Komponente | % |
|---|---|
| Ammoniumlaurylsulfat | 10.00 - 30.00 |
| Ammoniumlauryl-ethersulfat | 5.00 - 20.00 |
| Cocamidopropyl Betaine | 0.00 -15.00 |
| Lauramid DEA | 0.00 - 5.00 |
| Cocamid Mea | 0.00 - 5.00 |
| Dimethicone Copolyol (Dimethylsiloxanglykolcopolymer) | 0.00 - 5.00 |
| Cyclopentasiloxane | 0.00 - 5.00 |
| Erfindungsgemäße Polysiloxanverbindung | 0.50 - 5.00 |
| Polyquaternium-10 | 0.00 - 2.00 |
| Konservierungsmittel | 0.00 - 0.50 |
| Duftstoffe | 0.00 - 5.00 |
| Entionisiertes Wasser | q.s. 100% |
| Natriumchlorid | q.s. |

Nichtionisches Shampoo

Das Formulierungsbeispiel ist als Rahmenrezeptur gedacht Nichtionische Shampoos enthalten üblicherweise die folgenden Komponenten, ohne auf diese beschränkt zu sein:
Monoalkanolamide, Monoethanolamide, Monoisopropanolamide, Polyhydroxyderivative, Sucrosemonolaurat, Polyglycerinether, Aminoxide, Polyethoxylierte Derivative, Sorbitanderivative, Silicone, etc.

| Komponente | % |
|---|---|
| Lauramid DEA | 10.00 - 30.00 |
| Lauramid-Oxid | 5.00 - 20.00 |
| Cocamid Mea | 0.00 - 5.00 |
| Dimethicone Copolyol | 0.00 - 5.00 |
| Erfindungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

Amphoteres Shampoo

Das Formulierungsbeispiel ist als Rahmenrezeptur gedacht Formulierungen dieser Kategorie enthalten üblicherweise die folgenden Komponenten, ohne auf diese beschränkt zu sein:
N-Alkyl-iminodipropionate, N-Alkyl-iminopropionate, Aminosäuren, Aminosäurederivative, Amidobetaine, Imidazoliniumderivative, Sulfobetaine, Sultaine, Betaine, Silicone etc.

| Komponente | % |
|---|---|
| PEG-80-sorbitanlaurat | 10.00 - 30.00 |
| Lauroamphoglycinat | 0.00 -10.00 |
| Cocamidopropyl-Hydroxysultain | 0.00 -15.00 |
| PEG-150-distearat | 0.00 - 5.00 |
| Lanrylether-13-carboxylat | 0.00 - 5.00 |
| Erfindungsgemäße Polysiloxanverbindung | 0.50 - 5.00 |
| Duftstoffe | 0.00 - 5.00 |
| Entionisiertes Wasser | q.s. 100% |
| Natriumchlorid | q.s. |

Kationisches Shampoo

Das Formulierungsbeispiel ist nur als Rahmenrezeptur gedacht Formulierungen dieser Kategorie enthalten die folgenden Komponenten, ohne auf diese beschränkt zu sein:
Bis-Quartäre Ammoniumverbindungen, Bis-(trialkylammoniumacetyl)diamine, Amidoamine, Ammoniumalkylester, Silicone etc.

| Komponente | % |
|---|---|
| Laurylether-13-carboxylat | 10.00 - 30.00 |
| Isopropylmyristat | 5.00 - 20.00 |
| Cocamidopropyl-Betaine | 0.00 - -15.00 |
| Lauramid DEA | 0.00 - 5.00 |
| Cocamid MEA | 0.00 - 5.00 |
| Erfdungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

Festiger

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Ceteareth-20 | 0.10 - 10.00 |
| Steareth-20 | 0.10-10.00 |
| Stearyl-Alkohol | 0.10 -10.00 |
| Stearamidopropyl-Dimethylamin | 0.00-10.00 |
| Dicetyldimonium-Chlorid | 0.00 -10.00 |
| Erfindungsgemäße Polysiloxanverbindung | 0.50 - 5.00 |
| Cyclopentasiloxan | 0.00 - 5.00 |
| Dimethicone | 0.00 - 5.00 |
| Konservierungsmittel | 0.00 - 0.50 |
| Duftstoffe | 0.00 - 5.00 |
| Entionisiertes Wasser | q.s. 100% |

"Clear Rinse-Off"-Festiger

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Glycerin | 0.10-10.00 |
| Cetrimonium-Chlorid | 0.00 - 10.00 |

(fortgesetzt)

| Komponente | % |
|---|---|
| Erfindungsgemäße Polysiloxanverbindung | 0.50-5.00 |
| Hydroxyethylcellulose | 0.00 - 5.00 |
| Konservierungsmittel | 0.00 - 0.50 |
| Duftstoffe | 0.00 - 5.00 |
| Entionisiertes Wasser | q.s. 100% |

Schaumfestiger für Haare

Das Formulierungsbeispiel ist als Rahmenrezeptur gedacht. Formulierungen dieser Kategorie enthalten die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, Komprimierte Gase, etc.

| Komponente | % |
|---|---|
| Erfindungsgemäße Polysiloxanverbindung | 0.50 - 5.00 |
| Nonoxynol-15 | 0.00 - 2.00 |
| Nonoxynol-20 | 0.00 - 2.00 |
| Duftstoffe | 0.00 - 5.00 |
| Aereosoltreibmittel | 0.00 - 20.00 |
| Konservierungsmittel | 0.00 - 0.50 |
| Entionisiertes Wasser | q.s. 100% |

Pumpspray (Festiger) für Haare

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel Silicone Lösungsmittel, Ethanol, Isopropanol, Isoparaffinlösungsmittel, etc.

| Komponente | % |
|---|---|
| Erfindungsgemäße Polysiloxanverbindung | 0.50 - 5.00 |
| Cyclomethicone | 0.00 - 80.00 |
| Ethanol | 0.00 - 80.00 |
| Konservierungsmittel | 0.00 - 0.50 |
| Duftstoffe | 0.00 - 5.00 |
| Entionisiertes Wasser | q.s. 100% |

Festigerspray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, Komprimierte Gase, etc.

| Komponente | % |
|---|---|
| Erfindungsgemäße Polysiloxanverbindung | 0.50 - 5.00 |
| Cyclomethicone | 0.00 - 80.00 |
| Ethanol | 0.00 - 50.00 |
| Aereosoltreibmittel | 0.00 - 50.00 |
| Konservienmgsmittel | 0.00 - 0.50 |
| Duftstoffe | 0.00 - 5.00 |
| Entionisiertes Wasser | q.s. 100% |

Gelfestiger für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Verdickungsmittel, Cellulosederivative, Acrylsäurederivative, Fixativ-Polymere, Konditionierungschemikalien, Glykole, Glykolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Silicone, Lösungsmittel, Ethanol, Isopropanol, Isoparaffin-Lösungsmittel etc.

| Komponente | % |
|---|---|
| Erfindungsgemäße Polysiloxanverbindung | 0.50 - 5.00 |
| Hydroxyethylcellulose | 0.00 - 2.00 |
| Duftstoffe | 0.00 - 5.00 |
| Konservierungsmittel | 0.00 - 0.50 |
| Zitronensäure | 0.00 - 2.00 |
| Entionisiertes Wasser | q.s. 100% |

Styling Gel für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Acrylisäurederivative, Cellulosederivative, Vinylderivative, Konditionierungschemikalien, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffin-Lösungsmittel etc.

| Komponente | % |
|---|---|
| Erfindungsgemäße Polysiloxanverbindung | 0.50 - 5.00 |
| Fixative | 0.10-10.00 |
| Hydroxyethylcellulose | 0.00 - 2.00 |
| Duftstoffe | 0.00 - 5.00 |
| Zitronensäure | 0.00 - 2.00 |
| Entionisiertes Wasser | q.s. 100% |

Styling Spray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Vinylderivative, Fettsäuren, Fettsäureester, Ethoxylierte Fettsäuren, Ethoxylierte Fettsäureester, Fettalkohole, Ethoxylierte Fettalkohole, Glykole, Glykolester, Glycerin , Glycerin-Ester, Lanolin, Lanolinderivative, Mineral öl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel,

Ethanol, Isopropanol, Isoparaffinlösungsmittel, Butan, Propan, Isobutan, CFCs, Fluorierte Aerosoltreibmittel, Dimethylether, Komprimierte Gase, etc.

| Komponente | % |
|---|---|
| Erfindungsgemäße Polysiloxanverbindung | 0.50 - 5.00 |
| Cyclomethicone | 0.00 - 80.00 |
| Fixative | 0.10-10.00 |
| Ethanol | 0.00 - 50.00 |
| Aerosoltreibmittel | 0.00 - 50.00 |
| Konservierungsmittel | 0.00 - 0.50 |
| Duftstoffe | 0.00 - 5.00 |
| Entionisiertes Wasser | q.s. 100% |

Pumpspray (Styling) für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Vinylderivative, Fixativ-Polymere, Lacke, Fettsäuren, Fettsäureester, Ethoxylierte Fettsäuren, Ethoxylierte Fettsäureester, Fettalkohole, Ethoxylierte Fettalkohol, Glykole, Glykolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffinlösungsmittel, Butan, Propan, Isobutan, CFCs, Fluorierte Aerosoltreibmittel, Dimethylether, Komprimierte Gase, etc.

| Komponente | % |
|---|---|
| Erfindungsgemäße Polysiloxanverbindung | 0.50 - 5.00 |
| Fixative | 0.10-10.00 |
| Cyclomethicone | 0.00 - 80.00 |
| Ethanol | 0.00 - 50.00 |
| Konservierungsmittel | 0.00 - 0.50 |
| Duftstoffe | 0.00 - 5.00 |
| Entionisiertes Wasser | q.s. 100% |

Die Verwendung der erfindungsgemäßen Polysiloxanderivate führt bei Anwendung im Haarkosmetikbereich zu günstigen Effekten hinsichtlich Festigung, Glanz, Fixierung (Halt), Körper, Volumen, Feuchtigkeitsregulierung, Farbretention, Schutz vor Umwelteinflüssen (UV, Salzwasser u.s.w.), Wiederformbarkeit, antistatischen Eigenschaften, Färbbarkeit etc.

**Polysiloxanverbindongs-Beispiele:**

Die nachfolgenden Beispiele sollen die vorliegende Erfindung näher erläutern, ohne sie jedoch einzuschränken.

**Beispiel 1 (Erste Ausführungsform der Erfindung)**

1a) 205,3 g (0,5 mol) eines molmassenverteilten Polyethers der durchschnittlichen Struktur $CH_2=CHCH_2(OCH_2CH_2)_8OH$ wurden unter Stickstoff bei Raumtemperatur vorgelegt. Innerhalb von 30 Minuten wurde unter intensiven Rühren 63,4 g (0,55 mol) Chloressigsäurechlorid zugetropft. Während des Zutropfens stieg die Temperatur auf 67 °C an und eine intensive HCl-Entwicklung setzte ein. Nach Beendigung des Zutropfens wurde der Ansatz 40 Minuten auf 120 °C erhitzt. Abschließend wurden alle bis 120 °C und bei 20 hPa siedenden Bestandteile abdestilliert. 243,6 g einer gelben, viskosen Flüssigkeit der Zusammensetzung

$$CH_2{=}CHCH_2(OCH_2CH_2)_8OC(O)CH_2Cl$$

wurde erhalten. Gaschromatographisch wurde festgestellt, daß im Reaktionsprodukt weder Chloressigsäurechlorid, Chloressigsäure noch die OH-terminierten Oligoethylenglycole vorhanden sind.

1b) 58,4 g ($6*10^{-2}$ mol) des Chloressigsäureesters gemäß 1a) und 0,43 g einer 2,63 % Platin enthaltenden Lösung von Hexachloroplatinsäure in 2-Propanol wurden unter Stickstoff auf 130 °C erhitzt. Innerhalb von 1,5 Stunden wurden 155,3 g ($1{,}2*10^{-1}$ mol) eines $\alpha,\omega$-SiH funktionalisierten Siloxans der durchschnittlichen Zusammensetzung

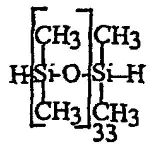

zugetropft. Nach weiteren 8 Stunden bei 130 °C wurde die Reaktion abgebrochen und durch SiH-Bestimmung ein Reaktionsumsatz von 97 % festgestellt. Im Verlauf der Hydrosilylierungsreaktion wurde aus dem anfänglich trüben, zweiphasigen System eine klare Lösung 186 g eines gelben Öls der Struktur

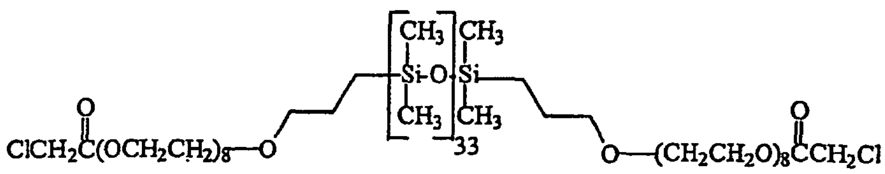

erhalten.

1c) 57 g ($1{,}6*10^{-2}$ mol) des Chloressigsäureesterderivates gemäß 1b wurden bei Raumtemperatur in 100 ml 2-Propanol gelöst. Anschließend wurden 1,86 g ($1{,}6*10^{-2}$ mol) Dimethylpiperazin zugegeben. Der Ansatz wurde 6 Stunden unter Rückflußtemperatur erhitzt. Es wurden abschließend alle bis 90 °C bei 1,2 hPa siedenden Bestandteile abdestilliert. Die Reaktion ergab 54 g eines grauen Produktes der Struktur

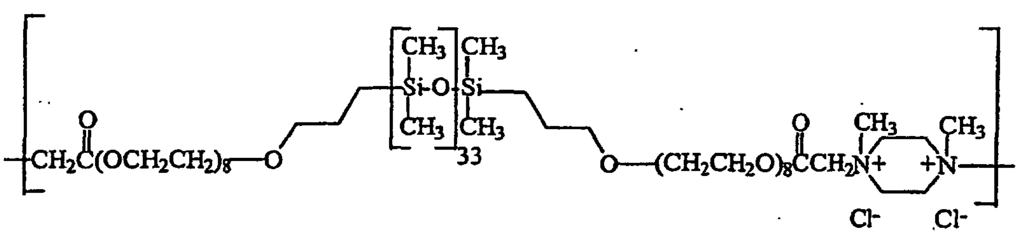

welches zunächst als sirupöse Masse vorlag und in Verlauf von 24 Stunden kristallisierte. Gaschromatographisch konnte unmittelbar nach Beendigung der Reaktion und vor der Entfernung der verdampfbaren Bestandteile kein Dimethylpiperazin mehr nachgewiesen werden.

| $^{13}$C-NMR: | |
|---|---|
| **Substruktur** | **shift (ppm)** |
| -**<u>C</u>**(O)-$CH_2$-$N^+$-$CH_2$-$CH_2$-$N^+$-$CH_2$-C(O)- | 167,3 |

(fortgesetzt)

| $^{13}C$-NMR: | |
|---|---|
| **Substruktur** | **shift (ppm)** |
| $-C(O)-\underline{\mathbf{C}}H_2-N^+-CH_2-CH_2-N^+-CH_2-C(O)-$ | 63,2/63,4 |
| $-C(O)-CH_2-N^+-\underline{\mathbf{C}}H_2-CH_2-N^+-CH_2-C(O)-$ | 60,3 |
| $-C(O)-CH_2-N^+-CH_2-\underline{\mathbf{C}}H_2-N^+-CH_2-C(O)-$ | 60,3 |
| $-C(O)-CH_2-N^+-CH_2-CH_2-N^+-\underline{\mathbf{C}}H_2-C(O)-$ | 63,2/63,4 |
| $-C(O)-CH_2-N^+-CH_2-CH_2-N^+-CH_2-\underline{\mathbf{C}}(O)-$ | 167,3 |
| $\underline{\mathbf{C}}H_3-N^+\equiv$ | 54,1 |

**Beispiel 2 (Erste Ausführungsform der Erfindung)**

2a) Es wurden 181,3 g (0,5 mol) eines Epoxysiloxans der Formel

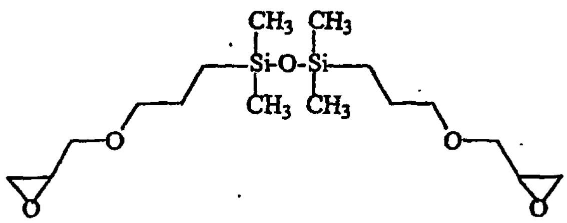

mit 101,2 g (1 mol) N-Methylpiperazin in 100 ml 2-Propanol gelöst und 4 Stunden bei 90 °C zur Reaktion gebracht. Nach Entfernung aller bis 130 °C und bei 20 hPa siedenden Bestandteile wurden 276 g eines hellbraunen, klaren Amins der Formel

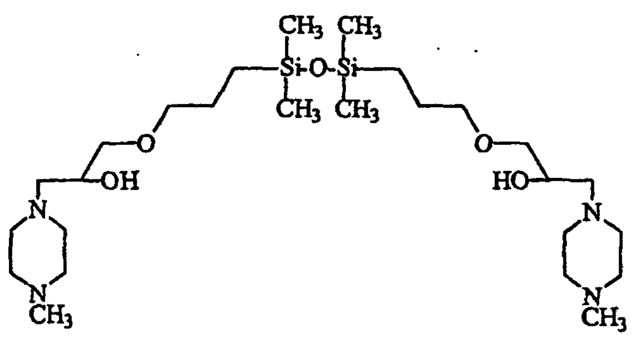

erhalten.
Gaschromatographisch wurde ein Reaktionsumsatz der beiden Komponenten von größer 99 % festgestellt.

2b) 57 g ($1{,}6*10^{-2}$ mol) des Chloressigsäureesterderivates gemäß 1b und 9,01 g ($1{,}6*10^{-2}$ mol) des Aminosiloxans gemäß 2a wurden bei Raumtemperatur in 100 ml 2-Propanol gelöst. Anschließend wurde die Reaktionsmischung 6 Stunden auf Rückflußtemperatur erhitzt Es wurden hiernach alle bis 100 °C und bei 10 hPa siedenden Bestandteile abdestilliert. Die Reaktion ergab 61,8 g eines braunen Produktes der Struktur

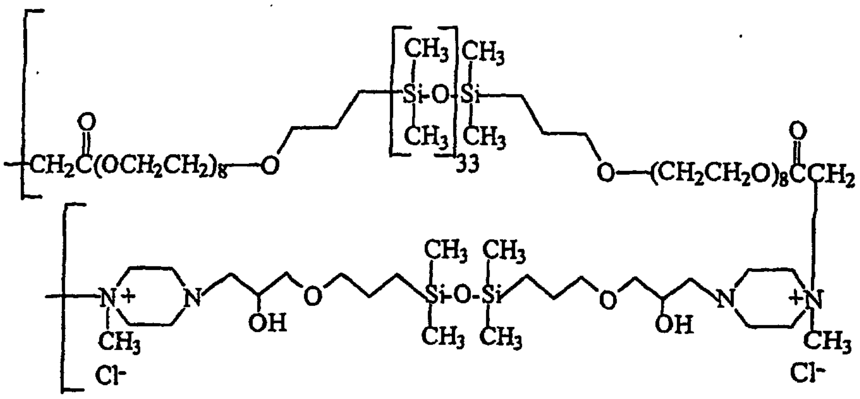

welches zunächst als sirupöse Masse vorlag und beim Erkalten ohne zu kristallisieren erstarrte.

| $^{13}$C-NMR: | |
|---|---|
| **Substruktur** | **shift (ppm)** |
| -**C**H(OH)-$CH_2$-N-$CH_2$-$CH_2$-$N^+$-$CH_2$-C(O)- | 64,9 |
| -CH(OH)-**C**$H_2$-N-$CH_2$-$CH_2$-$N^+$-$CH_2$-C(O)- | 53,4 |
| -CH(OH)-$CH_2$-N-**C**$H_2$-$CH_2$-$N^+$-$CH_2$-C(O)- | 46,1 |
| -CH(OH)-$CH_2$-N-$CH_2$-**C**$H_2$-$N^+$-$CH_2$-C(O)- | 60,2 |
| -CH(OH)-$CH_2$-N-$CH_2$-$CH_2$-$N^+$-**C**$H_2$-C(O)- | 61,8 |
| -CH(OH)-$CH_2$-N-$CH_2$-$CH_2$-$N^+$-$CH_2$-**C**(O)- | 166,7 |
| **C**$H_3$-$N^+$≡ | 50,7 |

Gemäß $^{13}$C-NMR Spektrum erfolgt die Quaternierung seklektiv an den methylsubstituierten Stickstoffatomen.

**Beispiel 3 (Erste Ausführungsform der Erfindung)**

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur® , bentonithaltigem Dash 2 in 1® sowie des in Beispiel 1 beschriebenen ethylenoxidmodifizierten Esterquats unterworfen. Es wurden folgende Randbedingungen eingehalten.

| | Streifen 1 | Streifen 2 | Streifen 3 |
|---|---|---|---|
| Streifengewicht (g) | 11,81 | 10,81 | 10,9 |
| Wassermenge (ml) | 590 | 540 | 550 |
| Detergenz | 0,59g Ariel Futur® | 0,54g Ariel Futur® | 0,55g Dash 2 in 1® |
| Quat Bsp. 1 | 0,17 g | - | - |
| Note ∅ | 1,7 | 2,4 | 1,9 |

Das Wasser wurde auf 60 °C erhitzt, die Detergenzien und im Falle des Baumwollstreifens 1 zusätzlich die Verbindung gemäß Beispiel 1 gelöst. Anschließend wurden die Baumwollstreifen in diesen Lösungen für 30 Minuten gewaschen. Nachfolgend wurden die Streifen fünfmal mit jeweils 600 ml Wasser gespült und abschließend 30 Minuten bei 120 °C getrocknet.

15 Testpersonen bewerteten die Baumwollstreifen auf die Weichheit des Griffs hin, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,7. Der Baumwollstreifen 2 wurde durchschnittlich mit 2,4 und der bentonitbehandelte Streifen 3 mit 1,9 bewertet.

**Beispiel 4 (Zweite Ansführungsform der Erfindung)**

4a) 211,1 g (0,15 mol Epoxygruppen) eines Epoxysiloxans der durchschnittlichen Zusammensetzung

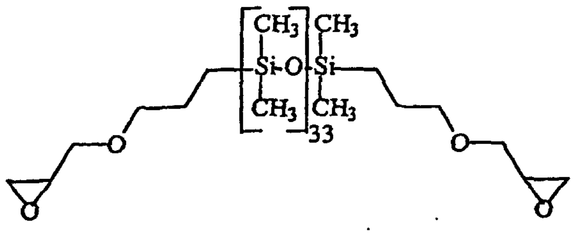

und 15,2 g (0,15 mol) N-Methylpiperazin wurden in 225 ml i-Propanol gelöst und 4 Stunden auf 90 °C erhitzt. Nach dem Reaktionsende wurde das Lösungsmittel durch Destillation im Wasserstrahlvakuum und abschließend im Ölpumpenvakuum entfernt. Es wurden 217 g eines klaren, gelblichen Produktes der Struktur

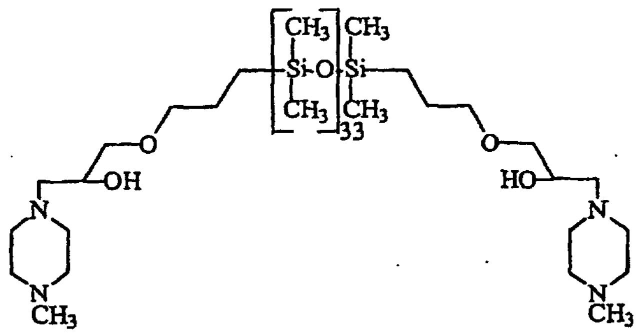

erhalten.

| $^{13}C$-NMR: | |
|---|---|
| **Substruktur** | **shift (ppm)** |
| -**C**H(OH)- | 66,07 |
| -CH(OH)-**C**$H_2$-N- | 60,74 |
| -CH(OH)-$CH_2$-N-**C**$H_2$- | 53,20 |
| -CH(OH)-$CH_2$-N-$CH_2$-**C**$H_2$- | 55,10 |
| **C**$H_3$-N= | 45,87 |

4b) 200 g (1,21 mol) Triethylenglycolmonomethylether wurden unter Stickstoff bei Raumtemperatur vorgelegt. Unter intensiven Rühren wurde innerhalb 30 Minuten 151 g (1,34 mol) Chloressigsäurechlorid zugetropft. Während des Zutropfens stieg die Temperatur auf 90 °C an und eine intensive HCl-Entwicklung setzte ein. Nach Beendigung des Zutropfens wurde der Ansatz 30 Minuten auf 130 °C erhitzt. Abschließend wurden alle bis 130 °C und bei 20 hPa siedenden Bestandteile abdestilliert. Es wurden 301 g einer hellgelben, viskosen Flüssigkeit der Zusammensetzung

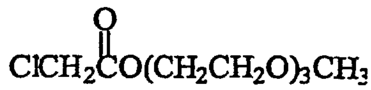

erhalten.

Die gaschromatographisch bestimmte Reinheit des Esters beträgt 99%.

| $^{13}$C-NMR: | |
|---|---|
| **Substruktur** | **shift (ppm)** |
| Cl**C**H$_2$- | 40,8 |
| ClCH$_2$-**C**(O)- | 167,3 |
| ClCH$_2$-CH$_2$-C(O)-O**C**H$_2$- | 65,2 |
| ClCH$_2$-CH$_2$-C(O)-OCH$_2$**C**H$_2$- | 68,7 |
| -CH$_2$-O**C**H$_3$ | 58,8 |

4c) 19,61 g (6,5∗10$^{-3}$ mol) des α,ω-Aminosiloxans gemäß Beispiel 4a) und 3,12 g (1,3∗10$^{-2}$ mol) des Chloressigsäureesters gemäß Beispiel 4b) wurden unter Stickstoff in 50 ml i-Propanol gelöst und 12 Stunden auf Rückflußtemperatur erhitzt. Nach Beendigung der Reaktion wurden alle bis 70 °C und bei 20 hPa siedenden Bestandteile entfernt. Es wurden 19,7 g eines gelblich-hellbraunen viskosen Öles der Formel

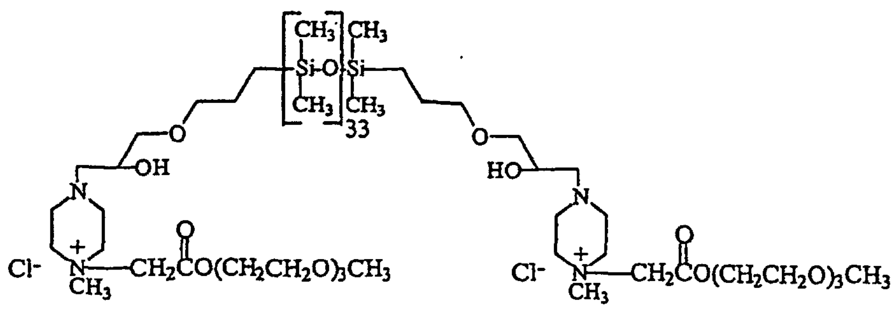

erhalten. Gaschromatographisch wurde ein quantitativer Umsatz des Esters festgestellt.

| $^{13}$C-NMR: | |
|---|---|
| **Substruktur** | **shift (ppm)** |
| -**C**H(OH)- | 65,9/66,1 |
| -CH(OH)-**C**H$_2$-N- | 52,6 |
| -CH(OH)-CH$_2$-N-**C**H$_2$- | 45,4 |
| -CH(OH)-CH$_2$-N-CH$_2$-**C**H$_2$- | 60,5/60,6 |
| -CH(OH)-CH$_2$-N-CH$_2$-CH$_2$-N$^+$-**C**H$_2$- | 61,4 |
| -CH(OH)-CH$_2$-N-CH$_2$-CH$_2$-N$^+$-CH$_2$-**C**(O)- | 169,6/169,9 |
| **C**H$_3$-N$^+$≡ | 52,9 |
| -CH$_2$-O**C**H$_3$ | 58,6 |

Gemäß $^{13}$C-NMR Spektrum erfolgt die Quartärnierung selektiv an den methylsubstituierten Stickstoffatomen.

**Beispiel 5 (Zweite Ansführungsform der Erfindung)**

Zum Nachweis der weichmachenden Eigenschaften wurden gebleichte und an der Oberfläche nicht weiter ausgerüstete Baumwollstreifen einem Waschprozeß in Gegenwart von Ariel Futur® , bentonithaltigem Dash 2 in 1® ,

sowie des in Beispiel 4 beschriebenen ethylenoxidmodifizierten Esterquats unterworfen. Es wurden folgende Randbedingungen eingehalten.

| | Streifen 1 | Streifen 2 | Streifen 3 |
|---|---|---|---|
| Streifengewicht (g) | 13,06 | 12,92 | 13,35 |
| Wassermenge (ml) | 653 | 646 | 667 |
| Detergenz | 0,65g Ariel Futur® | 0,64g Dash 2 in 1® | 0,67g Ariel Futur® |
| Esterquat Bsp. 4 | 0,2 g | - | - |
| Note ∅ | 1,2 | 2,0 | 2,8 |

Das Wasser wurde auf 60 °C erhitzt, die Detergenzien und im Falle des Baumwollstreifens 1 zusätzlich das Esterquat gemäß Beispiel 4 gelöst Anschließend wurden die Baumwollstreifen in diesen Lösungen für 30 Minuten gewaschen. Nachfolgend wurden die Streifen fünfmal mit jeweils 600 ml Wasser gespült und abschließend 30 Minuten bei 120 °C getrocknet.

14 Testpersonen bewerteten die drei Baumwollstreifen auf die Weichheit des Griffs hin, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,2. Der bentonitbehandelte Baumwollstreifen 2 wurde durchschnittlich mit 2,0 und der Streifen 3 mit 2,8 bewertet.

**Beispiel 6 (Dritte Ausführungsform der Erfindung)**

6a) 281,6 g (0,1 mol) eines Epoxysiloxans der durchschnittlichen Zusammensetzung

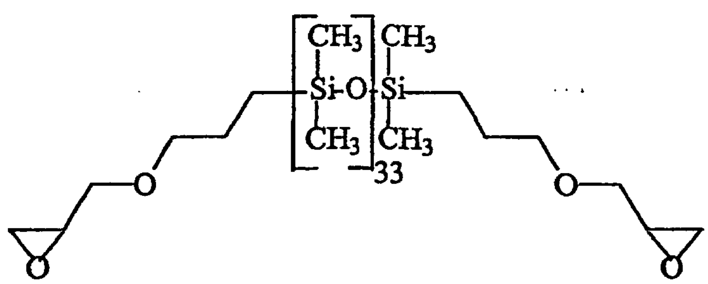

und 8,6 g (0,1 mol) Piperazin wurden in 300 ml i-Propanol gelöst und für 5 Stunden auf Rückflußtemperatur erhitzt. Nach Beendigung der Reaktion wurde das Lösungsmittel durch Destillation im Wasserstrahl- und anschließend im Ölpumpenvakuum entfernt. Es wurden 283 g eines klaren, honigartigen Produktes der Struktur

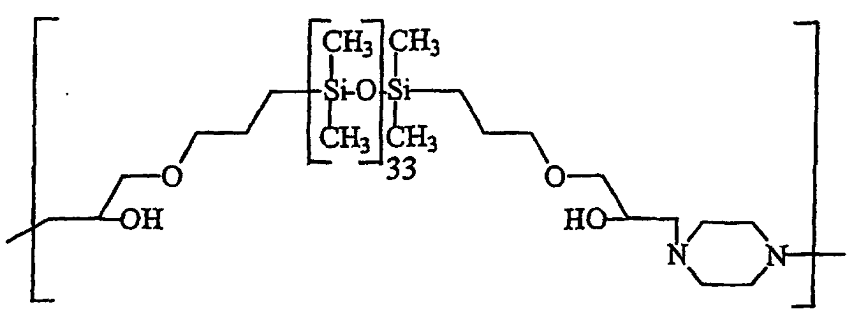

erhalten.

| $^{13}$C-NMR: | |
|---|---|
| **Substruktur** | **shift (ppm)** |
| -**C**H(OH)- | 66,1 |
| -CH(OH)-**C**$H_2$-N- | 60,7 |
| -CH(OH)-$CH_2$-N-**C**$H_2$- | 53,4 |
| -CH(OH)-$CH_2$-N-$CH_2$-**C**$H_2$-N- | 53,4 |

6b) In einer Glasapparatur wurden 200 g (1,21 mol) Triethylenglycolmonomethylether unter Stickstoff bei Raumtemperatur vorgelegt. Innerhalb von 30 Minuten wurden unter intensiven Rühren 151 g (1,34 mol) Chloressigsäurechlorid zugetropft. Während des Zutropfens stieg die Temperatur auf 90 °C an, und intensive HCl-Entwicklung setzte ein. Nach Beendigung des Zutropfens wurde der Ansatz 30 Minuten auf 130 °C erhitzt. Abschließend wurden alle bis 130 °C und 20 hPa siedenden Bestandteile abdestilliert. Es wurden 301 g einer hellgelben, viskosen Flüssigkeit der Zusammensetzung

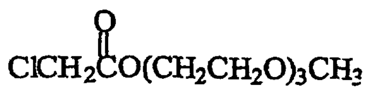

erhalten. Die gaschromatographisch bestimmte Reinheit des Esters beträgt 99%.

| $^{13}$C-NMR: | |
|---|---|
| **Substruktur** | **shift (ppm)** |
| Cl**C**$H_2$- | 40,8 |
| Cl$CH_2$-**C**(O)- | 167,3 |
| Cl$CH_2$-C(O)-O**C**$H_2$- | 65,2 |
| Cl$CH_2$-C(O)-O$CH_2$**C**$H_2$- | 68,7 |
| -$CH_2$-O**C**$H_3$ | 58,8 |

6c) 8,71 g (6∗$10^{-3}$ mol tertiäre Aminogruppen) des polymeren Aminosiloxans gemäß Beispiel 6a) und 0,72 g (3∗$10^{-3}$ mol) des Chloressigsäureesters gemäß Beispiel 6b) wurden unter Stickstoff in 20 ml i-Propanol gelöst und 11 Stunden auf Rückflußtemperatur erhitzt. Nach Beendigung der Reaktion wurden im Wasserstrahlpumpenvakuum und anschließend im Ölpumpenvakuum alle bis 70 °C und bei 2 hPa siedenden Bestandteile entfernt. Es werden 9 g einer gelben, hochgradig viskosen Masse der Formel

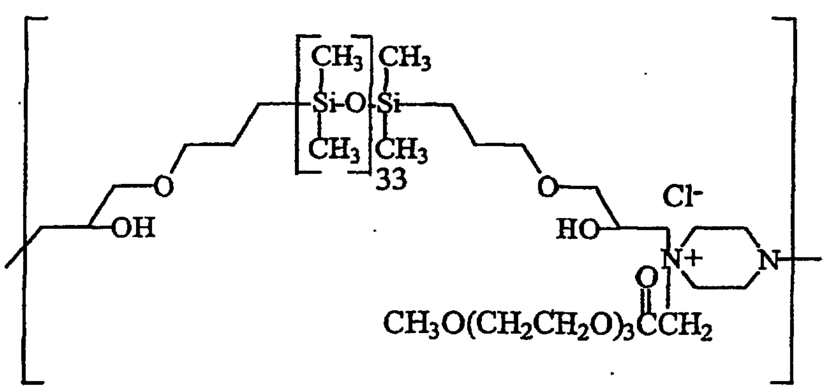

gewonnen.

| $^{13}$C-NMR: | |
|---|---|
| **Substruktur** | **shift (ppm)** |
| -**C**H(OH)- | 65,7 |
| -CH(OH)-**C**$H_2$-N- | 51,7 |
| -CH(OH)-$CH_2$-N-**C**$H_2$- | 43,3 |
| -CH(OH)-$CH_2$-N-$CH_2$-**C**$H_2$- | 64,1 |
| -CH(OH)-$CH_2$-N-$CH_2$-$CH_2$-$N^+$-**C**$H_2$- | 60,7 |
| -CH(OH)-$CH_2$-N-$CH_2$-$CH_2$-$N^+$-$CH_2$-**C**H(OH)- | 68,8 |
| -$N^+$-**C**$H_2$-C(O)- | 61,7 |
| -$N^+$-$CH_2$-**C**(O)- | 167,6 |

**Beispiel 7 (Dritte Ansführungsform der Erfindung)**

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur® , bentonithaltigem Dash 2 in 1® sowie des in Beispiel 6 beschriebenen ethylenoxidmodifizierten polyquartenären Polysiloxanester unterworfen. Es wurden folgende Randbedingungen eingehalten.

| | Streifen 1 | Streifen 2 | Streifen 3 |
|---|---|---|---|
| Streifengewicht (g) | 13,36 | 13,61 | 13,44 |
| Wassermenge (ml) | 668 | 681 | 670 |
| Detergenz | 0,66 g Ariel Futur® | 0,68 g Ariel Futur® | 0,65 g Dash 2 in 1® |
| Quat Bsp. 6 | 0,2 g | - | - |
| Note ∅ | 1,4 | 2,7 | 1,9 |

Das Wasser wurde auf 60°C erhitzt, die Detergenzien und im Falle des Baumwollstreifens 1 zusätzlich das ethylenoxidmodifizierte polyquarternäre Polysilxanester gemäß Beispiel 6 gelöst Anschließend wurden die Baumwollstreifen in diesen Lösungen für 30 Minuten gewaschen. Nachfolgend wurden die Streifen jeweils fünfmal mit 600 ml Wasser gespült und abschließend 30 Minuten bei 120°C getrocknet.

14 Testpersonen bewerteten die drei Baumwollstreifen auf die Weichheit des Griffs hin, wobei die Note 1 dem weichsten Streifen und die Note 3 dem als am härtesten empfundenen Streifen zugeteilt wurde.

Im Ergebnis der Bewertung erhielt der Baumwollstreifen 1 die Durchschnittsnote 1,4. Der Baumwollstreifen 2 wurde durchschnittlich mit 2,7 und der mit Bentonit behandelte Streifen 3 mit 1,9 bewertet.

**Beispiel 8 (Vierte Ausführungsform der Erfindung**

8a) 211,1 g (0,15 mol Epoxygruppen) eines Epoxysiloxans der durchschnittlichen Zusammensetzung

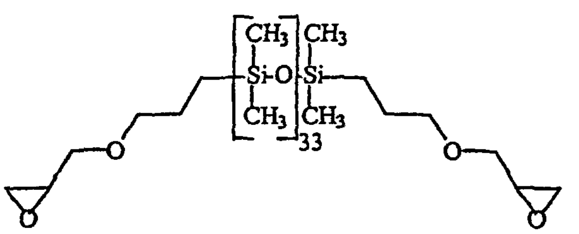

und 15,2 g (0,15 mol) N-Methylpiperazin wurden in 225 ml i-Propanol gelöst und 4 Stunden auf 90 °C erhitzt. Nach Beendigung der Reaktion wird das Lösungsmittel durch Destillation im Wasserstrahl- und abschließend Ölpumpenvakuum entfernt. Es werden 217 g eines klaren, gelblichen Produktes der Struktur

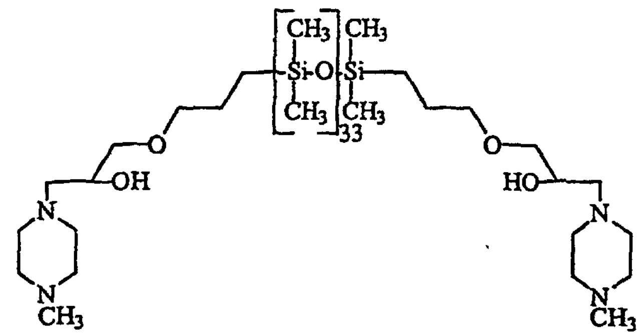

erhalten.

| $^{13}$C-NMR: | |
|---|---|
| **Substruktur** | **shift (ppm)** |
| -**C**H(OH)- | 66,07 |
| -CH(OH)-**C**$H_2$-N- | 60,74 |
| -CH(OH)-$CH_2$-N-**C**$H_2$- | 53,20 |
| -CH(OH)-$CH_2$-N-$CH_2$-**C**$H_2$- | 55,10 |
| **C**$H_3$-N= | 45,87 |

8b) 150 g (1 mol OH-Gruppen) eines Polyethylenglycols mit einem Molekulargewicht von 300 g/mol (6,4 Ethylenoxid-Einheiten) wurden unter Stickstoff bei Raumtemperatur vorgelegt. Unter intensiven Rühren wurden innerhalb 30 Minuten 152,4 g (1,2 mol) 3-Chlorpropionsäurechlorid zugetropft. Während des Zutropfens stieg die Temperatur auf 70 °C an und eine intensive HCl-Entwicklung setzte ein. Nach Beendigung des Zutropfens wurde der Ansatz 30 Minuten auf 120 °C erhitzt. Abschließend wurden alle bis 120 °C und bei 20 hPa siedenden Bestandteile abdestilliert. Es wurden 244 g einer hellgelben, viskosen Flüssigkeit der Zusammensetzung

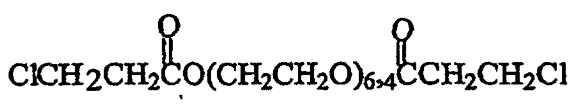

erhalten.

Gaschromatographisch konnte festgestellt werden, daß im Reaktionsprodukt die OH-terminierten Oligoethylenglycole nicht mehr vorhanden sind.

| $^{13}$C-NMR: | |
|---|---|
| **Substruktur** | **shift (ppm)** |
| Cl**C**$H_2$- | 39,19 |
| Cl$CH_2$-**C**$H_2$- | 37,49 |
| Cl$CH_2$-$CH_2$-C(O)- | 170,17 |
| Cl$CH_2$-$CH_2$-C(O)-O**C**$H_2$- | 64,01 |
| Cl$CH_2$-$CH_2$-C(O)-O$CH_2$**C**$H_2$- | 68,97 |

8c) 19,61 g (6,5∗$10^{-3}$ mol) des α,ω-Aminosiloxans gemäß Beispiel 8a) und 3,12 g (6,5∗$10^{-3}$ mol) des α,ω-3-Chlorpropionsäureesters gemäß Beispiel 8b) wurden unter Stickstoff in 50 ml i-Propanol gelöst und 12 Stunden auf Rückflußtemperatur erhitzt. Nach Beendigung der Reaktion wurden alle bis 70°C und bei 20 hPa siedenden Bestandteile entfernt. Es wurden 21,6 g eines gelblichen Wachses der Formel

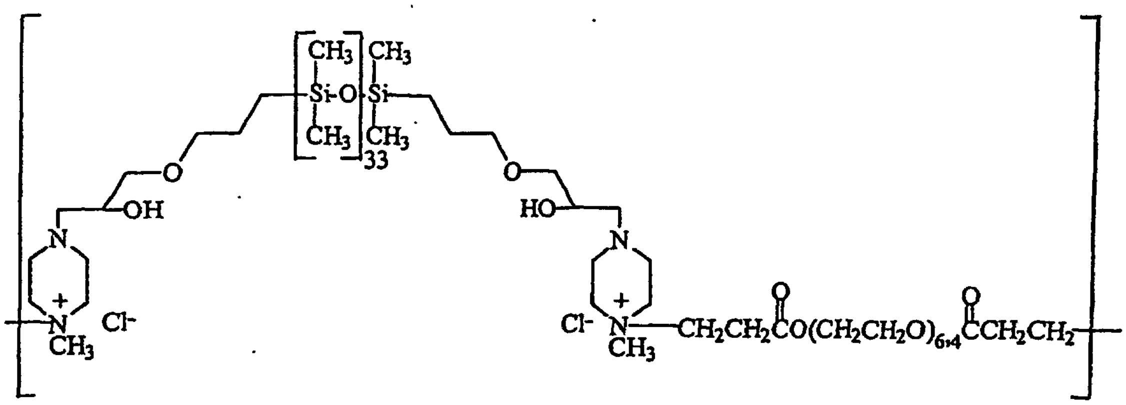

erhalten.

| $^{13}$C-NMR: | |
|---|---|
| **Substruktur** | **shift (ppm)** |
| -**C**H(OH)- | 68,9 |
| -CH(OH)-**C**$H_2$-N- | 52,8 |
| -CH(OH)-$CH_2$-N-**C**$H_2$- | 43,6 |
| -CH(OH)-$CH_2$-N-$CH_2$-**C**$H_2$- | 63,3 |
| -CH(OH)-$CH_2$-N-$CH_2$-$CH_2$-$N^+$-**C**$H_2$- | 60,2 |
| -CH(OH)-$CH_2$-N-$CH_2$-$CH_2$-$N^+$-$CH_2$-**C**$H_2$- | 25,5 |
| -CH(OH)-$CH_2$-N-$CH_2$-$CH_2$-$N^+$-$CH_2$-$CH_2$-**C**(O)- | 165,7 |
| **C**$H_3$-$N^+$≡ | 50,6 |

Gemäß $^{13}$C-NMR Spektrum erfolgt die Quartärnierung selektiv an den methylsubstituierten Stickstoffatomen.

**Beispiel 9 (Vierte Ausführungsform der Erfindung)**

9a) In Analogie zu Beispiel 8a) wurden 181,3 g (0,5 mol) eines Epoxysiloxans der Formel

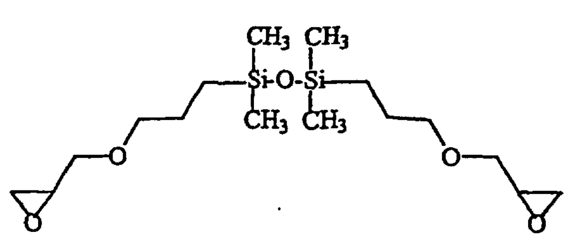

mit 101,2 g (1 mol) N-Methylpiperazin in 100 ml i-Propanol zur Reaktion gebracht Nach Entfernung aller bis 130 °C und bei 20 hPa siedenden Bestandteile wurden 276 g eines mittelbraunen, klaren Amins der Formel

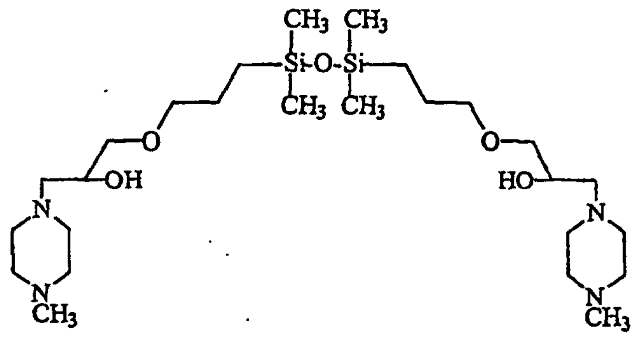

erhalten.

Gaschromatographisch wurde ein Reaktionsumsatz der beiden Komponenten von größer 99% festgestellt.

9b) 6,2 g (1,1∗$10^{-2}$ mol) des Aminosiloxans gemäß Beispiel 9a), 33,21 g (1,1∗$10^{-2}$ mol) des $\alpha,\omega$-Aminosiloxans gemäß Beispiel 8a) und 10,59 g (2,2∗$10^{-2}$ mol) des 3-Chlorpropionsäureesters gemäß Beispiel 8b) wurden in 50 ml i-Propanol aufgenommen und unter Stickstoff 10 Stunden auf Rückflußtemperatur erhitzt. Nach Beendigung der Reaktion wurden alle bis 40° C und bei 20 hPa siedenden Bestandteile entfernt. Es wurden 48,7 g einer braunen, wachsartigen Verbindung der mittleren Zusammensetzung

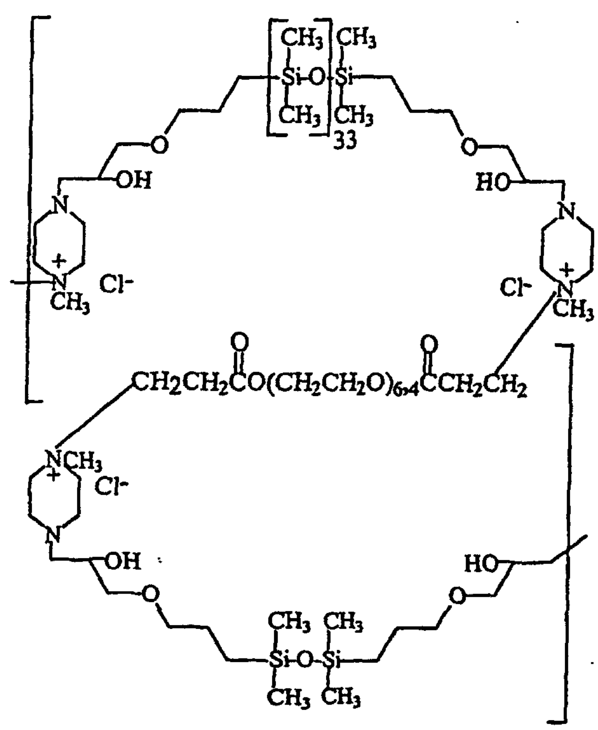

erhalten, in welcher die beiden unterschiedlichen Siloxanblöcke im Verhältnis 1: 1 vorliegen

| $^{13}$C-NMR: | |
|---|---|
| **Substruktur** | **shift (ppm)** |
| -**C̲**H(OH)- | 68,9 |
| -CH(OH)-**C̲**$H_2$-N- | 52,8 |

(fortgesetzt)

| $^{13}C$-NMR: | |
|---|---|
| **Substruktur** | **shift (ppm)** |
| $-CH(OH)-CH_2-N-\underline{\mathbf{C}}H_2-$ | 43,6 |
| $-CH(OH)-CH_2-N-CH_2-\underline{\mathbf{C}}H_2-$ | 63,3 |
| $-CH(OH)-CH_2-N-CH_2-CH_2-N^+-\underline{\mathbf{C}}H_2-$ | 60,2 |
| $-CH(OH)-CH_2-N-CH_2-CH_2-N^+-CH_2-\underline{\mathbf{C}}H_2-$ | 25,5 |
| $-CH(OH)-CH_2-N-CH_2-CH_2-N^+-CH_2-CH_2-\underline{\mathbf{C}}(O)-$ | 165,7 |
| $\underline{\mathbf{C}}H_3-N^+\equiv$ | 50,6 |

Signale für $CH_3$-Gruppen in tertiären Aminostrukturen (siehe NMR-Datensatz zu Beispiel 8a) werden nicht mehr gefunden.

**Beispiel 10 (Vierte Ausführungsform der Erfindung)**

Zum Nachweis der weichmachenden Eigenschaften wurden gebleichte und an der Oberfläche nicht weiter ausgerüstete Baumwollstreifen einem Waschprozeß in Gegenwart von Ariel Futur® , bentonithaltigem Dash 2 in 1® und des in Beispiel 8 beschriebenen ethylenoxidmodifizierten Esterquats unterworfen. Es wurden folgende Randbedingungen eingehalten.

| | Streifen 1 | Streifen 2 | Streifen 3 |
|---|---|---|---|
| Streifengewicht (g) | 13,43 | 12,94 | 13,17 |
| Wassermenge (ml) | 671 | 647 | 658 |
| Detergenz | 0,67 g Ariel Futur® | 0,64 g Dash 2 in 1® | 0,65 g Ariel Futur® |
| Esterquat Bsp. 8 | 0,2 g | | |
| Note ∅ | 1,6 | 1,9 | 2,5 |

Das Wasser wurde auf 60 °C erhitzt, die Detergenzien und im Falle des Baumwollstreifens 1 zusätzlich die Verbindung gemäß Beispiel 1 gelöst. Anschließend wurden die Baumwollstreifen in diesen Lösungen 30 Minuten gewaschen. Nachfolgend wurden die Streifen fünfmal mit jeweils 600 ml Wasser gespült und abschließend 30 Minuten bei 120 °C getrocknet.

17 Testpersonen bewerteten die drei Baumwollstreifen auf die Weichheit des Griffs hin, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,6. Der bentonitbehandelte Baumwollstreifen 2 wurde mit 1,9 und Streifen 3 durchschnittlich mit 2,5 bewertet

**Beispiel 11 (Fünfte Ausführangsform der Erfindung)**

11a) 238g (2,24 mol) Diethylenglycol werden unter Stickstoff bei Raumtemperatur vorgelegt. Unter intensiver Rührung werden innerhalb einer Stunde 558g (4,93 mol) Chloressigsäurechlorid zugetropft. Während des Zutropfens steigt die Temperatur auf 82°C an und eine intensive HCl-Entwicklung setzt ein. Nach Beendigung des Zutropfens wird der Ansatz für 30 Minuten auf 130°C erhitzt. Abschließend werden alle bis 130°C/20hPa siedenden Bestandteile abdestilliert. Es werden 566g eines hellgelben Öls der Zusammensetzung

$$ClCH_2C(O)OCH_2CH_2OCH_2CH_2OC(O)CH_2Cl$$

erhalten.

Die gaschromatographisch bestimmte Reinheit des Esters beträgt 99,2 %.

| $^{13}$C-NMR: | |
|---|---|
| **Substruktur** | **shift (ppm)** |
| Cl$\underline{C}H_2$- | 40,7 |
| $ClCH_2$-$\underline{C}$(O)- | 167,1 |
| $ClCH_2$-C(O)-O$\underline{C}H_2$- | 65,2 |
| $ClCH_2$-C(O)-$OCH_2\underline{C}H_2$- | 68,6 |

11b) In einem 1 Liter Dreihalskolben werden 18g Wasser und 8,62g (0,1 mol tertiäre Aminogruppen) N,N,N',N'-Tetramethyl-1,6-hexandiamin bei Raumtemperatur vorgelegt. Innerhalb von 5 Minuten werden 36,0g (0,09 mol) Dodecansäure in Form einer 50%igen Lösung in 2-Propanol zugesetzt. Nach Erwärmung des Ansatzes auf 50°C werden innerhalb von 30 Minuten 194,7g (0,09 mol Epoxygruppen) eines Epoxysiloxans der durchschnittlichen Zusammensetzung

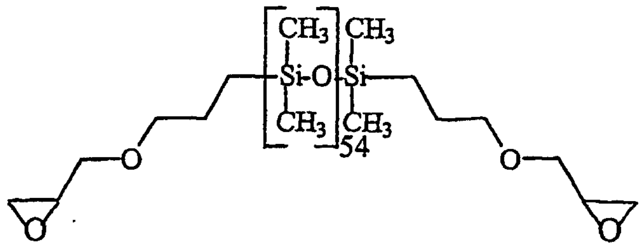

und 1,3g (0,01 mol Alkylhalogenidgruppen) des Esterderivates gemäß 11a) in 22,5ml 2-Propanol zugetropft. Die gelbe, trübe Mischung wird für 6 Stunden auf Rückflußtemperatur erhitzt. Nach Entfernung aller bis 100°C/2mmHg in Vakuum flüchtigen Bestandteile werden 210g eines gelben, trüben Materials gewonnen, welches das folgende Strukturelement umfaßt:

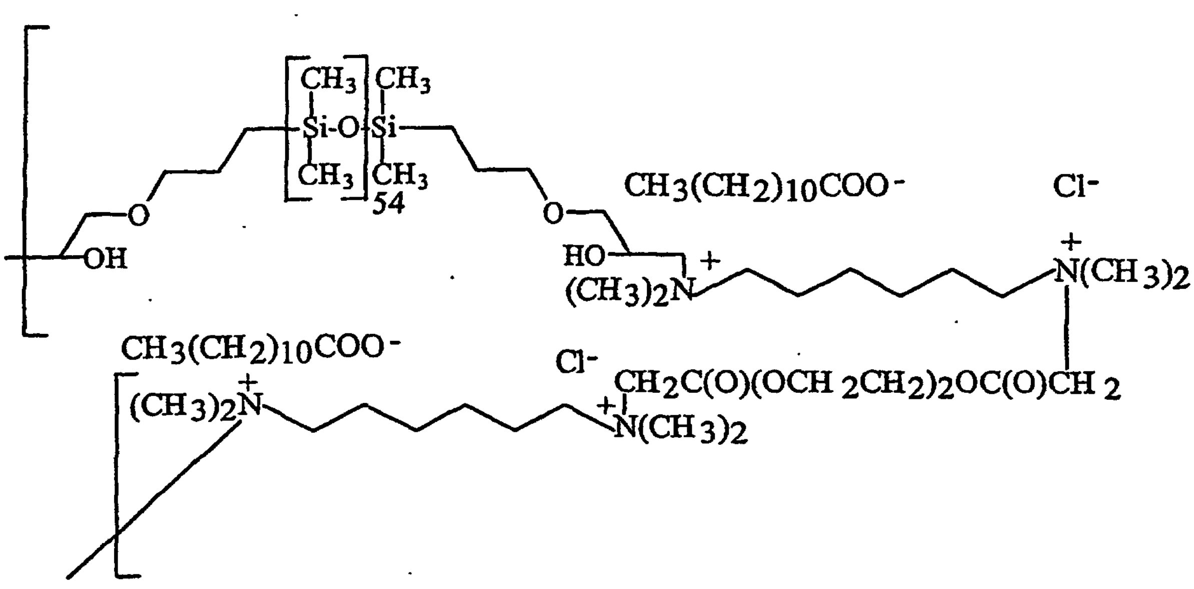

| $^{13}C$-NMR: | |
|---|---|
| **Substruktur** | **Shift (ppm)** |
| $-\underline{\mathbf{C}}H(OH)-CH_2-N^+[(CH_3)_2]-CH_2-CH_2-$ | 65,3 |
| $-CH(OH)-\underline{\mathbf{C}}H_2-N^+[(CH_3)_2]-CH_2-CH_2-$ | 64,6 |
| $-CH(OH)-CH_2-N^+[(\underline{\mathbf{C}}H_3)_2]-CH_2-CH_2-$ | 53,1/53,3 |
| $-CH(OH)-CH_2-N^+[(CH_3)_2]-\underline{\mathbf{C}}H_2-CH_2-$ | 64,8 |
| $-CH(OH)-CH_2-N^+[(CH_3)_2]-CH_2-\underline{\mathbf{C}}H_2-$ | 23,4 |
| $-O-\underline{\mathbf{C}}(O)-CH_2-N^+[(\underline{\mathbf{C}}H_3)_2]-CH_2-CH_2-$ | 174,2 |
| $-O-C(O)-\underline{\mathbf{C}}H_2-N^+[(\underline{\mathbf{C}}H_3)_2]-CH_2-CH_2-$ | 62,0 |
| $-O-C(O)-CH_2-N^+[(\underline{\mathbf{C}}H_3)_2]-CH_2-CH_2-$ | 53,7 |

**Beispiel 12 (Fünfte Ausführungsform der Erfindung) und Vergleichsbeispiel**

Zum Nachweis der Eignung als waschbeständiger, hydrophiler Weichmacher wurde weißer Baumwolljersey der nachfolgend beschriebenen Behandlung mit einer auf dem quartären Ammoniumsalz gemäß Beispiel 11 beruhenden Formulierung unterworfen. Zum Vergleich wurde ein kommerziell erhältlicher hydrophiler Weichmacher benutzt. Es wurden zunächst folgende klare Stammformulierungen hergestellt:

| Erfindungsgemäß Bsp. 11 | kommerziell erhältlicher hydrophiler Weichmacher Magnasoft® HSSD (OSi Specialities) |
|---|---|
| 17,7g Quat Beispiel 11 | 20,0g Siloxanweichmacher |
| 0,4g Essigsäure | 0,2g Essigsäure |
| 0,3g Na-Acetat | 79,8g dest Wasser |
| 10,5g Renex 36® (Henkel) | |
| 1,8g Renex 30® (Henkel) | |
| 67,3g dest. Wasser | |

20g dieser Stunmformulierungen wurden in 980g destilliertem Wasser gelöst. Anschließend erfolgt mit diesen effektiv 0,35% bzw. 0,4% Siloxanwirkstoff enthaltenden Formulierungen eine Ausrüstung von 60 cm x 90 cm großen und 87g schweren Baumwolljerseystücken im Foulardverfahren. Hierzu wird das Baumwollmaterial 5 Sekunden vollständig in die jeweilige Formulierung eingetaucht und nach Zwangsapplikation bei 120°C für 3 Minuten getrocknet.

Nachfolgend werden die Lappen geteilt und jeweils eine Hälfte fünf maschinellen Waschzyklen in Gegenwart eines Feinwaschmittels (1,7g Waschmittel/ Liter Waschflotte) unterzogen. Jeder Waschzyklus danert 25 Minuten, die Waschtemperatur beträgt 40°C.

An den ungewaschenen und gewaschenen Textilstücken wurde die Hydrophilie (Einziehzeit eines Wassertropfens in Sekunden) bestimmt und zusätzlich von 5 Testpersonen der Griff bewertet.

| | Hydrophilie (s) | Griff |
|---|---|---|
| Erfindungsgemäß Bsp. 11 ausgerüstet; Ungewaschen | 1 | Glatt, flach, weich |
| Erfindungsgemäß Bsp.11 ausgerüstet; 5 x gewaschen | <1 | Glatt, flach, mittelweich |
| kommerziell erhältlicher hydrophiler Weichmacher Ungewaschen | 1 | Glatt, flach |
| kommerziell erhältlicher hydrophiler Weichmacher 5 x gewaschen | 1 | Hart |

Die Ergebnisse zeigen, daß das erfindungsgemäß ausgerüstete Textilmaterial auch nach 5 Waschzyklen noch über die gewünschte Eigenschaftskombination aus Hydrophilie, ausgedrückt durch eine sehr kurze Tropfeneinziehzeit, und dem silicontypischem Griff verfügt.

**Beispiel 13 (Fünfte Ausführungsform der Erfindung)**

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur®, bentonithaltigem Dash 2 in 1® sowie des in Beispiel 11 beschriebenen Siliconquats unterworfen. Es wurden folgende Randbedingungen eingehalten.

| | Streifen 1 | Streifen 2 | Streifen 3 |
|---|---|---|---|
| Streifengewicht (g) | 12,82 | 13,06 | 13,30 |
| Wassermenge (ml) | 639 | 654 | 671 |
| Detergenz | 0,63g Ariel Futur® | 0,66g Ariel Futur® | 0,67g Dash 2 in 1® |
| Quat Bsp. 11 | 0,2 g | - | - |
| Note ∅ | 1,4 | 2,8 | 1,8 |

Das Wasser wird auf 60°C erhitzt, die Detergenzien und im Falle des Baumwollstreifens 1 zusätzlich das Siliconquat gemäß Beispiel 1 gelöst. Anschließend werden die Baumwollstreifen in diesen Lösungen für 30 Minuten gewaschen. Nachfolgend werden die Streifen in 5x600ml Wasser gespült und abschließend 30 Minuten bei 120°C getrocknet.

16 Testpersonen haben die drei Baumwollstreifen auf die Weichheit des Griffs hin bewertet, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,4. Der Baumwollstreifen 2 wurde durchschnittlich mit 2,8 und der bentonitbehandelte Streifen 3 mit 1,8 bewertet.

Die erfindungsgemäßen Polysiloxanverbindungen zeigen eine ausgezeichnete Waschbeständigkeit und Substantivität.

Zwar wird beispielsweise in der EP-A-282720 die Verwendung von polyquartären Polysiloxanen in kosmetischen Formulierungen, speziell zur Behandlung von Haaren beschrieben, und als Vorteile werden generell eine verbesserte Kämmbarkeit, ein guter Glanz, eine hohe antistatische Effektivität und eine verbesserte Auswaschbeständigkeit genannt.

Die letztgenannte Eigenschaft ist jedoch nicht mit Waschbeständigkeit gleichzusetzen. Während sich die Auswaschbeständigkeit aus Haaren auf den kurzzeitigen Angriff von vornehmlich Wasser und sehr milden, die Haut nicht irritierenden Tensiden bezieht, haben waschbeständige, hydrophile Weichmacher für Textilien dem Angriff konzentrierter Tensidlösungen mit hohem Fett- und Schmutzlösevermögen zu widerstehen. Diesen Tensidsystemen sind in modemen Waschmitteln stark alkalische Komplexbildner, oxidativ wirkende Bleichmittel und komplexe Enzymsysteme hinzugefügt Die Einwirkung erfolgt häufig über Stunden bei erhöhten Temperaturen. Aus diesen Gründen heraus ist eine Übertragung von Erfahrungen aus dem Kosmetikbereich auf das Gebiet der waschbeständigen Textilweichmacher nicht möglich. Die im Stand der Technik zitierte DE-OS 3236466 zeigt auf, daß zur Erzielung einer waschbeständigen Textilausrüstung auf vernetzungsfähige Systeme zu orientieren gewesen wäre.

Analog dazu war nicht zu erwarten, daß die erfindungsgemäßen Verbindungen als Weichmacher in auf nichtionischen oder anionischen/nichtionischen Tensiden beruhenden Formulierungen zur Wäsche von Fasern und Textilien wirksam sein könnten. Auch in diesen Fällen erfolgt über lange Zeiträume bei erhöhten Temperaturen die Einwirkung der aggressiven Detergenzienformulierung. Erschwerend kommt hinzu, daß die vorgelagerte Modifizierung der Faseroberfläche mit weichmachenden Substanzen entfällt

**Patentansprüche**

**1.** Polysiloxan-Verbindung enthaltend:

a) mindestens eine Polyalkylenoxid-Struktureinheit der allgemeinen Formeln:

-A-E-, -E-A-, -A-E-A'- und/oder A'-E-A-

worin

A = $-CH_2C(O)O-$, $-CH_2CH_2C(O)O-$, $-CH_2CH_2CH_2C(O)O-$, $-OC(O)CH_2-$, $-OC(O)CH_2-$, $-OC(O)CH_2CH_2-$ und/oder $-OC(O)CH_2CH_2CH_2-$

A' = $-CH_2C(O)-$, $-CH_2CH_2C(O)-$, $-CH_2CH_2CH_2C(O)-$, $-C(O)CH_2-$, $-C(O)CH_2-$, $-C(O)CH_2CH_2-$ und/oder $-C(O)CH_2CH_2CH_2-$

E = eine Polyalkylenoxidgruppe der allgemeinen Formeln:

$$-[CH_2CH_2O]_q-[CH_2CH(CH_3)O]_r-$$

und/oder

$$-[OCH(CH_3)CH_2]_r-[OCH_2CH_2]_q-$$

mit

q = 1 bis 200,
r = 0 bis 200,

wobei das endständige Sauerstoffatom der Gruppe A an die endständige $-CH_2$-Gruppe der Gruppe E, und das endständige Carbonyl-Kohlenstoffatom der Gruppe A' an das endständige Sauerstoffatom der Gruppe E jeweils unter Ausbildung von Estergruppen binden,
und/oder mindestens eine endständige Polyalkylenoxid-Struktureinheit der Formel

$$-A-E-R^2$$

worin A und E die oben genannte Bedeutung aufweisen, und

$R^2$ = H, geradkettiger, cyclischer oder verzweigter $C_1$-$C_{20}$-Kohlenwasserstoffrest der durch -O-, oder -C(O)- unterbrochen und mit -OH substituiert und acetylenisch, olefinisch oder aromatisch sein kann,

b) mindestens ein zweiwertiger oder dreiwertiger organischer Rest, der mindestens eine Ammoniumgruppe enthält,

c) mindestens eine Polysiloxan-Struktureinheit der allgemeinen Formel:

$$-K-S-K-,$$

mit

S =

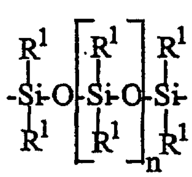

worin

$R^1$ = $C_1$-$C_{22}$-Alkyl, $C_1$-$C_{22}$-Fluoralkyl oder Aryl,
n = 0 bis 1000, und wenn mehrere Gruppen S in der Polysiloxanverbindung vorliegen, diese gleich oder verschieden sein können,

K = ein zweiwertiger oder dreiwertiger geradkettiger, cyclischer oder verzweigter $C_2$-$C_{40}$-Kohlenwasserstoffrest, der durch -O-, -NH-,

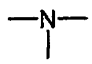
,

-$NR^1$-, -C(O)-, -C(S)-,

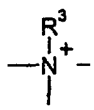
und
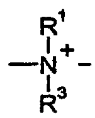

unterbrochen und mit -OH substituiert sein kann, worin

$R^1$ wie oben definiert ist, oder gegebenenfalls eine Bindung zu einem zweiwertigen Rest $R^3$ darstellt,

$R^3$ einen einwertigen oder zweiwertigen geradkettigen, cyclischen oder verzweigten $C_1$-$C_{20}$-Kohlenwasserstoffrest, der durch -O-, -NH-, -C(O)-, -C(S)- unterbrochen und mit -OH substituiert sein kann, oder -A-E-$R^2$ darstellt, worin A, E und $R^2$ wie oben definiert ist,

wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt,

d) mindestens einen organischen oder anorganischen Säurerest zur Neutralisation der aus der(n) Ammoniumgruppe(n) resultierenden Ladungen.

**2.** Polysiloxanverbindungen nach Anspruch 1, worin

K = ein zweiwertiger oder dreiwertiger geradkettiger, cyclischer oder verzweigter $C_2$-$C_{40}$-Kohlenwasserstoffrest, der durch -O-, -NH-,

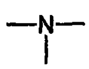
,

-$NR^1$-, -C(O)-, -C(S)- unterbrochen und mit -OH substituiert sein kann, worin $R^1$ wie oben definiert ist, und wobei die Reste K gleich oder verschieden voneinander sein können.

**3.** Polysiloxanverbindungen nach Anspruch 1 oder 2, worin der zuvor genannte organische Rest, der mindestens eine Ammoniumgruppe enthält, als Komponente b) ein Rest der allgemeinen Formel:

$$-N^1-F-N^1-$$

worin

$N^1$ eine quartäre Ammoniumgruppe der allgemeinen Formel

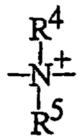

ist, worin

$R^4$ ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,

$R^5$ ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^4$ oder einem vierwertigen Rest F darstellt, und die Reste $R^4$ und $R^5$ innerhalb der Gruppe $-N^1-F-N^1-$ sowie in der Polysiloxanverbindung gleich oder verschieden voneinander sein können,

F = ein zweiwertiger oder vierwertiger geradkettiger, cyclischer oder verzweigter $C_2$-$C_{30}$-Kohlenwasserstoffrest darstellt, der durch -O-, -NH-,

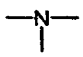 ,

-C(O)-, -C(S)-, eine Siloxankette S, wobei für S die oben genannten Bezüge gelten, unterbrochen und mit -OH substituiert sein kann,

ein Rest der allgemeinen Formel:

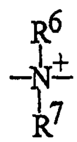

worin

$R^6$ = ein einwertiger oder zweiwertiger geradkettiger, cyclischer oder verzweigter $C_1$-$C_{30}$-Kohlenwasserstoffrest, der durch -O-, -NH-, -C(O)-, -C(S)- unterbrochen und mit -OH substituiert sein kann, oder eine Einfachbindung zu einem dreiwertigen Rest K darstellen kann,

$R^7$ ein einwertiger geradkettiger, cyclischer oder verzweigter $C_1$-$C_{20}$-Kohlenwasserstoffrest, der durch -O-, -NH- -C(O)-, -C(S)-unterbrochen und mit -OH substituiert sein kann,
oder $-A-E-R^2$ , worin $-A-E-R^2$ die oben genannte Bedeutung aufweist, oder eine Einfachbindung zu einem zweiwertigen Rest $R^6$ oder zu einem dreiwertigen Rest K darstellt,

und die Reste $R^6$ und $R^7$ gleich oder verschieden voneinander sein können,
oder ein Rest der Formel

$$-N^5-F^1-N^5-$$

ist, worin

$N^5$ eine Ammoniumgruppe der allgemeinen Formel

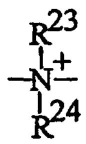

ist, worin

$R^{23}$ Wasserstoff, ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,

$R^{24}$ Wasserstoff, ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^{23}$ darstellt, und die Reste $R^{23}$ und $R^{24}$ innerhalb der Gruppe $-N^5-F^1-N^5-$ sowie in der Polysiloxanverbindung gleich oder verschieden voneinander sein können,

$F^1$ = ein zweiwertiger geradkettiger, cyclischer oder verzweigter Kohlenwasserstoffrest darstellt, der durch -O-, -NH-,

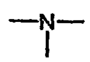 ,

-C(O)-, -C(S)- oder durch eine Gruppe -E- unterbrochen sein kann,

und worin eine Mehrzahl der Gruppen $N^5$ und $F^1$ jeweils gleich oder verschieden voneinander sein können.

**4.** Polysiloxane nach irgend einem der Ansprüche 1 bis 3 der allgemeinen Formel (I),

$$-[B-N^1-F-N^1]_m- \quad (I)$$

worin

m = 2 bis 500,

B = -A-E-K-S-K-E-A- und zusätzlich gegebenenfalls -A-E-A' - bzw. -A'-E-A- ist,

worin S, K, -A-E-, -E-A-, -A-E-A'- bzw. -A'-E-A- und $N^1$-F-$N^1$- wie oben definiert sind, und der Anteil der Gruppe -A-E-A'- bzw. -A'-E-A- in der Gruppe B so gewählt sein kann, dass die Masse von -A-E-A'- bzw. -A'-E-A- 0 bis 90 % der Masse des Polysiloxananteils S im Polymer beträgt.

**5.** Polysiloxan-Verbindung nach Anspruch 1 oder 2 der allgemeinen Formel (II),

$$R^2-E-A-N^2-K-S-K-N^2-A-E-R^2 \quad (II)$$

worin

S, K, -A-E-, -E-A- und $R^2$ die oben genannten Bedeutungen aufweisen, und

$N^2$ ein organischer Rest, der mindestens eine quartäre Ammoniumgruppe enthält, der allgemeinen Formel

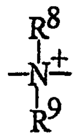

ist, worin

$R^8$ = ein einwertiger oder zweiwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann,

$R^9$ ein einwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann, oder eine Einfachbindung zu einem zweiwertigen Rest $R^8$ oder zu einem dreiwertigen Rest K darstellt, und die Reste $R^8$ und $R^9$ innerhalb der Polysiloxanverbindung der allgemeinen Formel (II) gleich oder verschieden voneinander sein können.

**6.** Polysiloxan-Verbindung nach Anspruch 1 oder 2 der allgemeinen Formel (III),

$$-[K\text{-}S\text{-}K\text{-}N^3]_{m}\text{-} \qquad \text{(III)}$$

in der

S, K und m wie oben definiert sind,

$N^3$ ein organischer Rest, der mindestens eine quartäre Ammoniumgruppe enthält, der allgemeinen Formel

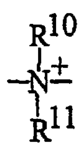

ist, worin

$R^{10}$ ein einwertiger geradkettiger, cyclischer oder verzweigter $C_1$-$C_{30}$-Kohlenwasserstoffrest, der durch -O-, -NH-, -C(O)-, -C(S)- unterbrochen und mit -OH substituiert sein kann oder eine Einfachbindung zu K darstellt,

$R^{11}$ = -A-E-$R^2$ , worin -A-B-$R^2$ die oben genannte Bedeutung aufweist.

**7.** Polysiloxan-Verbindungen nach Anspruch 1 oder 2 der allgemeinen Formel (IV),

$$-[N^4\text{-}K\text{-}S\text{-}K\text{-}N^4\text{-}A\text{-}E\text{-}A']_m\text{- bzw. -}[N^4\text{-}K\text{-}S\text{-}K\text{-}N^4\text{-}A'\text{-}E\text{-}A]_m\text{-} \qquad \text{(IV)}$$

worin

m, K, S, -A-E-A'- und -A'-E-A- wie oben definiert sind, und

$N^4$ ein organischer Rest, der mindestens eine quartäre Ammoniumgruppe enthält, der allgemeinen Formel

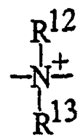

ist, worin

$R^{12}$ = ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann,

$R^{13}$ die Bedeutungen von $R^{12}$ aufweisen kann, oder eine Einfachbindung zu K oder $R^{12}$ darstellt,

und die Reste $R^{12}$ und $R^{13}$ gleich oder verschieden voneinander sein können.

**8.** Polysiloxanverbindung nach Anspruch 1 oder 2 der allgemeinen Formel (V)

$$-[N^5\text{-}F^1\text{-}N^5\text{-}Y\text{-}]_m \qquad (V)$$

worin

Y eine Gruppe der Formel

-K-S-K-

und

-A-E-A'- bzw. -A'-E-A-

ist, worin m, K, S, -A-E-A'- und -A'-E-A- wie oben definiert sind, die Gruppen K, S, -A-E-A'- und -A'-E-A- innerhalb der Polysiloxanverbindungen der allgemeinen Formel (V) gleich oder verschieden voneinander sein können, und das molare Verhältnis der Gruppe -K-S-K- und der Gruppe -A-E-A'- bzw. -A'-E-A- in der Polysiloxanverbindung der allgemeinen Formel (V) von 100: 1 bis 1 : 100 ist,

$N^5$ eine Ammoniumgruppe der allgemeinen Formel

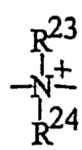

ist, worin

$R^{23}$ Wasserstoff, ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,

$R^{24}$ Wasserstoff, ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^{23}$ darstellt, und die Reste $R^{23}$ und $R^{24}$ innerhalb der Gruppe -$N^5$-$F^1$-$N^5$- sowie in der Polysiloxanverbindung gleich oder verschieden voneinander sein können,

$F^1$ = ein zweiwertiger geradkettiger, cyclischer oder verzweigter Kohlenwasserstoffrest darstellt, der durch -O-, -NH-,

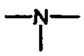 ,

-C(O)-, -C(S)- oder durch eine Gruppe -E- unterbrochen sein kann, worin E wie oben definiert ist,

und worin eine Mehrzahl von $N^5$ und $F^1$ jeweils gleich oder verschieden voneinander sein können.

**9.** Verwendung der Polysiloxanverbindungen nach irgend einem der Ansprüche 1 bis 8 in kosmetischen Formulierungen für die Haut- und Haarpflege, in Polituren für die Behandlung und Ausrüstung harter Oberflächen, in Formulierungen zum Trocknen von Automobilen und anderen harten Oberflächen, zum Beispiel nach maschinellen Wäschen, zur (Erst-)Ausrüstung von Textilien und Textilfasern, als separate Weichmacher nach dem Waschen von Textilien mit nichtionogenen oder anionischen/nichtionogen Detergenzienformulierungen, als Weichmacher in auf nichtionischen oder anionischen/nichtionischen Tensiden beruhenden Formulierungen zur Textilwäsche, sowie als Mittel zur Verhinderung bzw. Rückgängigmachung von Textilverknitterungen.

**10.** Zusammensetzungen, enthaltend mindestens eine der Polysiloxanverbindungen nach irgend einem der Ansprüche 1 bis 8, zusammen mit mindestens einem weiteren für die Zusammensetzung üblichen Inhaltsstoff.

## Claims

**1.** Polysiloxane compound containing:

a) At least one polyalkylene oxide structural unit with the general formula:

$$-A-E-,\ -E-A'-\ \text{and/or}\ -A'-E-A-$$

wherein

A = $-CH_2C(O)O-$, $-CH_2CH_2C(O)O-$, $-CH_2CH_2CH_2C(O)O-$, $-OC(O)CH_2-$, $OC(O)CH_2-$, $-OC(O)CH_2CH_2-$ and/or $-OC(O)CH_2CH_2CH_2-$

A' = $-CH_2C(O)-$, $-CH_2CH_2C(O)-$, $CH_2CH_2CH_2C(O)-$, $-C(O)CH_2-$, $-C(O)CH_2-$, $-C(O)CH_2CH_2-$ and/or $-C(O)CH_2CH_2CH_2-$

E = a polyalkylene oxide group of the general formulae:

$$-[CH_2CH_2O]_q-[CH_2CH(CH_3)O]_r-$$

and/or

$$-[OCH(CH_3)CH_2]_r-[OCH_2CH_2]_q-$$

With

q = 1 to 200,
r = 0 to 200,

whereby the terminal position oxygen atom of Group A binds to the terminal position $-CH_2$-group of Group E, and the terminal position carbonyl carbon atom of Group A' binds to the terminal position oxygen atom of Group E forming ester groups in each case,
and/or at least one terminal position polyalkylene oxide structural unit of the formula

$$-A-E-R^2$$

wherein A and E have the aforementioned meaning, and

$R^2$ = is H, straight chain, cyclical or branched $C_1$-$C_{20}$-hydrocarbon radical, which can be interrupted by -O-, or -C(O)- and substituted with -OH, and can be acetylenic, olefinic or aromatic

b) at least one bivalent or trivalent organic radical which contains at least one ammonium group,
c) at least one polysiloxane structural unit with the general formula:

$$-K-S-K-,$$

with

S =

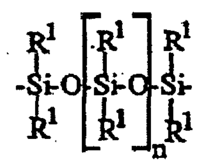

wherein

$R^1$ = $C_1$-$C_{22}$-alkyl, $C_1$-$C_{22}$-fluoralkyl or aryl,
n = 0 to 100, and these can be identical or different if several S Groups are present in the polysiloxane compound,

K = a bivalent or trivalent straight chain, cyclical or branched $C_2$-$C_{40}$ hydrocarbon residue which is interrupted by -O-, -NH-,

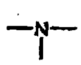,

$-NR^1-$, -C(O)-, -C(S)-,

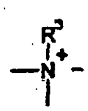 and 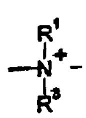

and substituted with -OH, wherein

$R^1$ is defined as above, or if need be represents a bond to a bivalent radical $R^3$
$R^3$ represents a monovalent or bivalent straight chain, cyclical or branched $C_1$-$C_{20}$ hydrocarbon radical which is interrupted by -O-, -NH-, -C(O)-, -C(S)- and can be substituted with -OH, or $-A-E-R^2$, wherein A, E and $R^2$ are defined as above,

whereby the residues K can be identical or different from each other, and in the event that K represents a trivalent radical, the saturation of the third valence takes place through a bonding to the above mentioned

organic radical which contains at least one ammonium group,
d) at least one organic or inorganic acid radical for neutralization of the charges resulting from the ammonium group(s).

**2.** Polysiloxane compounds according to claim 1, wherein

K = a bivalent or trivalent straight chain, cyclical or branched $C_2$-$C_{40}$ hydrocarbon radical which is interrupted by -O-, -NH-,

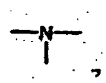

$NR^1$-, -C(O)-, -C(S)- and can be substituted with -OH, wherein $R^1$ is defined as above and whereby the radicals K can be identical or different from one another.

**3.** Polysiloxane compounds according to claim 1 or 2, wherein the previously mentioned organic radical, which contains at least one ammonium group, is a radical of the general formula as component b)

$$-N^1-F-N^1-$$

wherein

$N^1$ is a quaternary ammonium group of the general formula

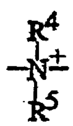

is, wherein

$R^4$ represents a monovalent or bivalent straight chain, cyclical or branched $C_1$-$C_{20}$ hydrocarbon residue, which is interrupted by -O-, -NH-, -C(O)-, -C(S)- and can be substituted with -OH,

$R^5$ represents a monovalent straight chain, cyclical or branched $C_1$-$C_{20}$ hydrocarbon residue which can be interrupted by -O-, -NH-, -C(O)-, -C(S)- and substituted with -OH, or a single bond to a bivalent radical $R^4$ or a quadrivalent radical F, and the radicals $R^4$ and $R^5$ within the group $-N^1F-N^1-$ as well as in the polysiloxane compound can be identical or different from one another,

F = represents a bivalent or quadrivalent straight chain, cyclical or branched $C_2$-$C_{30}$ hydrocarbon radical which can be interrupted by -O-, -NH-,

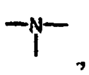

-C(O)-, -C(S)-, a siloxane chain S (whereby the above references apply for S) and substituted with -OH, a radical of the general formula:

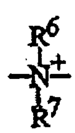

wherein

$R^6$ = a monovalent or bivalent straight chain, cyclical or branched $C_1$-$C_{30}$ hydrocarbon radical, which can be interrupted by -O-, -NH-, -C(O)-, -C(S)- and be substituted with -OH, or can represent a trivalent radical K,

$R^7$ a monovalent straight chain, cyclical or branched $C_1$-$C_{20}$ hydrocarbon radical which can be interrupted by -O-, -NH-, -C(O)-, -C(S)- and be substituted with -OH, or -A-E-$R^2$; wherein -A-E-$R^2$ has the aforementioned meaning, or represents a single bond to a bivalent radical $R^6$ or to a trivalent radical K

and the radicals $R^6$ and $R^7$ can be identical or different from each other,

or is a radical of the formula

$$-N^5-F^1-N^5-$$

wherein

$N^5$ is an ammonium group of the general formula

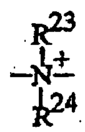

wherein

$R^{23}$ represents hydrogen, a monovalent or bivalent straight chain, cyclical or branched $C_1$-$C_{20}$ hydrocarbon radical which can be interrupted by-O-, -NH-, -C(O)-, -C(S)- and be substituted with -OH,

$R^{24}$ represents hydrogen, a monovalent straight chain, cyclical or branched $C_1$-$C_{20}$ hydrocarbon radical which can be interrupted by -O-, -NH-, -C(O)-, -C(S)- and be substituted with -OH, or represents a single bond to a bivalent radical $R^{23}$, and the radicals $R^{23}$ and $R^{24}$ can be identical or different from each other inside the group -$N^5$-$F^1$-$N^5$- as well as in the polysiloxane compound,

$F^1$ = represents a bivalent straight chain, cyclical or branched hydrocarbon radical which can be interrupted by -O-, -NH-,

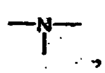

-C(O)-, -C(S)-or by the group -E-, and wherein a majority of groups $N^5$ and $F^1$ can be identical or different from one another in any given case.

**4.** Polysiloxanes according to any one of claims 1 to 3 of the general formula (I),

$$-[B-N^1-F-N^1]_m- \qquad (I)$$

wherein

m = 2 to 500,

B = -A-E-K-S-K-E-A- and in addition if need be is -A-E-A'- or -A'-E-A-,

wherein S, K, -A-E-, -E-A-, -A-E-A'- or -A'-E-A- and -$N^1$-F-$N^1$- are defined as above, and the proportion of Group -A-E-A'- or -A'-E-A- in Group B can be selected such that the mass of -A-E-A'- or -A'-E-A- comes to 0 to 90% of the mass of the polysiloxane component S in the polymer.

**5.** Polysiloxane compound according to claim 1 or 2 of the general formula (II),

$$R^2\text{-E-A-}N^2\text{-K-S-K-}N^2\text{-A-E-}R^2 \qquad \text{(II)}$$

wherein

S, K, -A-E-, -E-A- and $R^2$ have the aforementioned meanings and
$N^2$ is an organic radical which contains at least one quaternary ammonium group of the general formula

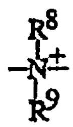

wherein

$R^8$ = a monovalent or bivalent straight chain, cyclical or branched $C_1$-$C_{20}$ hydrocarbon radical which can be interrupted by -O-, -NH-, -C(O)-, -C(S)- and can be substituted by -OH,
$R^9$ a monovalent straight chain, cyclical or branched $C_1$-$C_{20}$ hydrocarbon radical which can be interrupted by -O-, -NH-, -C(O)-, -C(S)- and be substituted with -OH, or represents a single bond to a bivalent radical $R^8$ or to a trivalent radical K, and the radicals $R^8$ and $R^9$ can be identical or different from each other within the polysiloxane compound of general formula (II).

**6.** Polysiloxane compound according to claim 1 or 2 of general formula (III),

$$\text{-[K-S-K-}N^3]_{m\text{-}} \qquad \text{(III)}$$

in which

S, K and m are defined as above,
$N^3$ is an organic radical which contains at least one ammonium group of the general formula

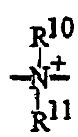

wherein

$R^{10}$ a monovalent straight chain, cyclical or branched $C_1$-$C_{30}$ hydrocarbon radical which can be interrupted by -O-, -NH-, -C(O)-, -C(S)- and be substituted by -OH or represents a single bond to K,
$R^{11}$ = -A-E-$R^2$, wherein -A-E-$R^2$ has the aforementioned meaning.

**7.** Polysiloxane compounds according to claim 1 or 2 of general formula (IV),

$$\text{-[}N^4\text{-K-S-K-}N^4\text{-A-E-A']}_{m\text{-}} \text{ or -[}N^4\text{-K-S-K-}N^4\text{-A'-E-A]}_{m\text{-}} \qquad \text{(IV)}$$

wherein

m, K, S, A-E-A'- and -A'-E-A- are defined as above, and
$N^4$ is an organic radical which contains at least one quaternary ammonium group of the general formula

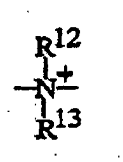

wherein

$R^{12}$ = is a monovalent or bivalent straight chain, cyclical or branched $C_1$-$C_{20}$ hydrocarbon radical which can be interrupted by -O-, -NH-, -C(O)-, -C (S)- and be substituted with -OH,
$R^{13}$ can have the meanings of $R^{12}$ or represents a single bond to K or $R^{12}$,

and the radicals $R^{12}$ and $R^{13}$ can be identical or different from each other.

**8.** Polysiloxane compound according to claim 1 or 2 of general formula (V)

$$-[N^5-F^1-N^5-Y-]_m \qquad (V)$$

wherein

Y is a group of the formula

-K-S-K-

and

-A-E-A'- or -A'-E-A-,

wherein m, K, S, -A-E-A'- and -A'-E-A- are defined as above, the groups K, S, -A-E-A'- and -A'-E-A- can be identical or different from each other within the polysiloxane compounds of general formula (V), and the molar ratio of Group -K-S-K- and Group -A-E-A'- or -A'-E-A- in the polysiloxane compound of general formula (V) is from 100:1 to 1:100,
$N^5$ is an ammonium group of the general formula

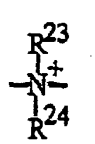

wherein

$R^{23}$ represents hydrogen, a monovalent or bivalent straight chain, cyclical or branched $C_1$-$C_{20}$ hydrocarbon radical which can be interrupted by-O-, -NH-, -C(O)-, -C(S)- and be substituted by -OH,
$R^{24}$ represents hydrogen, a monovalent straight chain, cyclical or branched $C_1$-$C_{20}$ hydrocarbon radical which can be interrupted by -O-, -NH-, -C(O)-, -C(S)- and be substituted with -OH, or represents a

single bond to a bivalent radical $R^{23}$, and the radicals $R^{23}$ and $R^{24}$ can be identical or different from each other within the Group $-N^5-F^1-N^5-$ as well as in the polysiloxane compound

$F^1$ = represents a bivalent straight chain, cyclical or branched hydrocarbon residue which can be interrupted by -O-, -NH-,

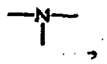

-C(O)-, -C(S)-or by a Group E, wherein E is defined as above, and wherein the majority of $N^5$ and $F^1$ can be identical or different from each other in any given case.

**9.** Use of polysiloxane compounds according to any one of claims 1 to 8 in cosmetic formulations for skin and hair care, in polishes for the treatment and outfitting of hard surfaces, in formulations for drying automobiles and other hard surfaces, for example following machine washing, for (initial) outfitting/fabric treatment of textiles and textile fibers, as separate softeners after washing textiles with non-ionogenic or anionic/non-ionogenic detergent formulations, as a softener in formulations for textile washes based upon non-ionic or anionic/non-ionic surfactants as well as agents for preventing or reversing textile crumpling/wrinkling.

**10.** Compositions containing at least one of the polysiloxane compounds according to any one of claims 1 to 8, together with at least one further ingredient typical for the composition.

## Revendications

**1.** Composé polysiloxane contenant :

a) au moins une unité structurale de poly(oxyde d'alkylène) des formules générales :

-A-E-, -E-A-, -A-E-A'- et/ou -A'-E-A-

où

A = $-CH_2C(O)O-$, $-CH_2CH_2C(O)O-$, $-CH_2CH_2CH_2C(O)O-$, $-OC(O)CH_2-$, $-OC(O)CH_2-$, $-OC(O)CH_2CH_2-$ et/ou $-OC(O)CH_2CH_2CH_2-$

A' = $-CH_2C(O)-$, $-CH_2CH_2C(O)-$, $-CH_2CH_2CH_2C(O)-$, $-C(O)CH_2-$, $-C(O)CH_2-$, $-C(O)CH_2CH_2-$ et/ou $-C(O)CH_2CH_2CH_2-$

E = un groupe poly(oxyde d'alkylène) des formules générales :

$$-[CH_2CH_2O]_q-[CH_2CH(CH_3)O]_r-$$

et/ou

$$-[OCH(CH_3)CH_2]_r-[OCH_2CH_2]_q-$$

avec

q = 1 à 200,
r = 0 à 200,

où l'atome d'oxygène terminal du groupe A est lié au groupe $-CH_2-$ terminal du groupe E, et l'atome de carbone de carbonyle terminal du groupe A' est lié à l'atome d'oxygène terminal du groupe E, dans chaque

cas en formant des groupes esters,
et/ou au moins une unité structurale de poly(oxyde d'alkylène) terminale de formule

$$-A-E-R^2$$

où

A et E  ont la signification citée ci-dessus, et
$R^2$ =  H, un groupement hydrocarboné en $C_1$-$C_{20}$ linéaire, cyclique ou ramifié, qui peut être interrompu par -O-, ou -C(O)- et substitué par -OH et qui peut être acétylénique, oléfinique ou aromatique,

b) au moins un groupement organique divalent ou trivalent, qui contient au moins un groupe ammonium,
c) au moins une unité structurale de polysiloxane de formule générale :

$$-K-S-K-,$$

avec

S =

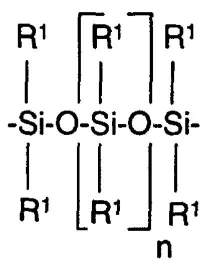

où

$R^1$ =  alkyle en $C_1$-$C_{22}$, fluoroalkyle en $C_1$-$C_{22}$ ou aryle,
n =  0 à 1 000, et quand plusieurs groupes S sont présents dans le composé polysiloxane, ceux-ci peuvent être identiques ou différents,

K =  un groupement hydrocarboné en $C_2$-$C_{40}$ divalent ou trivalent linéaire, cyclique ou ramifié, qui peut être interrompu par -O-, -NH-,

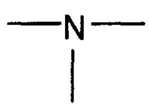

$-NR^1-$, -C(O)-, -C(S)-,

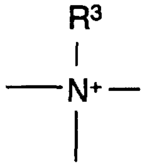

et

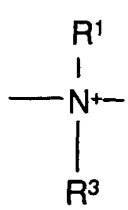

et substitué par -OH, où

$R^1$ est défini comme ci-dessus, ou représente éventuellement une liaison avec un groupement divalent $R^3$,
$R^3$ représente un groupement hydrocarboné en $C_1$-$C_{20}$ monovalent ou divalent, linéaire, cyclique ou ramifié, qui peut être interrompu par -O-, -NH-, -C(O)-, -C(S)- et substitué par -OH, ou -A-E-$R^2$, où A, E et $R^2$ sont définis comme ci-dessus,

où les groupements K peuvent être identiques ou différents, et dans le cas où K représente un groupement trivalent, la saturation de la troisième valence a lieu par une liaison avec le groupement organique cité précédemment, qui contient au moins un groupe ammonium.
d) au moins un groupement acide organique ou inorganique pour la neutralisation des charges résultant du ou des groupes ammonium.

**2.** Composés polysiloxanes selon la revendication 1 où

K = un groupement hydrocarboné en $C_2$-$C_{40}$ divalent ou trivalent linéaire, cyclique ou ramifié qui peut être interrompu par -O-, -NH-,

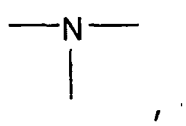

-$NR^1$-, -C(O)-, -C(S)- et substitué par -OH, où $R^1$ est défini comme ci-dessus, et où les groupements K peuvent être identiques ou différents.

**3.** Composés polysiloxanes selon la revendication 1 ou 2 où le groupement organique cité précédemment, qui contient au moins un groupe ammonium, comme composant b) est un groupement de formule générale :

$$-N^1-F-N^1-$$

où

$N^1$ est un groupe ammonium quaternaire de formule générale :

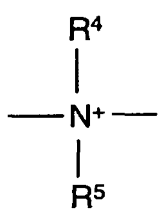

où

$R^4$ représente un groupement hydrocarboné en $C_1$-$C_{20}$ monovalent ou divalent linéaire, cyclique ou ramifié qui peut être interrompu par -O-, -NH-, -C(O)-, -C(S)- et substitué par -OH,
$R^5$ représente un groupement hydrocarboné en $C_1$-$C_{20}$ monovalent linéaire, cyclique ou ramifié, qui peut être interrompu par -O-, -NH-, -C(O)-, -C(S)- et substitué par -OH, ou une simple liaison avec un groupement divalent $R^4$ ou représente un groupement tétravalent F, et les groupements $R^4$ et $R^5$ dans le groupe -$N^1$-F-$N^1$- ainsi que dans le composé polysiloxane peuvent être identiques ou différents,

F = un groupement hydrocarboné en $C_2$-$C_{30}$ divalent ou tétravalent linéaire, cyclique ou ramifié, qui peut être interrompu par -O-, -NH-,

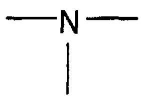

,

-C(O)-, -C(S)-, une chaîne siloxane S où ce qui a été dit précédemment est valable pour S, et substitué par -OH,

un groupement de formule générale :

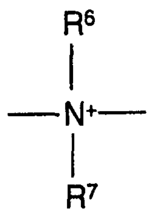

où

$R^6$ = un groupement hydrocarboné en $C_1$-$C_{30}$ monovalent ou divalent linéaire, cyclique ou ramifié, qui peut être interrompu par -O-, -NH-, -C(O)-, -C(S)- et substitué par -OH, ou une simple liaison avec un groupement trivalent K,

$R^7$ représente un groupement hydrocarboné en $C_1$-$C_{20}$ monovalent linéaire, cyclique ou ramifié qui peut être interrompu par -O-, -NH- -C(O)-, -C(S)- et substitué par -OH, ou -A-E-$R^2$, où -A-E-$R^2$ présente la signification citée ci-dessus, ou une simple liaison avec un groupement divalent $R^6$ ou avec un groupement trivalent K,

et les groupements $R^6$ et $R^7$ peuvent être identiques ou différents,
ou un groupement de formule

$$-N^5-F^1-N^5-$$

où

$N^5$ représente un groupe ammonium de formule générale :

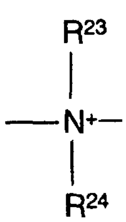

$R^{23}$ représente l'hydrogène, un groupement hydrocarboné en $C_1$-$C_{20}$ monovalent ou divalent linéaire, cyclique ou ramifié, qui peut être interrompu par -O-, -NH-, -C(O)-, -C(S)- et substitué par -OH,

$R^{24}$ représente l'hydrogène, un groupement hydrocarboné en $C_1$-$C_{20}$ monovalent linéaire, cyclique ou ramifié, qui peut être interrompu par -O-, -NH-, -C(O)-, -C(S)- et substitué par -OH, ou une simple liaison avec un groupement divalent $R^{23}$, et les groupements $R^{23}$ et $R^{24}$ à l'intérieur du groupe -$N^5$-$F^1$-$N^5$- ainsi que dans le composé siloxane peuvent être identiques ou différents,

$F^1$ = un groupement hydrocarboné divalent linéaire, cyclique ou ramifié, qui peut être interrompu par -O-, -NH-,

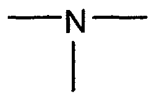 ,

-C(O)-, -C(S)- ou par un groupe -E-,

et où plusieurs des groupes $N^5$ et $F^1$ peuvent être identiques ou différents.

**4.** Polysiloxanes selon l'une quelconque des revendications 1 à 3 de formule générale (I)

$$-[B-N^1-F-N^1]_m- \quad (I)$$

où

m = 2 à 500,
B = -A-E-K-S-K-E-A- et en outre éventuellement -A-E-A'- ou -A'-E-A-,

où S, K, -A-E, -E-A-, -A-E-A'- ou -A'-E-A- et $-N^1-F-N^1-$ sont définis comme ci-dessus, et

la proportion du groupe -A-E-A'- ou -A'-E-A- dans le groupe B peut être choisie de telle manière que la masse de -A-E-A'- ou -A'-E-A- est 0 à 90 % de la masse de la partie polysiloxane S dans le polymère.

**5.** Composé polysiloxane selon la revendication 1 ou 2 de formule générale (II)

$$R^2-E-A-N^2-K-S-K-N^2-A-E-R^2 \quad (II)$$

où

S, K, -A-E-, -E-A- et $R^2$ présentent les significations citées ci-dessus, et

$N^2$ représente un groupement organique qui contient au moins un groupe ammonium quaternaire, de formule générale :

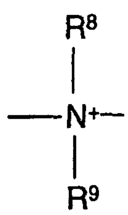

où

$R^8$ = un groupement hydrocarboné en $C_1$-$C_{20}$ monovalent ou divalent linéaire, cyclique ou ramifié qui peut être interrompu par -O-, -NH- -C(O)-, -C(S)- et substitué par -OH,

$R^9$ représente un groupement hydrocarboné en $C_1$-$C_{20}$ monovalent linéaire, cyclique ou ramifié qui peut être interrompu par -O-, -NH- -C(O)-, -C(S)- et substitué par -OH, ou une simple liaison avec un groupement divalent $R^8$ ou avec un groupement trivalent K, et les groupements $R^8$ et $R^9$ à l'intérieur du composé polysiloxane de formule générale (II) peuvent être identiques ou différents.

**6.** Composé polysiloxane selon la revendication 1 ou 2 de formule générale (III)

$$-[K\text{-}S\text{-}K\text{-}N^3]_m- \quad \text{(III)}$$

où

S, K et m sont définis comme ci-dessus,
$N^3$ représente un groupement organique qui contient au moins un groupe ammonium quaternaire, de formule générale

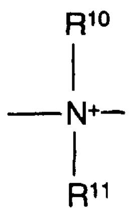

où

$R^{10}$ représente un groupement hydrocarboné en $C_1$-$C_{30}$ monovalent linéaire, cyclique ou ramifié qui peut être interrompu par -O-, -NH-, -C(O)-, C(S)- et substitué par -OH ou une simple liaison avec K,
$R^{11}$= -A-E-$R^2$, où -A-E-$R^2$ présente la signification citée ci-dessus.

**7.** Composés polysiloxanes selon la revendication 1 ou 2 de formule générale (IV)

$$-[N^4\text{-}K\text{-}S\text{-}K\text{-}N^4\text{-}A\text{-}E\text{-}A']_m- \text{ ou } -[N^4\text{-}K\text{-}S\text{-}K\text{-}N^4\text{-}A'\text{-}E\text{-}A]_m- \quad \text{(IV)}$$

où

m, K, S, -A-E-A'- et -A'-E-A- sont définis comme ci-dessus, et
$N^4$ représente un groupement organique qui contient au moins un groupe ammonium quaternaire, de formule générale

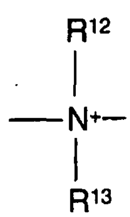

où

$R^{12}$ = un groupement hydrocarboné en $C_1$-$C_{20}$ monovalent ou divalent linéaire, cyclique ou ramifié, qui peut être interrompu par -O-, -NH-, -C(O)-, -C(S)- et substitué par -OH,
$R^{13}$ peut présenter les significations de $R^{12}$, ou représente une simple liaison avec K ou $R^{12}$,

et les groupements $R^{12}$ et $R^{13}$ peuvent être identiques ou différents.

**8.** Composé polysiloxane selon la revendication 1 ou 2 de formule générale (V)

$$-[N^5-F^1-N^5-Y-]_m \quad (V)$$

où

Y est un groupe de formule

-K-S-K-

et

-A-E-A'- ou -A'-E-A-,

où m, K, S, -A-E-A'- et -A'-E-A- sont définis comme ci-dessus, les groupes K, S, -A-E-A'- et -A'-E-A- à l'intérieur des composés polysiloxanes de formule générale (V) peuvent être identiques ou différents, et le rapport molaire du groupe -K-S-K- et du groupe -A-E-A'- ou -A'-E-A- dans le composé polysiloxane de formule générale (V) est de 100 : 1 à 1 : 100,

$N^5$ représente un groupe ammonium de formule générale

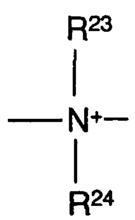

où

$R^{23}$ représente l'hydrogène, un groupement hydrocarboné en $C_1$-$C_{20}$ monovalent ou divalent linéaire, cyclique ou ramifié, qui peut être interrompu par -O-, -NH-, -C(O)-, -C(S)- et substitué par -OH,

$R^{24}$ représente l'hydrogène, un groupement hydrocarboné en $C_1$-$C_{20}$ monovalent linéaire, cyclique ou ramifié qui peut être interrompu par -O-, -NH-, -C(O)-, -C(S)- et substitué par -OH, ou une simple liaison avec un groupement divalent $R^{23}$, et les groupements $R^{23}$ et $R^{24}$ à l'intérieur du groupe $-N^5-F^1-N^5-$ ainsi que dans le composé polysiloxane peuvent être identiques ou différents,

$F^1$ = un groupement hydrocarboné divalent linéaire, cyclique ou ramifié, qui peut être interrompu par -O-, -NH-,

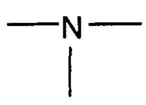

,

-C(O)-, -C(S)- ou par un groupe -E-, où E est défini comme ci-dessus,

et où plusieurs des $N^5$ et $F^1$ peuvent être identiques ou différents.

**9.** Utilisation des composés polysiloxanes selon l'une quelconque des revendications 1 à 8 dans des formulations cosmétiques pour les soins de la peau et des cheveux, dans des vernis pour le traitement et le finissage des

surfaces dures, dans des formulations pour le séchage d'automobiles et d'autres surfaces dures, par exemple après un lavage à la machine, pour le (premier) apprêtage de textiles et de fibres textiles, comme assouplissants séparés après le lavage des textiles avec des formulations détergentes non ionogènes ou anioniques/non ioniques, comme assouplissants dans des formulations pour le lavage des textiles basées sur des tensioactifs non ioniques ou anioniques/non ioniques, ainsi que comme agents pour éviter ou supprimer les froissures des textiles.

**10.** Compositions contenant au moins l'un des composés polysiloxanes selon l'une quelconque des revendications 1 à 8, en même temps qu'au moins un autre composant courant pour la composition.